(19) **Europäisches Patentamt / European Patent Office / Office européen des brevets**

(11) **EP 3 328 438 B1**

(12) **EUROPEAN PATENT SPECIFICATION**

(45) Date of publication and mention
of the grant of the patent:
**15.07.2026 Bulletin 2026/29**

(21) Application number: **16831335.1**

(22) Date of filing: **28.07.2016**

(51) International Patent Classification (IPC):
*A61K 47/32* (2006.01)  *A61K 47/42* (2017.01)
*A61L 27/14* (2006.01)  *A61L 27/22* (2006.01)
*A61L 27/56* (2006.01)  *A61L 27/54* (2006.01)
*A61L 27/46* (2006.01)  *A61K 9/50* (2006.01)

(52) Cooperative Patent Classification (CPC):
**A61L 27/56; A61K 9/5052; A61L 27/227;
A61L 27/46; A61L 27/54;** A61L 2300/62

(86) International application number:
**PCT/US2016/044411**

(87) International publication number:
**WO 2017/019841 (02.02.2017 Gazette 2017/05)**

(54) **POLYPEPTIDE MONOLITHS**

POLYPEPTID-MONOLITHEN

MONOLITHES POLYPEPTIDIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.07.2015 US 201562197693 P**

(43) Date of publication of application:
**06.06.2018 Bulletin 2018/23**

(73) Proprietor: **Trustees of Tufts College
Medford, MA 02155 (US)**

(72) Inventors:
• **OMENETTO, Fiorenzo G.**
**Lexington, Massachusetts 02421 (US)**
• **KAPLAN, David L.**
**Concord, Massachusetts 01742 (US)**
• **MARELLI, Benedetto**
**Lexington, Massachusetts 02421 (US)**
• **LI, Chunmei**
**Stoneham, Massachusetts 02180 (US)**

(74) Representative: **Marks & Clerk LLP
15 Fetter Lane
London EC4A 1BW (GB)**

(56) References cited:
WO-A1-2014/012101   WO-A2-2011/005381
US-A1- 2006 084 607   US-A1- 2011 008 437
US-A1- 2011 008 437   US-A1- 2011 111 031
US-A1- 2012 021 020   US-A1- 2015 025 005
US-B2- 7 449 180

• MATSUMOTO ET AL.: "Mechanisms of silk
fibroin sol-gel transitions", JOURNAL OF
PHYSICAL CHEMISTRY B, vol. 110, no. 43, 2
November 2006 (2006-11-02), pages 21630 -
21638, XP055108402
• WARWICKER, J. ET AL.: "The crystal structure of
silk fibroin", ACTA CRYSTALLOGRAPHICA, vol.
7, no. 8-9, 20 September 1954 (1954-09-20), pages
565 - 573, XP055349724

# EP 3 328 438 B1

**Description**

CROSS-REFERENCE TO RELATED APPLICATIONS

This patent application claims priority to and the benefit of, United States provisional patent application serial number 62/197,693, filed on July 28, 2015, entitled "Polypeptide Monoliths".

GOVERNMENT SUPPORT

This invention was made with government support under grant AR068048 awarded by the National Institutes of Health and grant FA9550-10-1-0172 awarded by the Air Force Office of Scientific Research. The government has certain rights in the invention.

BACKGROUND

Monoliths are three-dimensional blocks of material that are both solid and continuous. The term "monolith" is typically used to refer to materials characterized by properties (e.g., bulk density, hardness, resistance to fracture, etc.) that render them amenable to processing into hard structures. Thus monoliths of appropriate size may be machined into desirable shapes. Protein/polypeptide monoliths are of particular interest for a variety of reasons, including their potential biocompatibility and biodegradability. Additionally, appropriate monolith materials may tolerate inclusion or incorporation of additives that, for example, may provide some added functionality to the blocks. WO 2011/005381 describes a method of preparing a silk fibroin gel-encapsulated active agent. US 2011/008437 describes a drug delivery platform comprising a hydrogel formulation comprising a gel phase, the gel phase including hydrogel particles comprising silk fibroin.

SUMMARY OF THE INVENTION

The invention is set out in the appended claims. Among other things, the present disclosure provides solid materials comprising at least one amphiphilic polypeptide comprising silk fibroin, referred to herein as "amphiphilic polypeptide materials", in accordance with claim 1. In some embodiments, provided amphiphilic polypeptide materials are characterized in that such materials are three-dimensional. In some embodiments, provided amphiphilic polypeptide materials are three dimensional "blocks" also referred to herein as "monoliths." Such amphiphilic polypeptide materials exhibit unique structural characteristics and properties when compared with known monoliths. In some embodiments, provided amphiphilic polypeptide materials are three dimensional structures that are surprising large relative to any known protein/polypeptide monolith materials. In some embodiments, provided amphiphilic polypeptide materials are characterized in that they are capable of being machined using machine tools. In some embodiments, amphiphilic polypeptide materials generated are replicate nanoscale structures of natural fibers.

The present disclosure also provides a method of making amphiphilic polypeptide materials, in accordance with claim 14.

Implementations of the present disclosure are useful for a wide range of applications, including but not limited to: anti-counterfeiting materials, biomaterials, biomedical devices, commercial products, controlled degradation applications, controlled release applications, drug delivery, drug release, electronics, extrusion injection molding, materials for tunable degradation, optics, orthopedic devices, photonics, preservation of biologically labile compounds, preservation of heat labile compounds, prosthetics, regenerative medicine, robotics, sensing, tissue engineering applications, tissue regeneration, tissue scaffolding, triggered release applications and/or wound clotting.

The polypeptide materials are or comprise amphiphilic polypeptides and/or fragments thereof.

In some embodiments, provided amphiphilic polypeptide materials comprise amphiphilic polypeptides and/or fragments thereof that have a specific molecular weight. In some embodiments, amphiphilic polypeptide and/or fragments thereof comprise amphiphilic polypeptides having varied molecular weights. In some embodiments, an average molecular weight of amphiphilic polypeptide is for example, between about 100 Da and about 400 kDa.

In some embodiments, provided amphiphilic polypeptide materials are characterized by density. In some embodiments, provided amphiphilic polypeptide materials are characterized by a bulk density. In some embodiments, provided amphiphilic polypeptide materials have a bulk density that is comparable to the density of a native polypeptide when in its fibroin or globular structure form. In some embodiments, provided amphiphilic polypeptide materials are characterized by a bulk density that is uniform or continuous throughout the material. In some embodiments, provided amphiphilic polypeptide materials have a micro-density continuity. In some embodiments, provided amphiphilic polypeptide materials have a nano-density continuity.

In some embodiments, provided amphiphilic polypeptide materials are characterized by a density near that of natural amphiphilic polypeptide materials. In some embodiments, provided amphiphilic polypeptide materials are characterized by a density of about 0.05 kg/dm$^3$ and about 5.0 kg/dm$^3$. In some embodiments, for example, when an amphiphilic polypeptide is silk, provided amphiphilic polypeptide materials are characterized by a density of about 1.4 kg/dm$^3$.

In some embodiments, provided amphiphilic polypeptide materials are water-based. In some embodiments, provided amphiphilic polypeptide materials contain residual water. In some embodiments, provided amphiphilic polypeptide materials have a low bound solvent content. In some embodiments, provided amphiphilic polypeptide materials are water-based and characterized by a low bound water content. Provided amphiphilic polypeptide materials have a frozen and non-frozen bound water content of less than 50%.

In some embodiments, provided amphiphilic polypeptide materials are free of or are substantially free of non-aqueous solvents. In some embodiments, provided amphiphilic polypeptide materials do not contain residual non-aqueous solvents.

In some embodiments, provided amphiphilic polypeptide materials are characterized by amphiphilic polypeptides with structures self-assemble. In some embodiments, provided amphiphilic polypeptide materials self-assemble to form such three-dimensional structures. Amphiphilic polypeptides, in some embodiments, comprise a hydrophobic tail, an amino acid region, and a hydrophilic end.

In some embodiments, a hydrophilic end of an amphiphilic polypeptide material is designed to allow solubility of the molecule in water. In some embodiments, a hydrophilic end permits or enhances biological function of an amphiphilic polypeptide material.

In some embodiments, a hydrophobic tail of the amphiphilic polypeptide is repelled by water so that such tails aggregate.

In some embodiments, an amino acid domain of an amphiphilic polypeptide material is characterized by highly repetitive (i.e. crystalline) amino acid sequences intervaled by disordered (i.e. amorphous) sequences. In some embodiments, an amino acid region of provided amphiphilic polypeptide materials includes sequences that initiate intermolecular interactions and promote self-assembly. In some embodiments, provided amphiphilic polypeptide materials form cross-links which drive the folding of the proteins in long-range ordered molecular secondary, tertiary and quaternary structures (e.g. beta-sheets, coils, helices). As such, in some embodiments, individual domains of amphiphilic polypeptides associate with each other and result in growth of larger assemblies.

In some embodiments, provided amphiphilic polypeptide materials are characterized as crystalline and/or comprising nano-sized crystalline particles. In some embodiments, provided amphiphilic polypeptide materials comprise submicron size or nano-sized crystals, crystallized spheres and/or particles.

In some embodiments, provided amphiphilic polypeptide materials are characterized by their crystalline shape. In some embodiments, crystals have a high aspect ratio (i.e. needle shape).

In some embodiments, provided amphiphilic polypeptide materials are characterized by a network of amphiphilic polypeptide globules. In some embodiments, microstructures comprise globular structure form. In some embodiments, microstructures comprise a plurality of individual globules. In some embodiments, globules are nano-globules. In some embodiments, globules have at least one dimension between about 5 nm and about 45 nm.

In some embodiments, amphiphilic polypeptide globules aggregate through interactions with one another. In some embodiments, amphiphilic polypeptide globules have at least one dimension between about 5 nm and about 30 nm. In some embodiments, a plurality of globular structures have at least one dimension between about 5 nm and about 30 nm. Individual globule structures are aggregated with one another.

Amphiphilic polypeptide globules aggregate through interactions with one another. In some embodiments, globules aggregate with one another so that they form interlocking structures. In some embodiments, amphiphilic polypeptide globules aggregate to form structures of tens to hundreds of nanometers in size. In some embodiments, amphiphilic polypeptide globules aggregate to form amphiphilic polypeptide nano- and micro- structures. In some embodiments, amphiphilic polypeptide globules interact with each other to form a dense network. In some embodiments, a globular packing density may affect mechanical properties of provided materials.

In some embodiments, provided amphiphilic polypeptide materials are characterized as possessing secondary structure. In some embodiments, provided amphiphilic polypeptide materials are substantially amorphous, substantially crystalline, or combinations thereof. In some embodiments, provided amphiphilic polypeptide materials comprise helical structures, beta structures, or combinations thereof. In some embodiments, provided amphiphilic polypeptide materials are characterized by secondary structures such that relative amounts or concentrations of such secondary structures are uniformly distributed and/or consistent throughout a bulk of the material.

In some embodiments, provided amphiphilic polypeptide materials comprise beta sheet structure and are characterized by a crystalline orientation such that the materials exhibit crystalline alignment in accordance with a plane or a direction. In some embodiments, provided amphiphilic polypeptide materials are characterized by a tendency to cleave or split cleave along these distinct crystallographic structural planes.

In some embodiments, provided amphiphilic polypeptide materials are characterized by beta sheet character. In some embodiments, provided amphiphilic polypeptide materials are characterized by at least about 40% beta sheet. In some embodiments, provided amphiphilic polypeptide materials are characterized by at least about 45% beta sheet. In some embodiments, provided amphiphilic polypeptide materials are characterized by at least about 50% beta sheet. In some embodiments, provided amphiphilic polypeptide materials are characterized by at least about 55% beta sheet.

In some embodiments, provided amphiphilic polypeptide materials are characterized by toughness. In some embodiments, toughness reflects a threshold resistance to damage from pressure or an impact. In some embodiments, provided amphiphilic polypeptide materials are characterized fracture toughness, that is an indication of an amount of stress required to propagate a preexisting flaw.

In some embodiments, provided amphiphilic polypeptide materials are characterized by a response to an applied compression, expansion, deformation, etc. In some embodiments, for example, provided amphiphilic polypeptide materials are characterized by an elastic modulus value in a range between about 100 Pa and about 5000 kPa. In some embodiments, provided amphiphilic polypeptide materials are characterized by a compressive modulus value in a range between about 500 Pa and about 100 GPa. In some embodiments, provided amphiphilic polypeptide materials are characterized by a storage modulus value in a range between about 1 kPa and about 3000 kPa. In some embodiments, provided amphiphilic polypeptide materials are characterized by a resistance to deformation from an applied shear stress of up to about 1 GPa. In some embodiments, provided amphiphilic polypeptide materials are characterized by a resistance to damage from an applied pull-out force of about 500 N.

In some embodiments, provided amphiphilic polypeptide materials are characterized in that they include amphiphilic polypeptides and fragments thereof within a particular molecular weight range so that, as described herein, the materials display one or more desirable characteristics. In some embodiments, provided technologies for selecting, designing, and/or producing amphiphilic polypeptide materials as described herein include selection, design and/or production of amphiphilic polypeptide materials within a particular molecular weight range, so that materials having one or more desired (e.g., pre-determined desired) characteristics are provided. In some embodiments, for example, a response of provided amphiphilic polypeptides materials to an applied compression, expansion, or deformation is directly correlated to a molecular weight of its amphiphilic polypeptides and/or fragments thereof. In some embodiments, when the molecular weight of amphiphilic polypeptides and/or fragments which comprise an amphiphilic polypeptide material increases, a compressive modulus for the material increases. In some embodiments, when the molecular weight of amphiphilic polypeptides and/or fragments which comprise an amphiphilic polypeptide material increases, a shear stress value for the material increases. In some embodiments, when the molecular weight of amphiphilic polypeptides and/or fragments which comprise an amphiphilic polypeptide material increases, a pull out strength for the material increases.

In some embodiments, provided amphiphilic polypeptide materials display anisotropy. In some embodiments, provided amphiphilic polypeptide materials display anisotropy with respect to mechanical properties. In some embodiments, provided amphiphilic polypeptide materials are characterized in that certain properties of such materials are dependent according to crystal direction or with alignment of a crystal plane.

In some embodiments, provided amphiphilic polypeptide materials are characterized by a porosity. In some embodiments, provided amphiphilic polypeptide materials have a porosity between about 0.5% and about 98.5%.

In some embodiments, provided amphiphilic polypeptide materials are characterized by their size. In some embodiments, provided amphiphilic polypeptide materials are characterized by their dimensions, such height, width, depth, diameter, or combinations thereof. In some embodiments, provided amphiphilic polypeptide materials are characterized by their volume.

In some embodiments, provided amphiphilic polypeptide materials are or form blocks having dimensions that are only limited according to an availability of materials and/or the volume of a template, mold, or substrate that is used to form these materials.

In some embodiments, provided amphiphilic polypeptide materials are blocks or monoliths that characterized as continuous in that they are or comprise a single piece of material. In some embodiments, provided amphiphilic polypeptide materials are macrostructures that are both solid and continuous throughout their full size and/or volume.

In some embodiments, provided amphiphilic polypeptide materials are such that they can exist in any shape that is desired. In some embodiments, provided amphiphilic polypeptide materials assume a shape of a substrate, such as a mold or a template. In some embodiments, provided amphiphilic polypeptide materials are in a desirable shape because they were molded using a cast, a frame, a form, a mold, a template during processing. In some embodiments, for example, provided amphiphilic polypeptide materials have assumed a desired shape after being added, deposited, introduced, and/or poured into a template, mold, or onto a substrate and molded.

In some embodiments, provided amphiphilic polypeptide materials possess a particular shape because the materials were machined into that shape. In some embodiments, provided amphiphilic polypeptide materials are machined using standard machine tools, tooling, and methods. In some embodiments, provided amphiphilic polypeptide materials are mechanically manipulated, for example, using machine and/or hand tools.

In some embodiments, provided amphiphilic polypeptide materials comprise an exposed surface. In some embodiments, an exposed surface of provided amphiphilic polypeptide materials is approximately flat.

In some embodiments, provided amphiphilic polypeptide materials have a surface roughness that is in the order of only few nm. In some embodiments, provided amphiphilic polypeptide materials have a surface roughness that is characterized by small deviations so that such a surface enables integration of electronic component. In some embodiments, for example, provided amphiphilic polypeptide materials have an RMS surface roughness of less than about 5 nm.

Provided amphiphilic polypeptide materials exhibit thermoplasticity. In some embodiments, provided amphiphilic polypeptide materials are susceptible to heat and pressure. In some embodiments, provided amphiphilic polypeptide materials characterized in that they can adopt at least two different states, a cold state and a heated state. In some embodiments, when heated provided amphiphilic polypeptide materials deform or bend with heat and pressure. In some embodiments, for example, when heated above about 60 °C, provided amphiphilic polypeptide materials bend or deform from a normal state when a pressure is applied. In some embodiments, without the addition of heat, such materials do not bend or deform.

In some embodiments, a surface of provided amphiphilic polypeptide materials are susceptible to heat and pressure. In some embodiments, a surface of provided amphiphilic polypeptide materials when embossed will exhibit a pattern from the embossing. In some embodiments, embossing a surface results in a pattern having a minimum feature size of about 1 nm.

In some embodiments, provided amphiphilic polypeptide materials comprise a pattern formed in or on its surface. In some embodiments, provided amphiphilic polypeptide materials comprise a nanopattern formed in or on its surface. The term "nanopatterned" as used herein refers to patterning provided on a surface having structural features of a size that can be appropriately measured on a nanometer scale, for example, between about a nanometer and a few microns are typical of the patterning used in accordance with the present disclosure. In some embodiments, provided amphiphilic polypeptide materials may be molded, machined, or otherwise fabricated as an optical device, including, for example, diffraction gratings, photonic crystals, optofluidic devices, waveguides, and the like. In some embodiments, a pattern or nanopattern in a surface of a provided amphiphilic polypeptide material was formed using methods known in the art, for example nanoimprinting. In some embodiments, nanoimprinting includes contacting a surface with a substrate having a pattern (e.g. a nanopattern or micropattern) formed therein. In some embodiments, a substrate's surface is formed using e-beam lithography.

In some embodiments, provided amphiphilic polypeptide materials are biocompatible.

In some embodiments, provided amphiphilic polypeptide materials contain, encompass, include, incorporate, or store additives, agents, and/or functional moieties. In some embodiments, provided amphiphilic polypeptide materials encapsulate, encompass, include, incorporate, and/or store additives, agents, and/or functional moieties within its material monolithic construct.

In some embodiments, provided amphiphilic polypeptide materials store macromolecules and/or biologically active molecules in its monolithic construct. In some embodiments, additives, agents, and/or functional moieties are evenly distributed though provided amphiphilic polypeptide materials. In some embodiments, additives, agents, and/or functional moieties are concentrated in a specific portion or area of provided amphiphilic polypeptide materials relative to the whole of such provided amphiphilic polypeptide materials. In some embodiments, additives, agents, and/or functional moieties are concentrated on a surface of such provided amphiphilic polypeptide materials.

In some embodiments, provided amphiphilic polypeptide materials include additives, agents, and/or functional moieties that are or comprise labile compounds. In some embodiments, additives, agents, and/or functional moieties are or comprise macromolecules and/or biologically active molecules. In some embodiments, additives, agents, and/or functional moieties that are biologically active have a biological function. In some embodiments, provided amphiphilic polypeptide materials that comprise biologically active/functional additives, agents, and/or functional moieties at the cellular level can be used or support functions such as attack, defense, prevention, and/or protection.

In some embodiments, provided amphiphilic polypeptide materials preserve the activity of encapsulated additives, agents, and/or functional moieties that are biologically active. In some embodiments, provided amphiphilic polypeptide materials are arranged and constructed so that when additives, agents, and/or functional moieties that are biologically active are added, encapsulated, encompassed, included, incorporated, and/or stored in in provided amphiphilic polypeptide materials, such materials are characterized in that the structure and/or biological activity of such additives, agents, and/or functional moieties is retained so that it is not materially degraded, reduced, and/or inhibited. In some embodiments, for example, when biologically active additives, agents, and/or functional moieties are encapsulated in an amphiphilic polypeptide material as described here the biologically active material retains its activity such that it is not materially degraded, reduced, and/or inhibited for up to a year.

In some embodiments, provided amphiphilic polypeptide materials are maintained outside 'normal' storage conditions. In some embodiments, normal storage conditions include about ambient temperature, pressure, and/or relative humidity. In some embodiments, when exposed to outside 'normal' storage conditions, provided amphiphilic polypeptide materials preserve the activity of encapsulated additives, agents, and/or functional moieties that are biologically active.

In some embodiments, provided amphiphilic polypeptide materials are biodegradable.

In some embodiments, provided amphiphilic polypeptide materials are characterized in that such materials decompose, degrade, delaminate, or dissolve. In some embodiments, silk fibroin-based materials are characterized in that such materials decompose, degrade, delaminate, or dissolve to release an additive, agent, and/or functional moiety.

In some embodiments, provided amphiphilic polypeptide materials are introduced *in vivo.* In some embodiments, provided amphiphilic polypeptide materials decompose, degrade, delaminate, or dissolve when present *in vivo.* In some embodiments, provided amphiphilic polypeptide materials decompose, degrade, delaminate, or dissolve without significant immunological response when present *in vivo.* In some embodiments, provided amphiphilic polypeptide materials exhibit predictable degradation kinetics. In some embodiments, provided amphiphilic polypeptide materials are resorbed *in vivo* and replaced with natural tissues.

In some embodiments, technologies and processes provided herein permit fabrication of amphiphilic polypeptide materials.

In some embodiments, methods of forming, manufacturing, providing, and/or preparing amphiphilic polypeptide materials include steps for transforming an amphiphilic polypeptide solution to an amphiphilic polypeptide material, i.e. a solid. In some embodiments, methods provided herein comprise forming an amphiphilic polypeptide material without preparing a hydrogel intermediate.

In some embodiments, methods of forming, manufacturing, providing, and/or preparing amphiphilic polypeptide materials include an intermediate gel formation step. In some embodiments, steps for transforming an amphiphilic polypeptide solution to a solid include a solution to gel to solid transformation. In some embodiments, methods provided herein comprise forming a hydrogel intermediate from an aqueous amphiphilic polypeptide solution. In some embodiments, a hydrogel intermediate forms an amphiphilic polypeptide material.

In some embodiments, mechanical properties of provided amphiphilic polypeptide materials are tunable through control of its fabrication process.

In some embodiments, methods comprise providing an amphiphilic polypeptide and/or fragments thereof.

In some embodiments, provided amphiphilic polypeptide materials are produced by all aqueous processing steps. In some embodiments, the present disclosure also provides methods of making and using amphiphilic polypeptide materials by biomimetic, all aqueous processing steps. In some embodiments, amphiphilic polypeptide materials that are generated by biomimetic process of natural amphiphilic polypeptide structures.

In some embodiments, methods comprise providing an amphiphilic polypeptide and/or fragments having an average molecular weight between about 100 Da and about 400 kDa.

Provided methods include providing an aqueous solution comprising an amphiphilic polypeptide comprising silk fibroin, referred to herein as an "aqueous amphiphilic polypeptide solution". In some embodiments, methods comprise providing, preparing, and/or manufacturing an amphiphilic polypeptide solution. In some embodiments, an amphiphilic polypeptide solution includes a solvent that is or comprises water. In some embodiments, methods of manufacturing provided amphiphilic polypeptide materials do not comprise, use, or require organic and/or non-aqueous solvents in their fabrication.

In some embodiments, methods include providing an aqueous amphiphilic polypeptide solution that comprises phosphate buffered saline (PBS), Dulbecco's Modified Eagle Medium (DMEM), other media, other buffers, or combinations thereof.

In some embodiments, methods include providing an aqueous amphiphilic polypeptide solution that is or comprises amphiphilic polypeptides having a concentration in a range between about 0.1 % and about 30 %. In some embodiments, methods include providing an aqueous amphiphilic polypeptide solution that is or comprises amphiphilic polypeptides having a concentration in a range between about 20 % and about 30 %.

In some embodiments, provided amphiphilic polypeptide materials are produced by dehydrating an aqueous amphiphilic polypeptide solution.

In some embodiments, methods provided herein comprise dehydrating an aqueous amphiphilic polypeptide solution. In some embodiments, water removal is controlled. In some embodiments, mechanical properties of provided amphiphilic polypeptide materials are tunable through control through dehydration (i.e. water removal).

In some embodiments, as noted above, methods of inducing a transformation from a solution to a solid does not include formation of a gel intermediate.

In some embodiments, methods of inducing a transformation from a solution to a solid (i.e. forming an amphiphilic polypeptide material) does not include a gelation step or preparing a hydrogel intermediate. In some embodiments, methods provided herein comprise dehydrating an aqueous amphiphilic polypeptide solution through controllably delaying a gel intermediate so that a solid forms without forming a hydrogel intermediate.

In some embodiments, methods of forming, manufacturing, providing, and/or preparing amphiphilic polypeptide materials include steps for transforming an amphiphilic polypeptide solution to a solid. In some embodiments, methods of forming, manufacturing, providing, and/or preparing amphiphilic polypeptide materials include steps of slowly dehydrating an amphiphilic polypeptide solution.

In some embodiments, transforming an aqueous polypeptide solution to a solid includes slowly dehydrating an aqueous solution, for example, by controlling an aqueous polypeptide solution's pH and its concentration. In some embodiments, removal of water from an aqueous amphiphilic polypeptide solution is controlled, for example, by: varying pH, by varying pressure, by varying temperature, or by varying exposure time. In some embodiments, method comprise inducing a transformation of a solution having a pH that is near neutral or about neutral.

In some embodiments, methods of forming, manufacturing, providing, and/or preparing amphiphilic polypeptide materials include steps of removing water from an amphiphilic polypeptide solution. In some embodiments, removing water includes dehydrating an amphiphilic solution at a temperature above about 0 °C. In some embodiments, removing water includes dehydrating an amphiphilic solution at a temperature above about 10 °C.

In some embodiments, after removing water from an amphiphilic solution additional water is removed at an elevated temperature. In some embodiments, an elevated temperature is between about 30 °C and about 60 °C.

In some embodiments, inducing a transformation from a solution to a solid comprises removing water from the solution at a temperature of about 10 °C to form a solid followed by removing water from the solid at a temperature of about 45 °C to form a material.

In some embodiments, methods of forming, manufacturing, providing, and/or preparing amphiphilic polypeptide materials include a biomimetic process. In some embodiments, methods comprise inducing a solution to solid transformation.

In some embodiments, provided amphiphilic polypeptide materials are characterized by a network of amphiphilic polypeptide globules. In some embodiments, globules are nano-globules. In some embodiments, globules have at least one dimension between about 5 nm and about 45 nm.

In some embodiments, amphiphilic polypeptide globules aggregate through interactions with one another. In some embodiments, amphiphilic polypeptide globules have at least one dimension between about 5 nm and about 30 nm and form interactions with one another so that they aggregate. In some embodiments, globules aggregate with one another so that they form an interlocking structure. In some embodiments, amphiphilic polypeptide globules aggregate to form interlocking structures of tens to hundreds of nanometers in size. In some embodiments, amphiphilic polypeptide globules interact with each other to form a dense network. In some embodiments, a globular packing density may affect mechanical properties of provided materials.

In some embodiments, methods of forming, manufacturing, providing, and/or preparing amphiphilic polypeptide materials include steps of slowly dehydrating an amphiphilic polypeptide solution to form a 25-30 % solution. In some embodiments, steps of slowly dehydrating are controlled at a pH of about 8 to prevent hydrogel formation. In some embodiments, a concentrated amphiphilic polypeptide solution is loaded into a mold and further dehydrated at above 0 °C. In some embodiments, a concentrated amphiphilic polypeptide solution is loaded into a mold and further dehydrated at above 10 °C. In some embodiments, any remaining water is removed by further dehydration at elevated temperatures, for example about 30 °C to about 60 °C.

In some embodiments, as noted above, methods of making an amphiphilic polypeptide material include transforming an aqueous polypeptide solution to a solid after forming an amphiphilic polypeptide gel intermediate. In some embodiments, methods of making an amphiphilic polypeptide material include a solution-gel-solid transition. In some embodiments, methods include transforming an aqueous polypeptide solution to a gel. In some embodiments, methods include dehydrating a gel to form a solid.

In some embodiments, present methods comprise inducing a sol-gel transition in an amphiphilic polypeptide solution.

In some embodiments, present methods comprise transitioning an amphiphilic polypeptide solution via a sol-gel transition to form an amphiphilic polypeptide gel. In some embodiments, transforming an aqueous polypeptide solution to a solid includes forming a amphiphilic polypeptide gel intermediate through means known in the art. In some embodiments, methods of transitioning from an amphiphilic polypeptide solution to an amphiphilic polypeptide gel, for example, include, a step of electrogelling, sonicating, vortexing, altering solution pH, altering solution temperature, exposing a solution to polar solvents, etc. In some embodiments, gels self-assemble. In some embodiments, an amphiphilic polypeptide gel may be formed via a sol-gel transition by any methods known to those of skill in the art and/or already reported in the literature.

In some embodiments, gelation via a sol-gel transition occurs when polymer chains of an amphiphilic polypeptide either chemically or physically crosslink. In some embodiments, cross-linking into networks is triggered by chemical reagents (e.g., cross-linkers) or physical stimulants (e.g., pH and/or temperature). In some embodiments, cross-linking comprises forming secondary structures, such as helical structures, beta structures, or combinations thereof.

In some embodiments, methods comprise tailoring crystallinity, for example, increasing a percentage of beta sheet content of provided amphiphilic polypeptide materials occurs *a priori,* during gel fabrication by regulating a sol-gel transition. In some embodiments, steps to increase crystallinity are known.

In some embodiments, methods comprise inducing anisotropy in provided amphiphilic polypeptide materials. In some embodiments, anisotropy is induced during a sol-gel transition, for example by applying an electric field. In some embodiments, methods comprise facilitating polymorphism.

In some embodiments, methods comprise inducing crystalline orientation. In some embodiments, methods comprise maximizing a length of repeating structures.

In some embodiments, an amphiphilic polypeptide gel is injected, inserted, placed, poured, pressed, and/or transferred to a mold and/or a substrate. In some embodiments, a mold and/or a substrate maintains and/or retains a defined shape for an amphiphilic polypeptide gel.

In some embodiments, present methods comprise inducing a gel to solid transition in an amphiphilic polypeptide gel.

In some embodiments, transforming an aqueous polypeptide solution to a solid includes providing an amphiphilic polypeptide gel. In some embodiments, methods of forming an amphiphilic polypeptide material include dehydrating an amphiphilic polypeptide gel.

In some embodiments, a gel-solid transition is achieved by removing free water from a gel. In some embodiments, a gel-solid transition is achieved by removing at least some freezing-bound water. In some embodiments, a gel-solid transition is achieved by fusing amphiphilic polypeptide gel particles into a solid amphiphilic polypeptide material through controlled evaporation and/or by dehydrating an amphiphilic polypeptide gel.

In some embodiments, dehydrating an amphiphilic polypeptide results in self-assembly of a solid amphiphilic polypeptide material. In some embodiments, self-assembling occurs by a combination of hydrogen-bonding between beta-sheet forming amino acids and hydrophobic collapse of the tails to yield micelles.

In some embodiments, dehydrating and forming a solid from a gel occurs at ambient conditions. In some embodiments, dehydrating requires time to remove water.

In some embodiments, dehydrating time for an amphiphilic polypeptide gel depends on its size and geometry. In some embodiments, larger gels results in longer dehydrating time. In some embodiments, gels having a geometry with low exposed surface area results in longer dehydrating time.

In some embodiments, dehydrating time for an amphiphilic polypeptide gel depends on concentration. In some embodiments, for example, low amphiphilic polypeptide concentration and higher solvent content results in longer dehydrating times.

In some embodiments, dehydrating may be accelerated by changing conditions, such as, for example, temperature, pressure, humidity, adding a flow of forced gas, increasing a flow-rate of a forced gas, etc. In some embodiments, gel-solid transition conditions are held constant, for example, a temperature is set and held constant through a gel-solid transition. In some embodiments, gel-solid transition conditions vary either randomly or according to a set ramp rate.

In some embodiments, a gel-solid transition is achieved by dehydrating an amphiphilic polypeptide gel at a temperature between about 0 °C and about 90 °C. In some embodiments, a gel-solid transition is faster at a higher temperature. In some embodiments, a gel-solid transition is achieved by dehydrating an amphiphilic polypeptide gel at room temperature.

In some embodiments, a gel-solid transition is achieved by dehydrating an amphiphilic polypeptide gel at a pressure. In some embodiments, a gel-solid transition is achieved by dehydrating an amphiphilic polypeptide gel at a pressure above atmospheric pressure. In some embodiments, a gel-solid transition is faster at higher pressure. In some embodiments, a gel-solid transition is achieved by dehydrating an amphiphilic polypeptide gel at atmospheric pressure.

In some embodiments, a gel-solid transition is achieved by dehydrating an amphiphilic polypeptide gel at a relative humidity between about 0 % and about 70%. In some embodiments, a gel-solid transition is faster at a lower humidity.

In some embodiments, a gel-solid transition is achieved by dehydrating an amphiphilic polypeptide gel by exposing such an amphiphilic polypeptide gel to a forced gas. In some embodiments, a gel-solid transition is faster when using a forced gas. In some embodiments, a gas is or comprises nitrogen, argon, carbon dioxide, compressed dried air, oxygen, neon, helium, among others known in the art.

In some embodiments, a gel-solid transition is achieved by dehydrating an amphiphilic polypeptide through sealing an amphiphilic polypeptide gel in a desiccator with a desiccant. In some embodiments, a desiccant is a hygroscopic substance that induces or sustains a dry state within its vicinity.

In some embodiments, a desiccant is or comprises alumina, aluminum amalgam, barium oxide, barium perchlorate, boric anhydride, calcium chloride (anhydrous), calcium oxide, calcium sulphate (anhydrous), copper (II) sulphate (anhydrous), magnesium amalgam, magnesium perchlorate (anhydrous), magnesium sulphate (anhydrous), phosphorus pentoxide, potassium (metal), potassium carbonate (anhydrous), silica gel, sodium (metal), sodium hydroxide, sodium potassium alloy, sodium sulfate (anhydrous), sulfuric acid (concentrated).

In some embodiments, provided amphiphilic polypeptide materials that are manufactured according to present methods include a time to fabricate that is less than 1 week.

In some embodiments, a gel-solid transition is achieved by dehydrating an amphiphilic polypeptide through formation of a liquid-liquid interface, where an amphiphilic polypeptide material forms by quasi-static removing of solvent by diffusion in a coagulation bath.

In some embodiments, steps of encapsulating, encompassing, including, incorporating, and/or storing occurs during/after a gel to solid transition. In some embodiments, at least one additives, agents, and/or functional moieties is encapsulated, encompassed, included, incorporated, and/or stored in an amphiphilic polypeptide material.

In some embodiments, methods of forming amphiphilic polypeptide materials as disclosed herein comprise encapsulating, encompassing, including, incorporating, and/or storing at least one additives, agents, and/or functional moieties. In some embodiments, an at least one additives, agents, and/or functional moieties is a biolabile compound, biologically active or functional compound. In some embodiments, an at least one additives, agents, and/or functional moieties is not a biolabile compound, for example, a porogen.

In some embodiments, steps of encapsulating, encompassing, including, incorporating, and/or storing occurs in solution and/or prior to a sol-gel transition. In some embodiments, at least one of additives, agents, and/or functional moieties is introduced in an amphiphilic polypeptide solution.

In some embodiments, methods of forming amphiphilic polypeptide materials as disclosed herein comprise encapsulating, encompassing, including, incorporating, and/or storing at least one additives, agents, and/or functional moieties. In some embodiments, methods comprise steps of encapsulating, encompassing, including, incorporating, and/or storing during a sol-gel transition. In some embodiments, methods comprise steps of encapsulating, encompassing, including, incorporating, and/or storing during a sol-gel transition occurs either before substantial gelation or after substantial gelation has occurred in an amphiphilic polypeptide solution. In some embodiments, steps of encapsulating, encompassing, including, incorporating, and/or storing occurs after a sol-gel transition. In some embodiments, at least one additives, agents, and/or functional moieties is encapsulated, encompassed, included, incorporated, and/or stored in an amphiphilic polypeptide gel.

In some embodiments, the present methods comprise manufacturing amphiphilic polypeptide materials in ambient conditions, condition such as for example, temperature, pressure, air/gas flow, and /or humidity, as described herein. In some embodiments, all aqueous and ambient processing conditions are biocompatible. In some embodiments, biocompatible processing is favorable so that biologically active materials may not be adversely affected during material formation.

In some embodiments, methods comprise tailoring crystallinity of provided amphiphilic polypeptide materials *a posteriori,* after solid formation with post-processing techniques known to those skilled in the art, for example heating, immersing in a polar solvent, etc.

In some embodiments, methods include steps that alter an amphiphilic polypeptide and/or a surface of an amphiphilic polypeptide, such as machining, manipulating with hand tools. In some embodiments, provided amphiphilic polypeptide materials are machinable. In some embodiments, provided amphiphilic polypeptide materials are characterized in that machining reduces their volume of relative to its initial volume. In some embodiments, machining reduces a volume provided amphiphilic polypeptide materials to form a structure having a definite shape.

In some embodiments, methods include steps that alter an amphiphilic polypeptide and/or a surface of an amphiphilic polypeptide, such as embossing, nanoimprinting. In some embodiments, a step of nanoimprinting or embossing changes surface features (e.g. imprint diffraction grating). In some embodiments, steps of imprinting or embossing include forming structures between a couple of nanometers to several hundred millimeters.

In some embodiments, anisotropy is used to tailor properties and/or mechanics of provided amphiphilic polypeptide materials. In some embodiments, anisotropy is induced using post-processing techniques. In some embodiments, provided methods include inducing anisotropy in provided amphiphilic polypeptide materials at a material drying stage (i.e. a gel-solid transition or solution to solid). In some embodiments, inducing anisotropy in provided amphiphilic polypeptide materials occurs through semi-confinement of a gel in non-water permeable environments, which results in preferential water evaporation.

In some embodiments, mechanical properties of provided amphiphilic materials are tunable. In some embodiments, tunable mechanical properties of provided amphiphilic materials, include for example brittleness, compressibility, crystallinity, ductility, elasticity, machinability, shear stress, etc. In some embodiments, mechanical properties of provided amphiphilic materials are tunable by varying its residual bound water content during formation. In some embodiments, materials are tunable by varying its density during formation. In some embodiments, materials are tunable by varying its polypeptide molecular weight during formation. In some embodiments, materials are tunable by varying its polypeptide solution concentration during formation. In some embodiments, materials are tunable by varying its solution pH during formation. In some embodiments, materials are tunable by gelation timing and conditions.

BRIEF DESCRIPTION OF THE DRAWING

The foregoing and other objects, aspects, features, and advantages of the present invention will become more apparent and better understood by referring to the following description taken in conjunction with the accompanying figures in which:

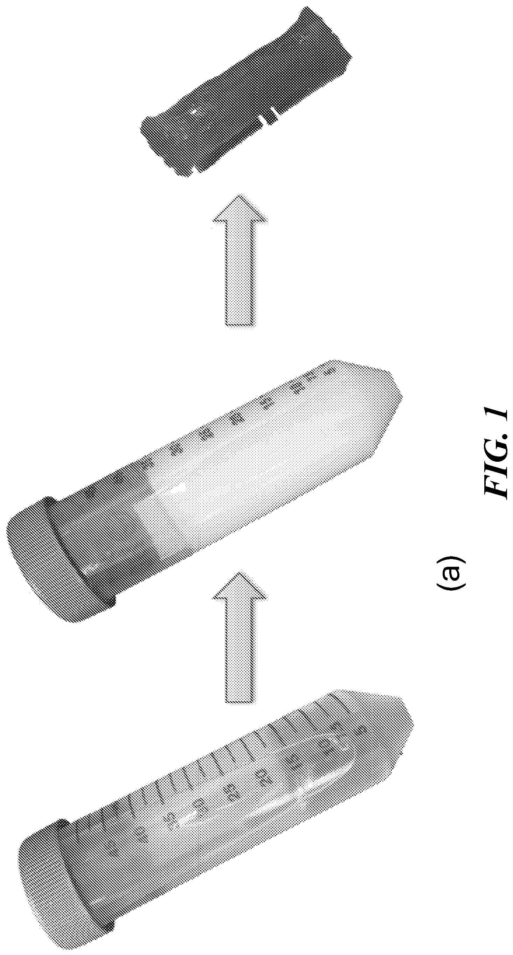

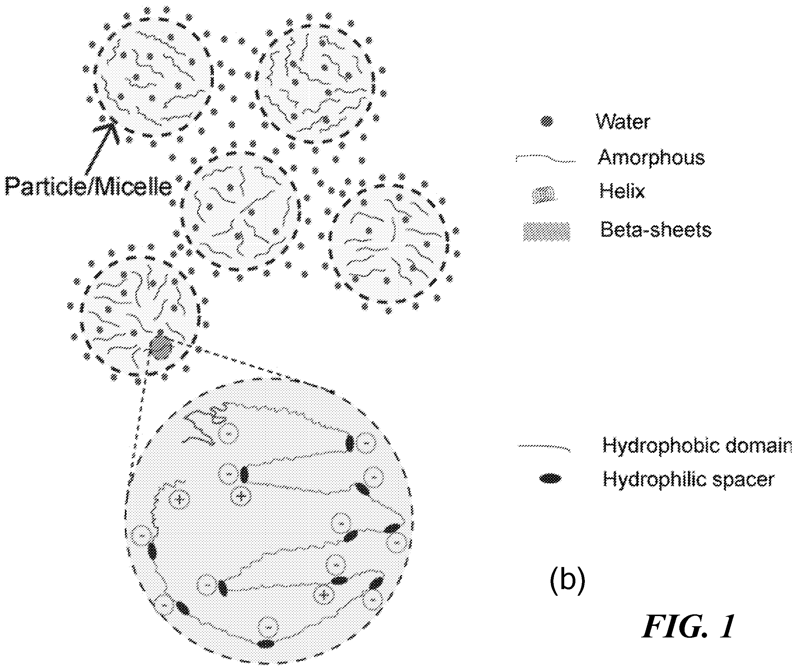

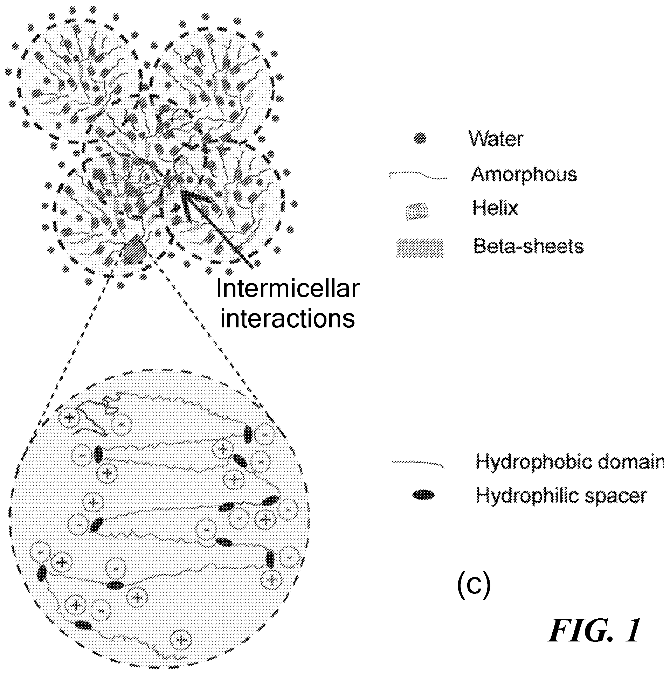

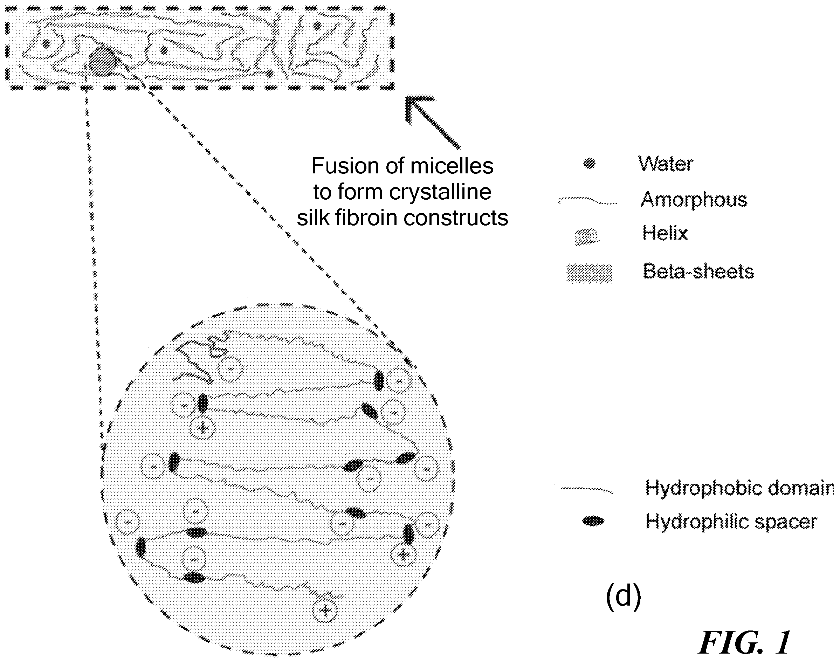

FIG. 1 shows an all-water process by which silk fibroin monoliths form. FIG. 1 at panel (a) shows a silk solution to sol-gel to gel-solid transition. A silk fibroin sol-gel transition is achieved through previously reported processes. A sol-gel transition imparts the formation of inter- molecular and intra-molecular crosslinking. Gels are then converted to solid monoliths by water removal through exposure to still or forced air, yielding a dense, and translucent, amber-like silk fibroin block. FIG. 1 at panel (b) shows a silk solution. FIG. 1 at panel (c) shows a sol-gel transition. FIG. 1 at panel (d) shows a gel-solid transition.

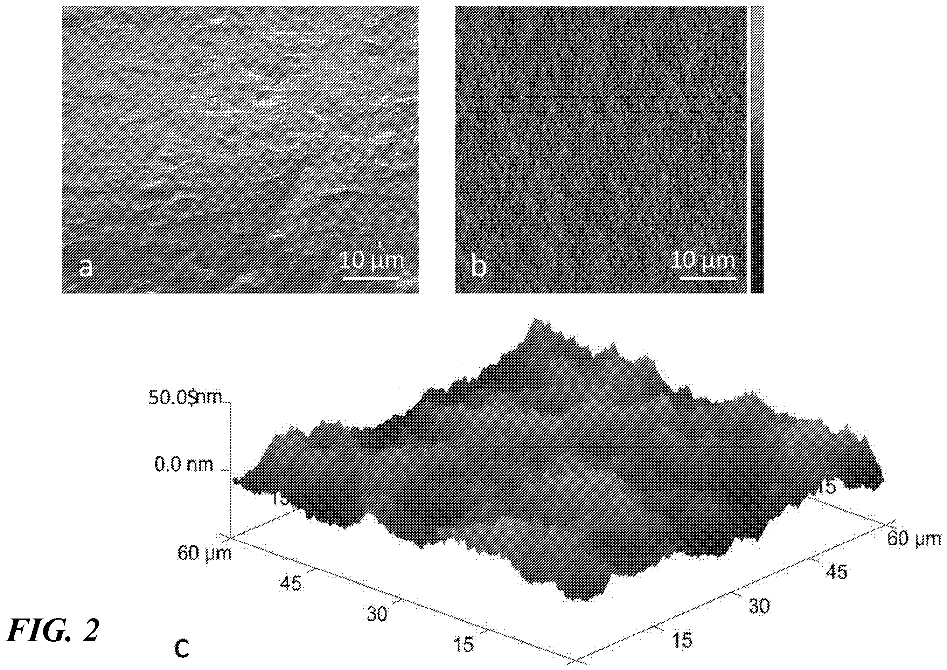

FIG. 2 shows a morphological characterization of silk fibroin monoliths. FIG. 2 at panel (a) shows an Scanning Electron Microscope (SEM) image of a silk fibroin monolith. FIG. 2 at panel (b) shows an Atomic Force Microscope (AFM) image of a silk fibroin monolith. FIG. 2 at panel (c) shows an AFM analyses of formation of smooth surfaces, with an RMS of few nm.

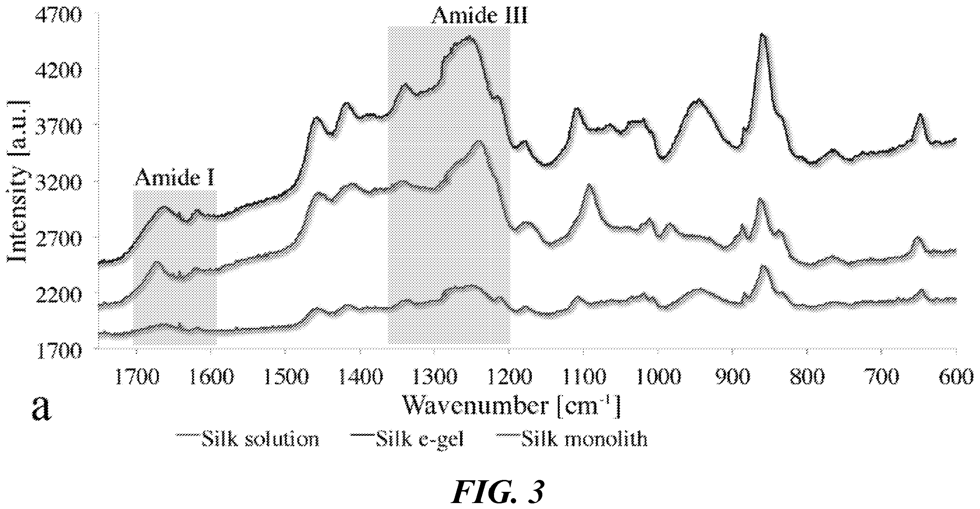

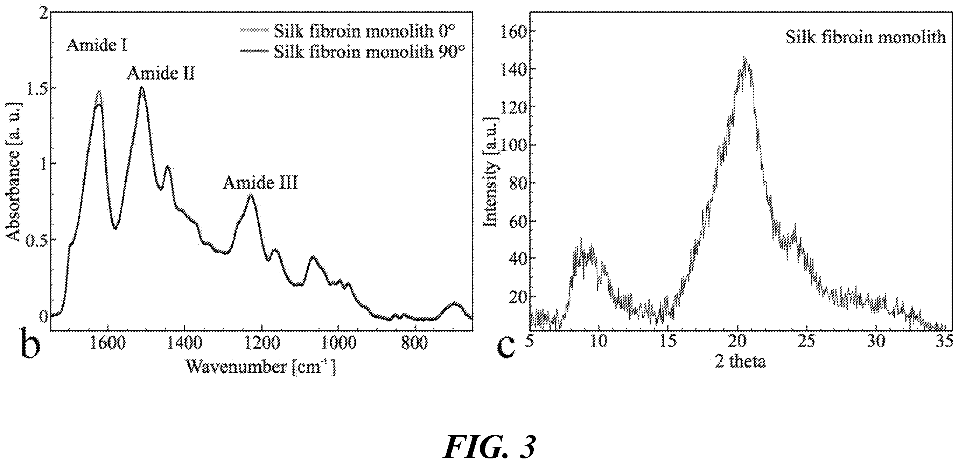

FIG. 3 shows spectroscopic characterization of silk fibroin monoliths obtained through electrogelation. FIG. 3 at panel (a) shows a μRaman analysis was used to monitor silk fibroin conformational changes during the process of monolith formation. FIG. 3 at panel (b) shows an ATR-FTIR with an Amide I peak centered at 1621 cm$^{-1}$ that were used to silk fibroin monoliths with highly crystalline, antiparallel beta-sheeted structures. FIG. 3 at panel (c) shows X-ray diffraction (peak around 8 and 21 2theta) measurements.

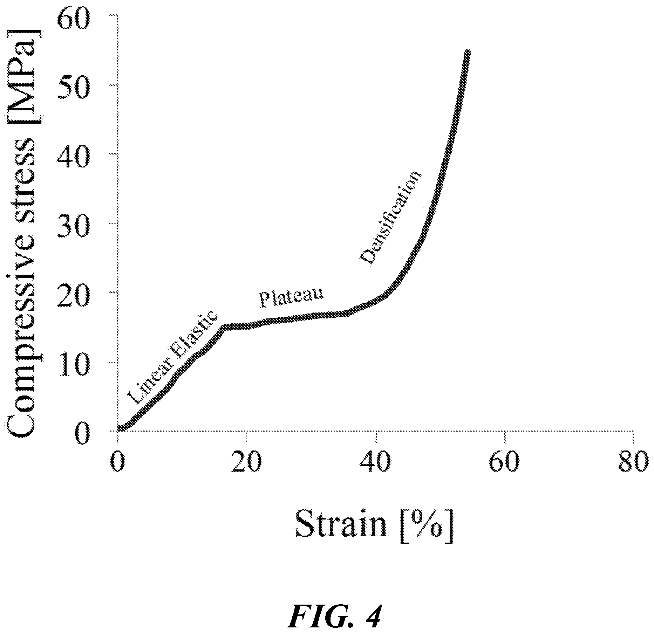

FIG. 4 shows mechanical properties of silk fibroin monoliths.. Cylindrical specimens were assessed through compression tests to evaluate the mechanical properties of silk monoliths obtained through electrogelation. The typical three regions of the densification behavior of a solid monolith were visible. As shown, these regions include: linear elastic, plateau, and densification.

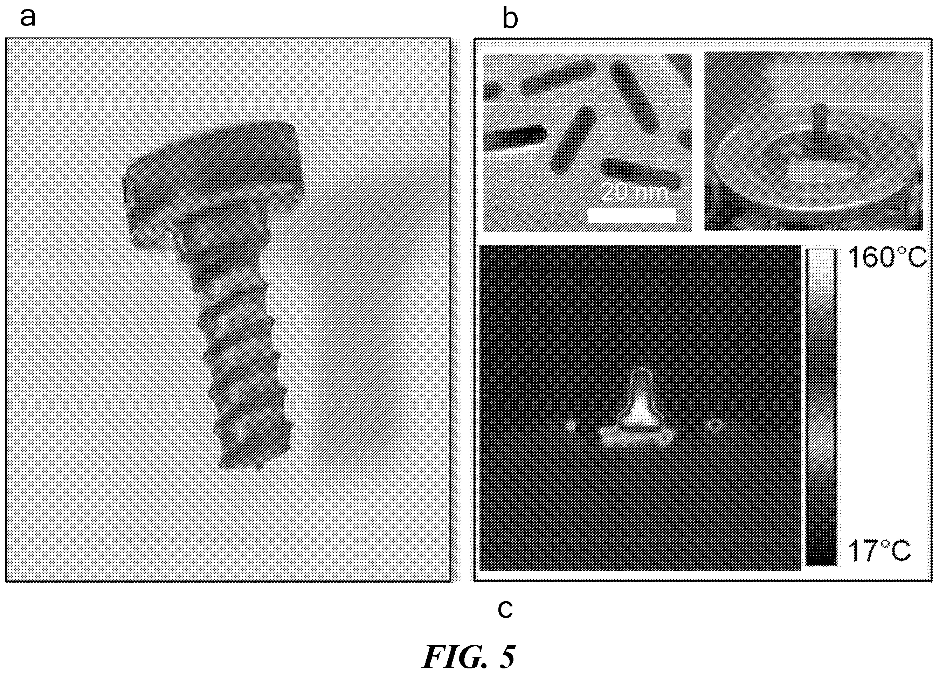

FIG. 5 shows a silk fibroin monolith and the effects of illumination. FIG. 5 at panel (a) and panel (b) shows a monolith of silk fibroin bulk-loaded with Au nanorods (AuNR) and machined into a 1 cm screw. FIG. 5 at panel (c) shows illumination of a monolith of silk fibroin bulk-loaded with Au nanorods (AuNR) and machined into a 1 cm screw with an LED.

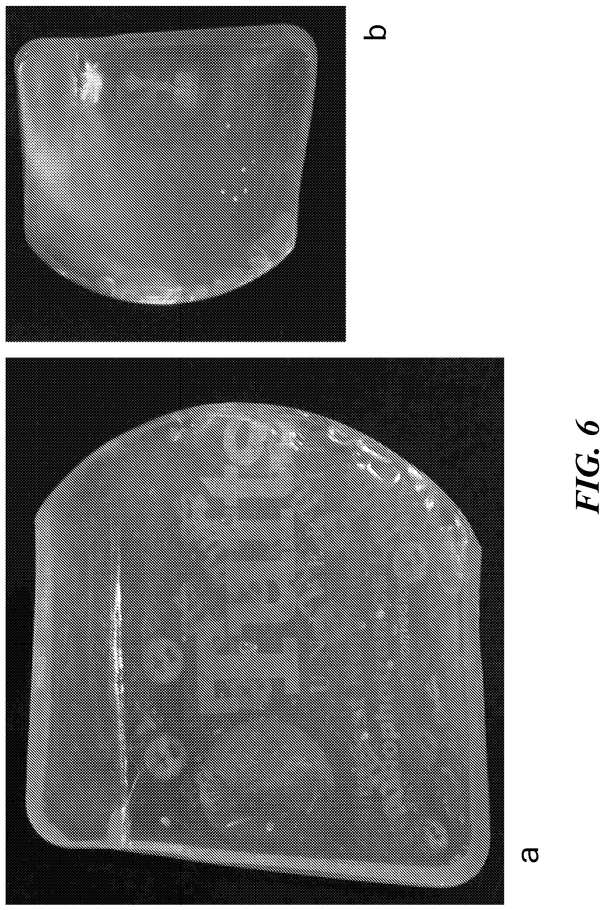

FIG. 6 shows a silk fibroin monolith and its surface thermoplasticity. FIG. 6 at panel (a) shows a patterned surface of the silk fibroin monolith. Panel (a) illustrates a silk fibroin monolith having a size of about 50 x 50 x 5 mm that has been patterned superficially during the gel-solid transition at room temperature through embossing at 100 kPa (method effective in the 10 Pa - 500 MPa range). The plastic deformation of the material is stable over time (up to 2 months and counting) and at temperature between -80 °C - 220 °C. FIG. 6 at panel (b) shows a surface of the silk fibroin monolith opposite to the patterned surface exhibiting the monolith's transparency/optical property.

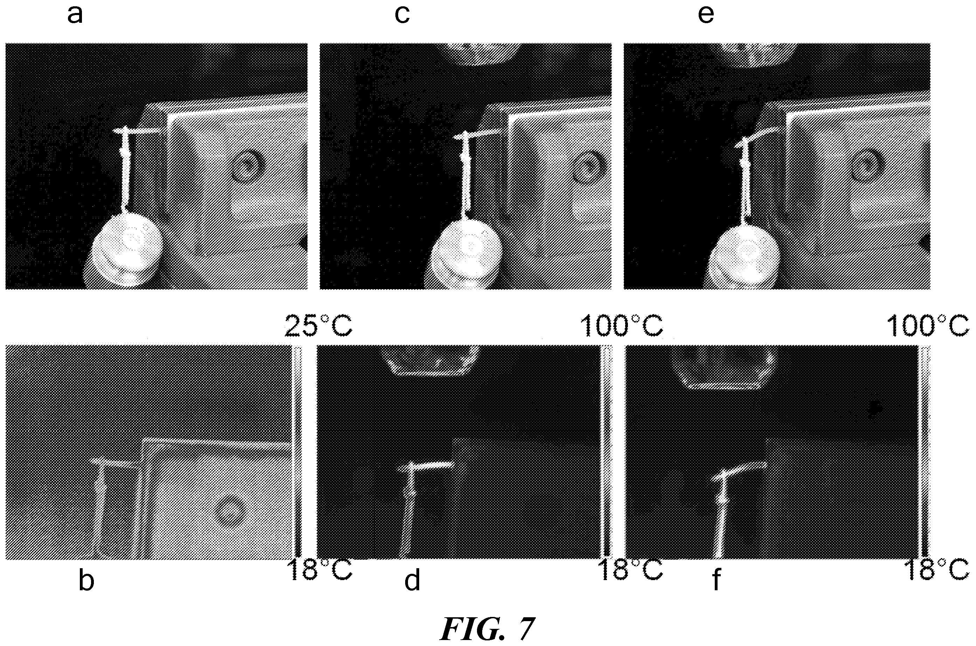

FIG. 7 shows a silk fibroin monolith and its bulk thermoplasticity. FIG. 7 at panel (a) shows a photographic image of a silk fibroin pin (⊘≅1 mm, l=60 mm) that has been gripped on one of its extremities. The opposite end of the pin carries a load of 200 g. FIG. 7 at panel (b) shows an infrared thermography (IRT) image of the pin corresponding to the photograph of panel (a). FIG. 7 at panel (c) shows a photographic image of a silk fibroin pin (⊘≅1mm, l=60 mm) that has been gripped on one of its extremities. The opposite end of the pin carries a load of 200 g. The pin is heating with a heat gun. FIG. 7 at panel (d) shows infrared thermography (IRT) image of the pin corresponding to the photograph of panel (c). FIG. 7 at panel (e) shows a photographic image of a silk fibroin pin (⊘≅1 mm, l=60 mm) that has been gripped on one of its extremities. The opposite end of the pin carries a load of 200 g. The pin is heating with a heat gun. FIG. 7 at panel (f) shows infrared thermography (IRT) image of the pin corresponding to the photograph of panel (e).

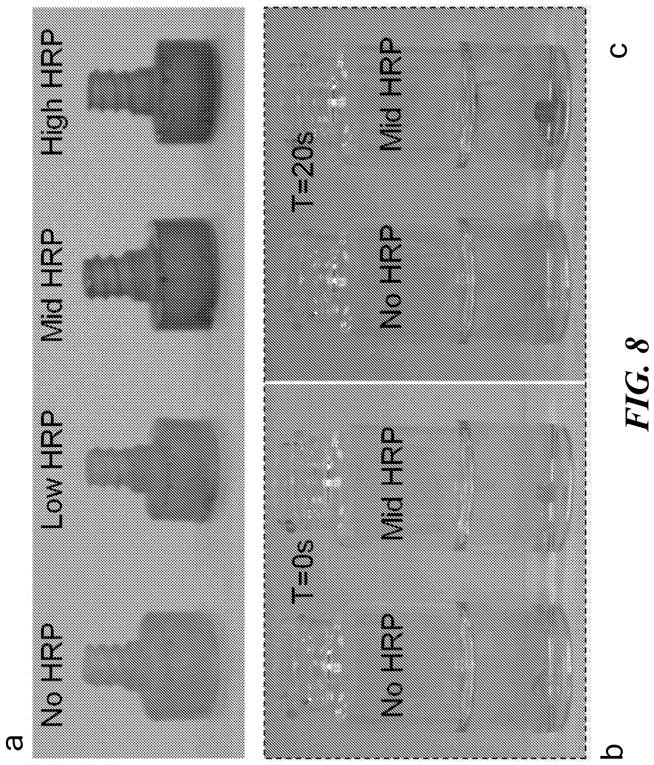

FIG. 8 shows silk fibroin monoliths and their ability to preserve and release heat-labile molecules. FIG. 8 at panel (a) shows a photographic image of four silk fibroin monoliths machined into screws. FIG. 8 at panel (a) at the far left and annotated "No HRP", shows a silk fibroin monolith machined into a screw in which no horse radish peroxidase ("HRP") has been bulk loaded into the monolith. FIG. 8 at panel (a) at second from the left and annotated "Low HRP", shows a silk fibroin monolith machined into a screw in which 0.2 U/10 mg$_{silk}$ HRP has been bulk loaded into the monolith. FIG. 8 at panel (a) at second from the right and annotated "Mid HRP", shows a silk fibroin monolith machined into a screw in which 1 U/10 mg$_{silk}$ HRP has been bulk loaded into the monolith. FIG. 8 at panel (a) at the far right and annotated "High HRP", shows a silk fibroin monolith machined into a screw in which 2 U/10mg$_{silk}$ HRP has been bulk loaded into the monolith. FIG. 8 at panel (b) shows a photographic images of silk fibroin monoliths machined into screws and submerged in a solvent. The images show the screws at T=0 s or concurrent with submersion. FIG. 8 at panel (b) on the left and annotated "No HRP" shows a silk fibroin monolith machined into a screw in which no HRP has been bulk loaded into the monolith and submerged in a solvent, T=0 s. FIG. 8 at panel (b) on the right and annotated "Mid HRP" shows a silk fibroin monolith machined into a screw in which 1U/10 mg$_{silk}$ HRP has been bulk loaded into the monolith and submerged in a solvent, T=0 s. FIG. 8 at panel (c) shows a photographic images of silk fibroin monoliths machined into screws and submerged in a solvent. The images show the screws at T=20 s. FIG. 8 at panel (c) on the left and annotated "No HRP" shows a silk fibroin monolith machined into a screw in which no HRP has been bulk loaded into the monolith and submerged in a solvent, T=20 s. FIG. 8 at panel (c) on the right and annotated "Mid HRP" shows a silk fibroin monolith machined into a screw in which 1 U/10 mg$_{silk}$ HRP has been bulk loaded into the monolith and submerged in a solvent, T=20 s.

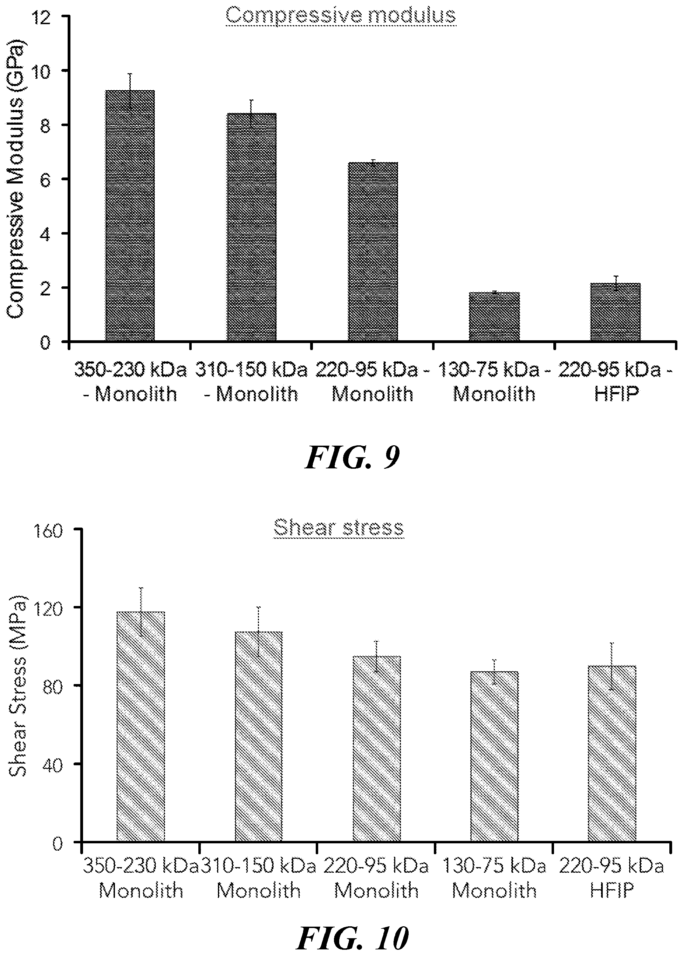

FIG. 9 shows a graph comparing the compressive modulus for monoliths of different molecular weights prepared in aqueous conditions and in hexafluoroisopropanol, HFIP.

FIG. 10 shows a graph comparing the shear stress for monoliths of different molecular weights prepared in aqueous conditions and in hexafluoroisopropanol, HFIP.

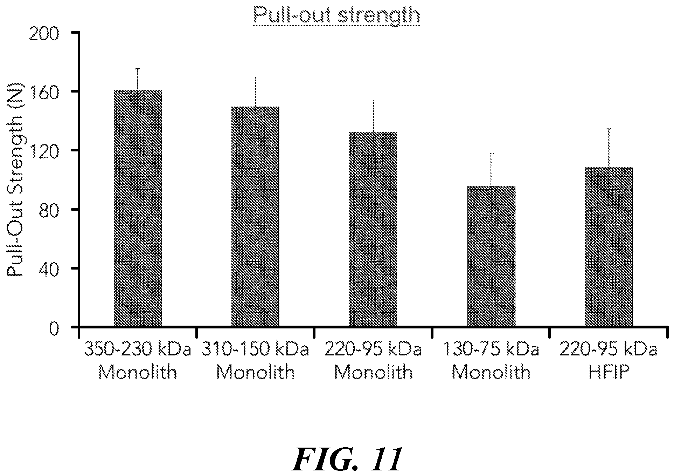

FIG. 11 shows a graph comparing the pull-out strength for monoliths of different molecular weights prepared in aqueous conditions and in hexafluoroisopropanol, HFIP.

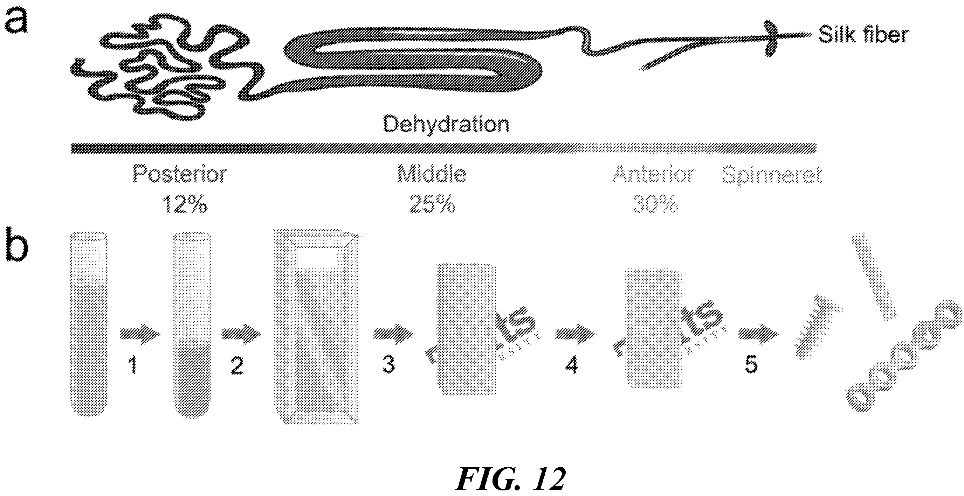

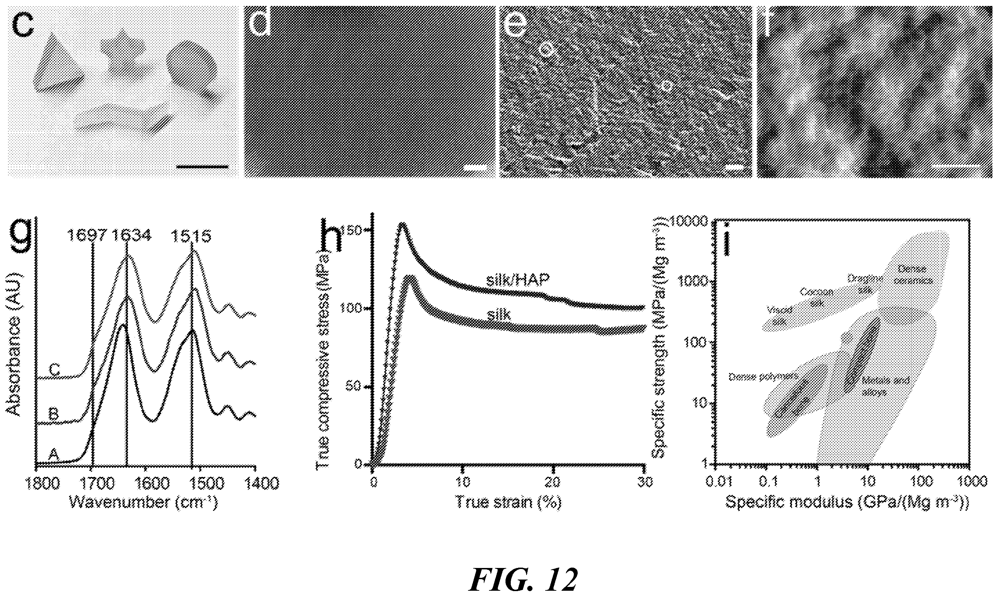

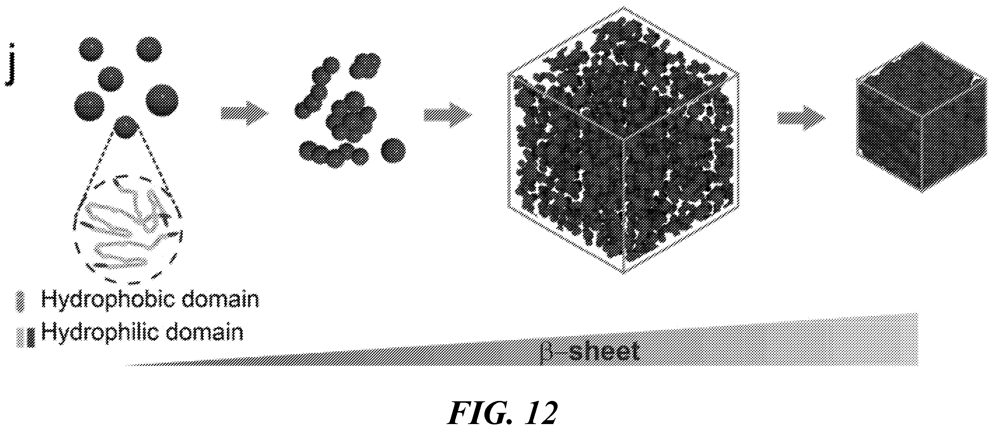

FIG. 12 shows a schematic illustration of the fabrication process and characterization of the biomimetic silk materials. FIG. 12 at panel (a) shows an illustration of spinning duct of silkworm. FIG. 12 at panel (b) shows solid silk materials that were obtained by the biomimetic gradual dehydration of silk aqueous solution. (1) Concentrate silk solution to 25-30% (wt/wt). (2) Inject concentrated silk solution to silk blank molds. (3) Remove water by forced air flow at 10°C for 3-4 days. (4) Remove the remaining water by drying in fume hood for 4 days and then in an oven at 45°C for 4 days. (5) Machine silk blank into orthopedic devices of desired geometry. FIG. 12 at panel (c) shows various shapes machined from biomimetic silk materials (scale = 1 cm). FIG. 12 at panel (d), (e) show SEM images and FIG. 12 at panel (f) shows AFM images of the cross section of a silk rod of 1.5 mm diameter (scale: 40 $\mu$m) in FIG. 12 at panel (d), 2 $\mu$m in FIG. 12 at panel (e), and 200 nm in FIG. 12 at panel (f). FIG. 12 at panel (g) shows FTIR spectra of silk materials (curve A: freeze-dried concentrated silk solution; curve B: silk materials obtained after dehydration at 10 °C and room temperature; and curve C: silk blanks before machining. FIG. 12 at panel (h) shows a true stress-strain curve of biomimetic silk materials. FIG. 12 at panel (i) shows a comparison of specific strength and modulus of the biomimetic silk material with natural silk materials, bone, polymer, ceramic and metal/alloys. (Adapted with permission from nature publishing group, see U. G. K. Wegst, 14 Nature Mater., 23 (2015)). The yellow ellipse represents the silk and silk/HAP materials in this study. FIG. 12 at panel (j) shows a structural evolution of silk molecules via a biomimetic fabrication process as disclosed herein.

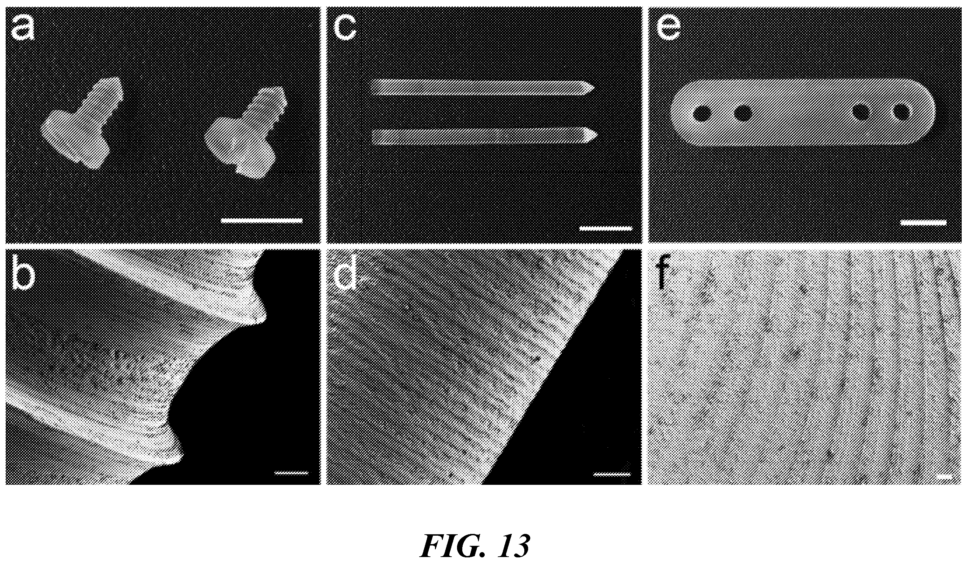

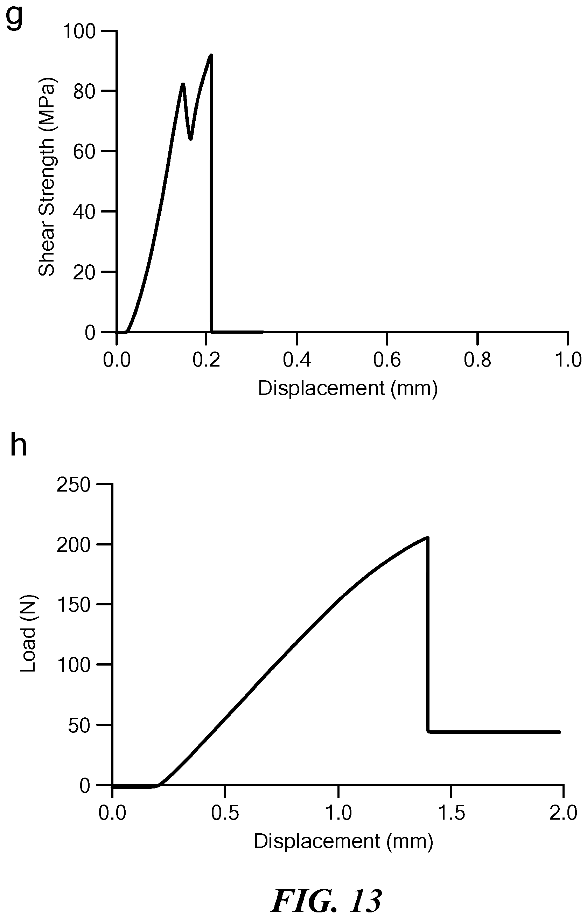

FIG. 13 shows silk orthopedic devices. FIG. 13 at panel (a) shows a bone screw with HA thread. (Scale = 5 mm). The major diameter of the screw was ~ 1.8 mm and pitch was 600 $\mu$m. FIG. 13 at panel (b) shows an SEM image of cortical bone screw (scale = 100 $\mu$m). FIG. 13 at panel (c) shows silk IM rods of 1.5 mm diameter, scale = 5 mm. FIG. 13 at panel (d) shows an SEM image of silk IM rods, scale = 5 m. FIG. 13 at panel (e) shows four-hole silk plate of 29 mm length and 2 mm thickness (scale = 5 mm). FIG. 13 at panel (f) shows an SEM image of milled surface of silk plate (scale = 100 $\mu$m). FIG. 13 at panel (g) shows a representative shear strength-displacement curve of 1.5 mm diameter silk pin by double lap-shear mechanical test. FIG. 13 at panel (h) shows representative load-displacement curve of the four-hole silk plate of 29 mm length and 2 mm thickness by four-point bending test. FIG. 13 at panel (i) shows a model of the assembly process of the biomimetic silk materials. The silk molecules form oligomeric aggregates in concentrated silk solution. Dehydration leads to the condensation and assembly of the silk molecules into densely packed solid materials.

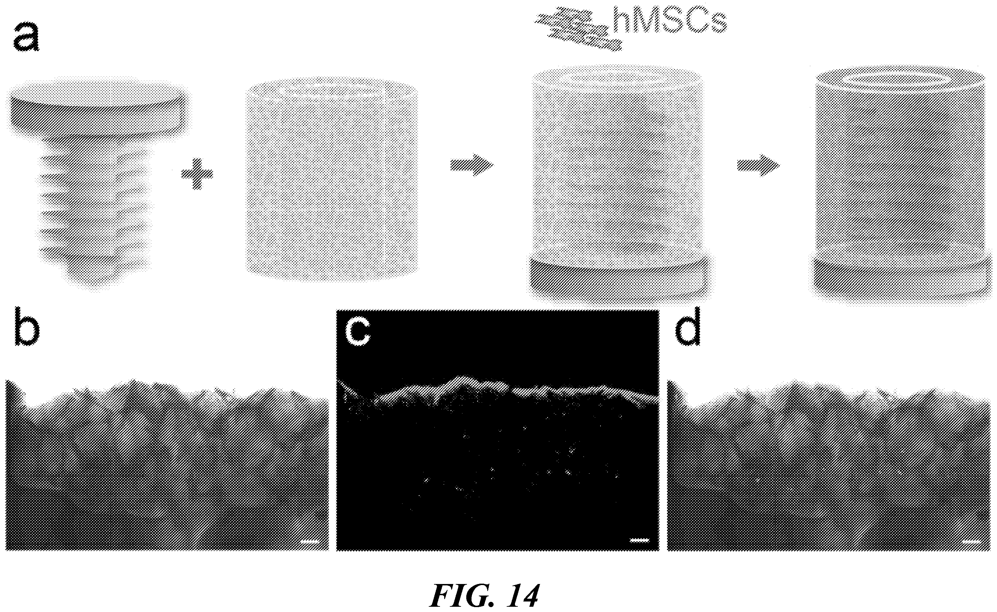

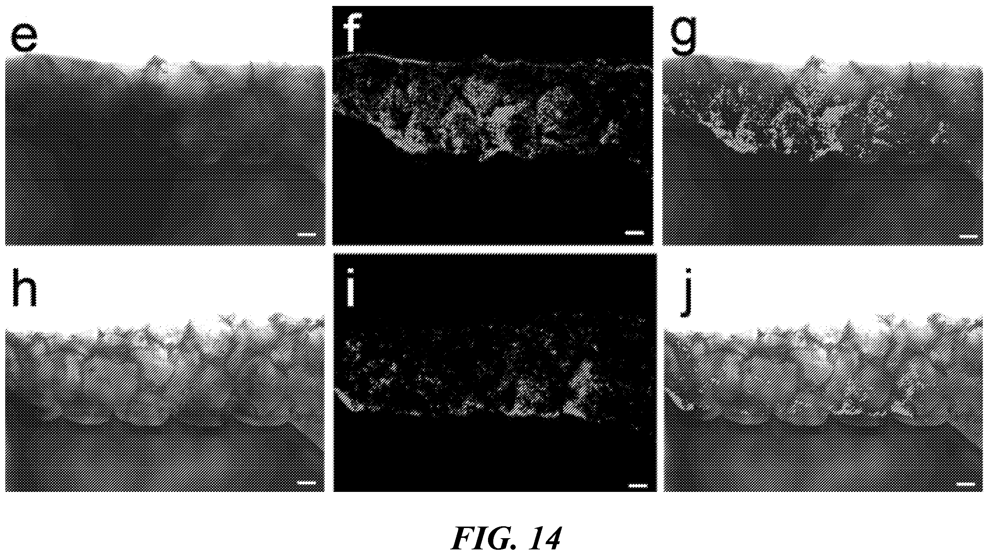

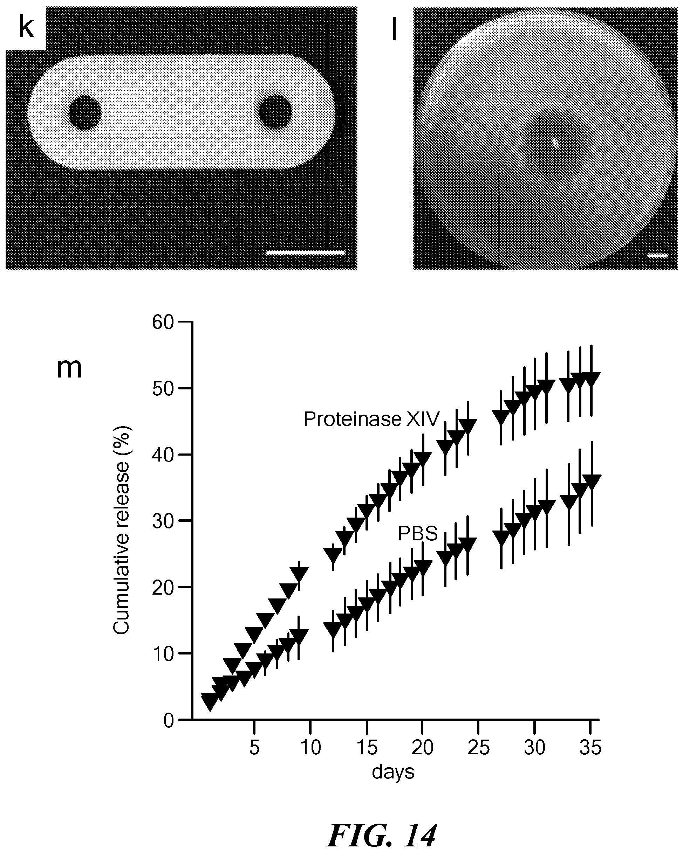

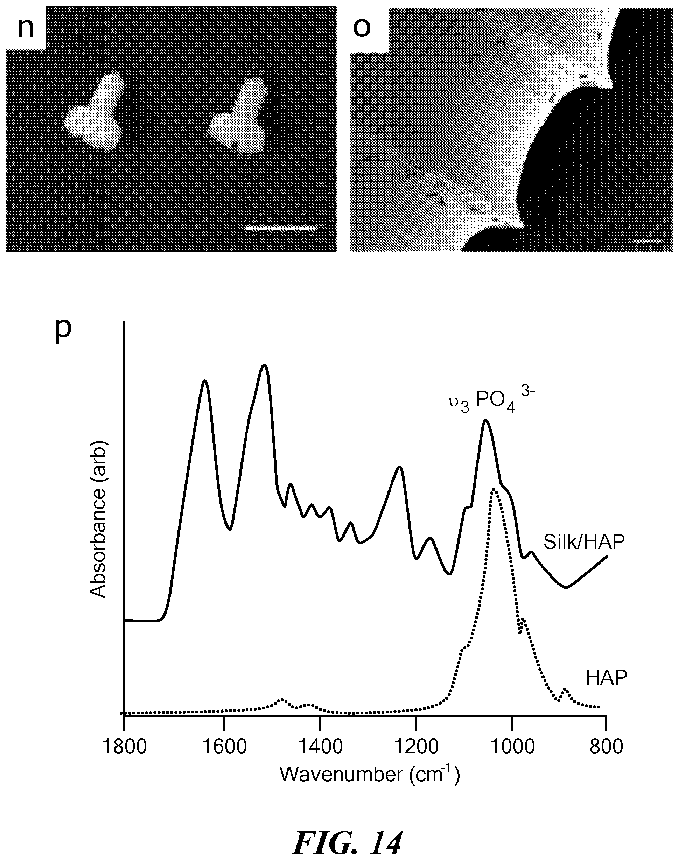

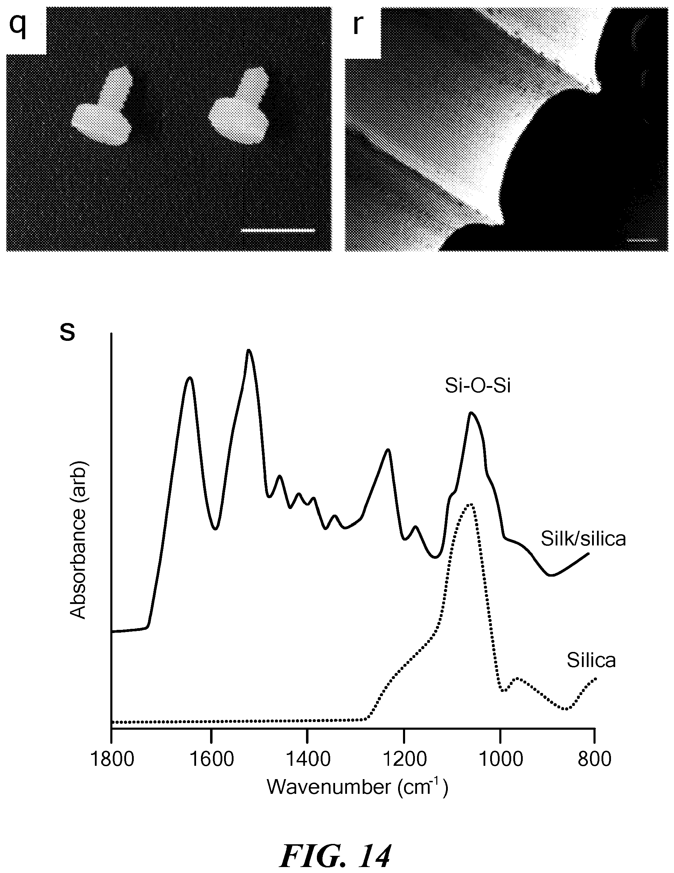

FIG. 14 shows functionalized silk-based orthopedic devices. FIG. 14 at panel (a) - panel (j) shows in vitro differentiation of hMSCs induced by BMP-2/P24 incorporated silk screws. FIG. 14 at panel (a) shows a schematic illustrating the in vitro cell culture setup. BMP-2/P24 incorporated silk screws were inserted into a tubular silk sponge with pore size of 400-500 $\mu$m and hMSCs were seeded in the sponge. Osteogenic differentiation of hMSCs was assessed with OsteoImage fluorescent staining for HAP after 6-week culture in osteogenic medium. FIG. 14 at panel (b), panel (e), and panel (h) show transmitted light, FIG. 14 at panel (c), panel (f), and panel (i) show fluorescent images. FIG. 14 at panel (b) - panel (d) show overlay images demonstrated the mineralization pattern in osteogenic control. FIG. 14 at panel (e) - panel (g) show overlay images demonstrated the mineralization pattern in BMP-2. FIG. 14 at panel (h) - panel (j) show overlay images demonstrated the mineralization pattern in P24 groups. (Scale = 200 $\mu$m). FIG. 14 at panel (k) shows bone plate containing 5% (wt/wt) ciprofloxacin (scale = 5 mm). FIG. 14 at panel (l) shows a clear zone of inhibition formed around ciprofloxacin releasing silk rods against *S. aureus* cultures (scale = 1mm). FIG. 14 at panel (m) shows the cumulative release of ciprofloxacin from ciprofloxacin incorporated silk rod over 36 days. FIG. 14 at panel (n) and panel (q) shows the major diameter of the screw was ~1.8 mm. FIG. 14 at panel (o) and panel (r) shows optical images. FIG. 14 at panel (p) and panel (s) shows SEM images. FIG. 14 at panel (n) - panel (p) shows FTIR spectra of the silk/HAP (90/10 wt/wt) and FIG. 14 at panel (q) - panel (s) shows silk/silica (90/10 wt/wt) composite screw with HA bone thread. Scale = 5 mm in (FIG. 14 at panel (n) and panel (q) and 100 $\mu$m in FIG. 14 at panel (o) and panel (r).

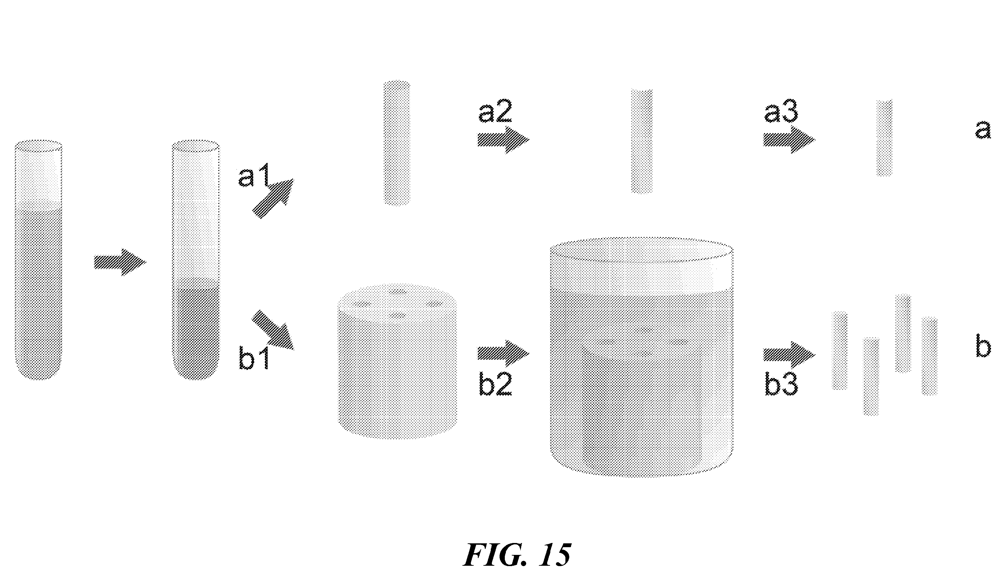

FIG. 15 shows a schematic illustration of alternative processes of fabrication silk block materials. FIG. 15 at path (a) shows a pre-gelation process. The silk blanks can be machined into pins, screws and plate. FIG. 15 at path (b) shows a fast dehydration process.

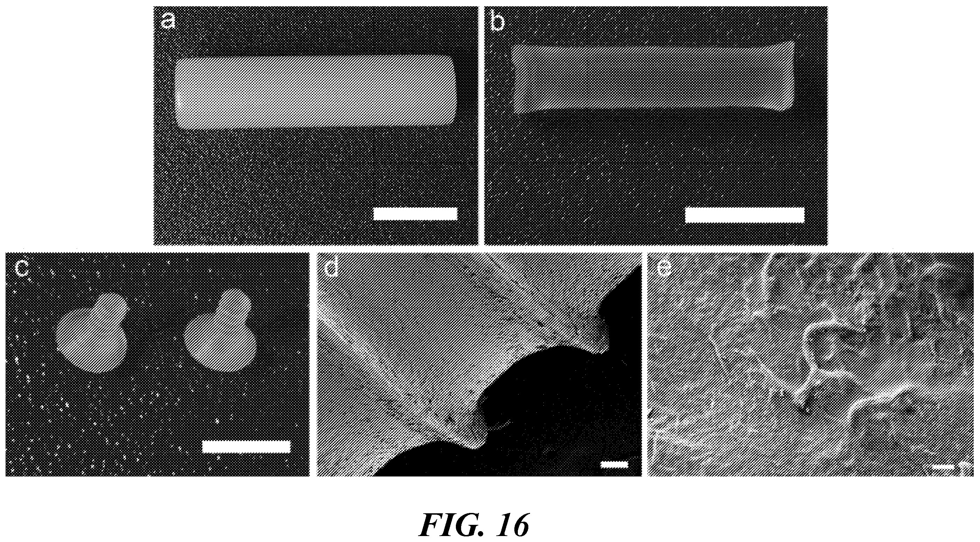

FIG. 16 shows a pregelation process to fabricate silk blanks. FIG. 16 at panel (a) shows a hydrogel obtained from 25% SF solution after incubation at room temperature for about 7 days (scale = 5 mm). FIG. 16 at panel (b) shows solid silk blank after hydrogel dehydration (scale = 5 mm). FIG. 16 at panel (c) shows screws with HA bone screw threads (scale = 5 mm). (d) SEM image of the screw (scale = 100 $\mu$m). FIG. 16 at panel (e) shows SEM image showing the screw surface morphology (scale = 2 $\mu$m).

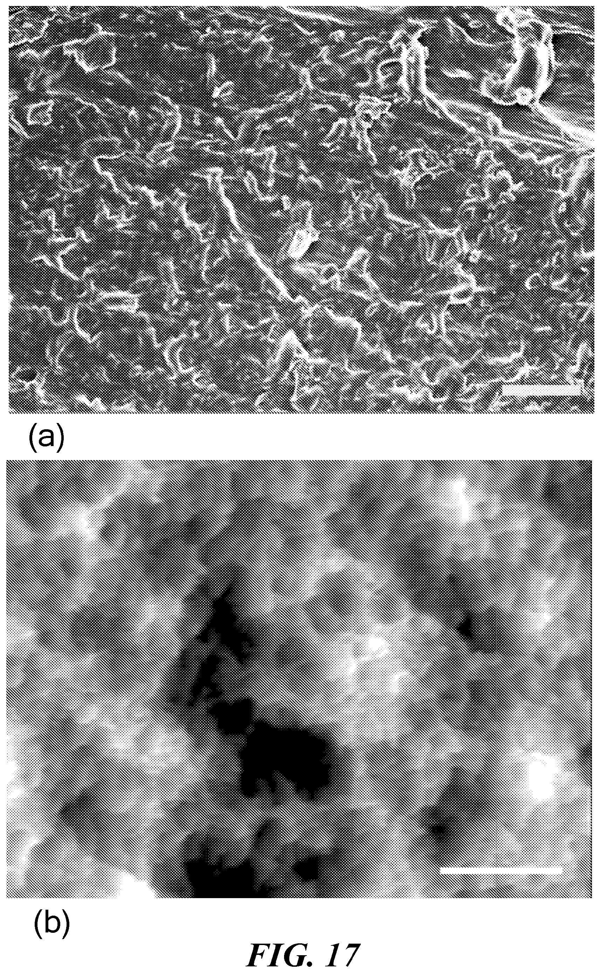

FIG. 17 at panel (a) shows an SEM image of a surface of a silk bone screw. (Scale = 10 $\mu$m). FIG. 17 at panel (b) shows an AFM image of the cross section of silk pins machined from silk blanks obtained by fast dehydration process. Scale = 200 nm

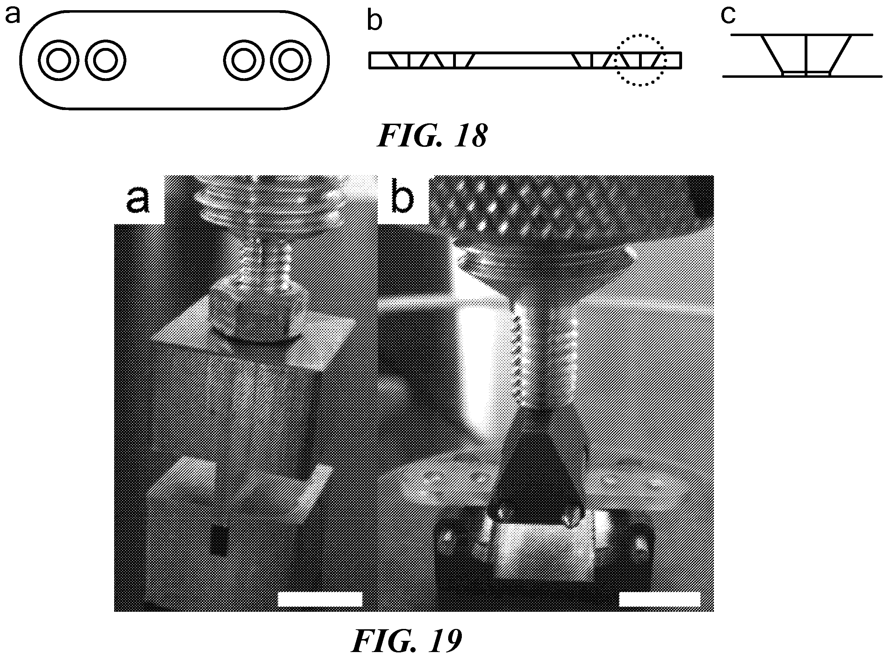

FIG. 18 shows a drawing of 4-hole plates (29 mm length, 7 mm width, 2 mm thickness, 1.6/3 mm diameter holes placed 3.5 mm from the end and 4.5 mm apart). FIG. 18 at panel (a) shows a top view of a 4-hole plate. FIG. 18 at panel (b) shows a side view of a 4-hole plate. FIG. 18 at panel (c) shows a magnified view of the holes of a 4-hole plate.

FIG. 19 shows mechanical testing fixtures for silk orthopedic devices. FIG. 19 at panel (a) shows a top and bottom of a shear strength fixture. FIG. 19 at panel (b) shows a plate bending fixture. (Scale = 1 cm).

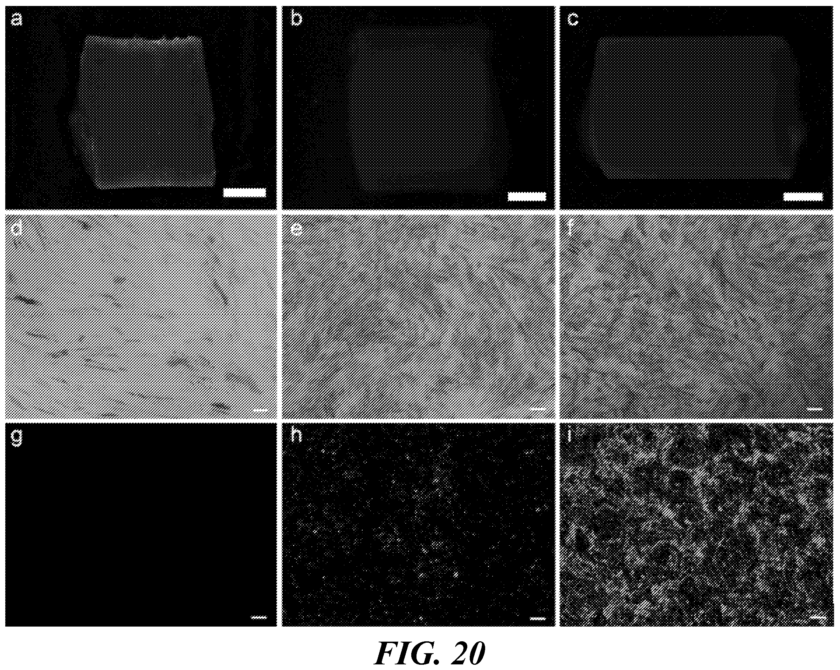

FIG. 20 shows immunostaining of BMP-2 incorporated in silk rods and bioactivity of BMP-2 released from silk rods. FIG. 20 at panel (a) shows BMP-2 incorporated silk rod. FIG. 20 at panel (b) shows BMP-2 incorporated silk rod with only secondary antibody. FIG. 20 at panel (c) shows silk rod. (Scale = 500 $\mu$m). FIG. 20 at panel (d) - panel (f) shows alkaline phosphatase staining. FIG. 20 at panel (g) - panel (i) OsteoImage staining of BMP-2 released form silk rods. FIG. 20 at panel (d) and FIG. 20 at panel (g) show growth medium control. FIG. 20 at panel (e) and panel (h) show osteogenic control. FIG. 20 at panel (f) and panel (i) show BMP-2 incorporated silk rods in osteogenic medium. (Scale = 100 $\mu$m). Enhanced ALP activity and hydroxyapatite formation in the presence of BMP2-incorporated silk rods demonstrated the biactivity of BMP2 released from silk rods. Scale = 500 $\mu$m in (a)-(c) and 100 $\mu$m in (d)-(f).

DEFINITIONS

In order for the present disclosure to be more readily understood, certain terms are first defined below. Additional definitions for the following terms and other terms are set forth throughout the specification.

In this application, unless otherwise clear from context, the term "a" may be understood to mean "at least one." As used in this application, the term "or" may be understood to mean "and/or." In this application, the terms "comprising" and "including" may be understood to encompass itemized components or steps whether presented by themselves or together with one or more additional components or steps. Unless otherwise stated, the terms "about" and "approximately" may be understood to permit standard variation as would be understood by those of ordinary skill in the art. Where ranges are provided herein, the endpoints are included. As used in this application, the term "comprise" and variations of the term, such as "comprising" and "comprises," are not intended to exclude other additives, components, integers or steps.

As used in this application, the terms "about" and "approximately" are used as equivalents. Any numerals used in this application with or without about/approximately are meant to cover any normal fluctuations appreciated by one of ordinary skill in the relevant art.

"Administration": As used herein, the term "administration" refers to the administration of a composition to a subject. Administration may be by any appropriate route. For example, in some embodiments, administration may be bronchial (including by bronchial instillation), buccal, enteral, interdermal, intra-arterial, intradermal, intragastric, intramedullary, intramuscular, intranasal, intraperitoneal, intrathecal, intravenous, intraventricular, mucosal, nasal, oral, rectal, subcutaneous, sublingual, topical, tracheal (including by intratracheal instillation), transdermal, vaginal and vitreal.

"Affinity": As is known in the art, "affinity" is a measure of the tightness with a particular ligand binds to its partner. Affinities can be measured in different ways. In some embodiments, affinity is measured by a quantitative assay. In some such embodiments, binding partner concentration may be fixed to be in excess of ligand concentration so as to mimic physiological conditions. Alternatively or additionally, in some embodiments, binding partner concentration and/or ligand concentration may be varied. In some such embodiments, affinity may be compared to a reference under comparable conditions (e.g., concentrations).

"Agent": As used herein, the term "agent" may refer to a compound or entity of any chemical class including, for example, polypeptides, nucleic acids, saccharides, lipids, small molecules, metals, or combinations thereof. As will be clear from context, in some embodiments, an agent can be or comprise a cell or organism, or a fraction, extract, or component thereof. In some embodiments, an agent is agent is or comprises a natural product in that it is found in and/or is obtained from nature. In some embodiments, an agent is or comprises one or more entities that is man-made in that it is designed, engineered, and/or produced through action of the hand of man and/or is not found in nature. In some embodiments, an agent may be utilized in isolated or pure form; in some embodiments, an agent may be utilized in crude form. In some embodiments, potential agents are provided as collections or libraries, for example that may be screened to identify or characterize active agents within them. Some particular embodiments of agents that may be utilized in accordance with the present disclosure include small molecules, antibodies, antibody fragments, aptamers, siRNAs, shRNAs, DNA/RNA hybrids, antisense oligonucleotides, ribozymes, peptides, peptide mimetics, small molecules, etc. In some embodiments, an agent is or comprises a polymer. In some embodiments, an agent is not a polymer and/or is substantially free of any polymer. In some embodiments, an agent contains at least one polymeric moiety. In some embodiments, an agent lacks or is substantially free of any polymeric moiety.

"Analog": As used herein, the term "analog" refers to a substance that shares one or more particular structural features, elements, components, or moieties with a reference substance. Typically, an "analog" shows significant structural similarity with the reference substance, for example sharing a core or consensus structure, but also differs in certain discrete ways. In some embodiments, an analog is a substance that can be generated from the reference substance by chemical manipulation of the reference substance. In some embodiments, an analog is a substance that can be generated through performance of a synthetic process substantially similar to (e.g., sharing a plurality of steps with) one that generates the reference substance. In some embodiments, an analog is or can be generated through performance of a synthetic process different from that used to generate the reference substance

"Amino acid": As used herein, the term "amino acid," in its broadest sense, refers to any compound and/or substance that can be incorporated into a polypeptide chain, e.g., through formation of one or more peptide bonds. In some embodiments, an amino acid has the general structure H2N-C(H)(R)-COOH. In some embodiments, an amino acid is a naturally-occurring amino acid. In some embodiments, an amino acid is a synthetic amino acid; in some embodiments, an amino acid is a D-amino acid; in some embodiments, an amino acid is an L-amino acid. "Standard amino acid" refers to any of the twenty standard L-amino acids commonly found in naturally occurring peptides. "Nonstandard amino acid" refers to any amino acid, other than the standard amino acids, regardless of whether it is prepared synthetically or obtained from a natural source. In some embodiments, an amino acid, including a carboxy- and/or amino-terminal amino acid in a polypeptide, can contain a structural modification as compared with the general structure herein. For example, in some embodiments, an amino acid may be modified by methylation, amidation, acetylation, and/or substitution as compared with the general structure. In some embodiments, such modification may, for example, alter the circulating half-life of a polypeptide containing the modified amino acid as compared with one containing an otherwise identical unmodified amino acid. In some embodiments, such modification does not significantly alter a relevant activity of a polypeptide containing the modified amino acid, as compared with one containing an otherwise identical unmodified amino acid. As will be clear from context, in some embodiments, the term "amino acid" is used to refer to a free amino acid; in some embodiments, it is used to refer to an amino acid residue of a polypeptide.

"Antibody": As used herein, the term "antibody" refers to a polypeptide that includes canonical immunoglobulin sequence elements sufficient to confer specific binding to a particular target antigen. As is known in the art, intact antibodies as produced in nature are approximately 150 kD tetrameric agents comprised of two identical heavy chain polypeptides (about 50 kD each) and two identical light chain polypeptides (about 25 kD each) that associate with each other into what is commonly referred to as a "Y-shaped" structure. Each heavy chain is comprised of at least four domains (each about 110 amino acids long)- an amino-terminal variable (VH) domain (located at the tips of the Y structure), followed by three constant domains: CH1, CH2, and the carboxy-terminal CH3 (located at the base of the Y's stem). A short region, known as the "switch", connects the heavy chain variable and constant regions. The "hinge" connects CH2 and CH3 domains to the rest of the antibody. Two disulfide bonds in this hinge region connect the two heavy chain polypeptides to one another in an intact antibody. Each light chain is comprised of two domains - an amino-terminal variable (VL) domain, followed by a carboxy-terminal constant (CL) domain, separated from one another by another "switch". Intact antibody tetramers are comprised of two heavy chain-light chain dimers in which the heavy and light chains are linked to one another by a single disulfide bond; two other disulfide bonds connect the heavy chain hinge regions to one another, so that the dimers are connected to one another and the tetramer is formed. Naturally-produced antibodies are also glycosylated, typically on the CH2 domain. Each domain in a natural antibody has a structure characterized by an "immunoglobulin fold" formed from two beta sheets (e.g., 3-, 4-, or 5-stranded sheets) packed against each other in a compressed antiparallel beta barrel. Each variable domain contains three hypervariable loops known as "complement determining regions" (CDR1, CDR2, and CDR3) and four somewhat invariant "framework" regions (FR1, FR2, FR3, and FR4). When natural antibodies fold, the FR regions form the beta sheets that provide the structural framework for the domains, and the CDR loop regions from both the heavy and light chains are brought together in three-dimensional space so that they create a single hypervariable antigen binding site located at the tip of the Y structure. Amino acid sequence comparisons among antibody polypeptide chains have defined two light chain ($\kappa$ and $\lambda$) classes, several heavy chain (e.g., $\mu$, $\gamma$, $\alpha$, $\varepsilon$, $\delta$) classes, and certain heavy chain subclasses ($\alpha$1, $\alpha$2, $\gamma$1, $\gamma$2, $\gamma$3, and y4). Antibody classes (IgA [including IgA1, IgA2], IgD, IgE, IgG [including IgG1, IgG2, IgG3, IgG4], IgM) are defined based on the class of the utilized heavy chain sequences. For purposes of the present disclosure, in certain embodiments, any polypeptide or complex of polypeptides that includes sufficient immunoglobulin domain sequences as found in natural antibodies can be referred to and/or used as an "antibody", whether such polypeptide is naturally produced (e.g., generated by an organism reacting to an antigen), or produced by recombinant engineering, chemical synthesis, or other artificial system or methodology.. In some embodiments, an antibody is monoclonal; in some embodiments, an antibody is monoclonal. In some embodiments, an antibody has constant region sequences that are characteristic of mouse, rabbit, primate, or human antibodies. In some embodiments, an antibody sequence elements are humanized, primatized, chimeric, etc., as is known in the art. Moreover, the term "antibody" as used herein, will be understood to encompass (unless otherwise stated or clear from context) can refer in appropriate embodiments to any of the art-known or developed constructs or formats for capturing antibody structural and functional features in alternative presentation. For example, in some embodiments, the term can refer to bi- or other multi-specific (e.g., zybodies, etc.) antibodies, Small Modular ImmunoPharmaceuticals ("SMIPsTM"), single chain antibodies, cameloid antibodies, and/or antibody fragments. In some embodiments, an antibody may lack a covalent modification (e.g., attachment of a glycan) that it would have if produced naturally. In some embodiments, an antibody may contain a covalent modification (e.g., attachment of a glycan, a payload [e.g., a detectable moiety, a therapeutic moiety, a catalytic moiety, etc], or other pendant group [e.g., poly-ethylene glycol, etc.].

"Article": As used herein, the term "article" is a manufactured format of a material. In some embodiments, and article may be a block, construct, fabric, fiber, film, foam, gel, implant, mat (*e.g.*, woven and/or non-woven), mesh, needle, particle, powder, scaffold, sheet, or tube. In some embodiments, an article is in a dry (*e.g.*, lyophilized) format. In some embodiments, an article contains a liquid or solvent (*e.g.*, an aqueous or organic liquid); in some such embodiments, the liquid is or comprises water (*e.g.*, as may be present in a gel, such as a hydrogel).

"Associated" or "Associated with": As used herein, the term "associated" or "associated with" typically refers to two or more entities in physical proximity with one another, either directly or indirectly (e.g., via one or more additional entities that serve as a linking agent), to form a structure that is sufficiently stable so that the entities remain in physical proximity under relevant conditions, e.g., physiological conditions. In some embodiments, associated entities are covalently linked to one another. In some embodiments, associated entities are non-covalently linked. In some embodiments, associated entities are linked to one another by specific non-covalent interactions (i.e., by interactions between interacting ligands that discriminate between their interaction partner and other entities present in the context of use, such as, for example. streptavidin/avidin interactions, antibody/antigen interactions, etc.). Alternatively or additionally, a sufficient number of weaker non-covalent interactions can provide sufficient stability for moieties to remain associated. Exemplary non-covalent interactions include, but are not limited to, affinity interactions, metal coordination, physical adsorption, host-guest interactions, hydrophobic interactions, pi stacking interactions, hydrogen bonding interactions, van der Waals interactions, magnetic interactions, electrostatic interactions, dipole-dipole interactions, etc.

"Binding": It will be understood that the term "binding", as used herein, typically refers to a non-covalent association between or among two or more entities. "Direct" binding involves physical contact between entities or moieties; indirect binding involves physical interaction by way of physical contact with one or more intermediate entities. Binding between two or more entities can typically be assessed in any of a variety of contexts - including where interacting entities or moieties are studied in isolation or in the context of more complex systems (e.g., while covalently or otherwise associated with a carrier entity and/or in a biological system or cell).

"Binding agent": In general, the term "binding agent" is used herein to refer to any entity that binds to a target of interest as described herein. In many embodiments, a binding agent of interest is one that binds specifically with its target in that it discriminates its target from other potential bidning partners in a particular interaction contect. In general, a binding agent may be or comprise an entity of any chemical class (e.g., polymer, non-polymer, small molecule, polypeptide, carbohydrate, lipid, nucleic acid, etc). In some embodiments, a binding agent is a single chemical entity. In some embodiments, a binding agent is a complex of two or more discrete chemical entities associated with one another under relevant conditions by non-covalent interactions. For example, those skilled in the art will appreciate that in some embodiments, a binding agent may comprise a "generic" binding moiety (e.g., one of biotin/avidin/streptaviding and/or a class-specific antibody) and a "specific" binding moiety (e.g., an antibody or aptamers with a particular molecular target) that is linked to the partner of the generic biding moiety. In some embodiments, such an approach can permit modular assembly of multiple binding agents through linkage of different specific binding moieties with the same generic binding poiety partner. In some embodiments, binding agents are or comprise polypeptides (including, e.g., antibodies or antibody fragments). In some embodiments, binding agents are or comprise small molecules. In some embodiments, binding agents are or comprise nucleic acids. In some embodiments, binding agents are aptamers. In some embodiments, binding agents are polymers; in some embodiments, binding agents are not polymers. In some embodiments, binding agents are nonn-polymeric in that they lack polymeric moieties. In some embodiments, binding agents are or comprise carbohydrates. In some embodiments, binding agents are or comprise lectins. In some embodiments, binding agents are or comprise peptidomimetics. In some embodiments, binding agents are or comprise scaffold proteins. In some embodiments, binding agents are or comprise mimeotopes. In some embodiments, binding agents are or comprise stapled peptides. In certain embodiments, binding agents are or comprise nucleic acids, such as DNA or RNA.

"Biocompatible": The term "biocompatible", as used herein, refers to materials that do not cause significant harm to living tissue when placed in contact with such tissue, e.g., *in vivo.* In certain embodiments, materials are "biocompatible" if they are not toxic to cells. In certain embodiments, materials are "biocompatible" if their addition to cells *in vitro* results in less than or equal to 20% cell death, and/or their administration *in vivo* does not induce significant inflammation or other such adverse effects.

"Biodegradable": As used herein, the term "biodegradable" refers to materials that, when introduced into cells, are broken down (e.g., by cellular machinery, such as by enzymatic degradation, by hydrolysis, and/or by combinations thereof) into components that cells can either reuse or dispose of without significant toxic effects on the cells. In certain embodiments, components generated by breakdown of a biodegradable material are biocompatible and therefore do not induce significant inflammation and/or other adverse effects *in vivo.* In some embodiments, biodegradable polymer materials break down into their component monomers. In some embodiments, breakdown of biodegradable materials (including, for example, biodegradable polymer materials) involves hydrolysis of ester bonds. Alternatively or additionally, in some embodiments, breakdown of biodegradable materials (including, for example, biodegradable polymer materials) involves cleavage of urethane linkages. Exemplary biodegradable polymers include, for example, polymers of hydroxy acids such as lactic acid and glycolic acid, including but not limited to poly(hydroxyl acids), poly(lactic acid)(PLA), poly(glycolic acid)(PGA), poly(lactic-co-glycolic acid)(PLGA), and copolymers with PEG, polyanhydrides, poly(ortho) esters, polyesters, polyurethanes, poly(butyric acid), poly(valeric acid), poly(caprolactone), poly(hydroxyalkanoates, poly(lactide-co-caprolactone), blends and copolymers thereof. Many naturally occurring polymers are also biodegradable, including, for example, proteins such as albumin, collagen, gelatin and prolamines, for example, zein, and polysaccharides such as alginate, cellulose derivatives and polyhydroxyalkanoates, for example, polyhydroxybutyrate blends and copolymers thereof. Those of ordinary skill in the art will appreciate or be able to determine when such polymers are biocompatible and/or biodegradable derivatives thereof (e.g., related to a parent polymer by substantially identical structure that differs only in substitution or addition of particular chemical groups as is known in the art).

"Biologically active": As used herein, the phrase "biologically active" refers to a substance that has activity in a biological system (e.g., in a cell (e.g., isolated, in culture, in a tissue, in an organism), in a cell culture, in a tissue, in an organism, *etc*.). For instance, a substance that, when administered to an organism, has a biological effect on that organism, is considered to be biologically active. It will be appreciated by those skilled in the art that often only a portion or fragment of a biologically active substance is required (e.g., is necessary and sufficient) for the activity to be present; in such circumstances, that portion or fragment is considered to be a "biologically active" portion or fragment.

"Characteristic portion": As used herein, the term "characteristic portion" is used, in the broadest sense, to refer to a portion of a substance whose presence (or absence) correlates with presence (or absence) of a particular feature, attribute, or activity of the substance. In some embodiments, a characteristic portion of a substance is a portion that is found in the substance and in related substances that share the particular feature, attribute or activity, but not in those that do not share the particular feature, attribute or activity. In certain embodiments, a characteristic portion shares at least one functional characteristic with the intact substance. For example, in some embodiments, a "characteristic portion" of a protein or polypeptide is one that contains a continuous stretch of amino acids, or a collection of continuous stretches of amino acids, that together are characteristic of a protein or polypeptide. In some embodiments, each such continuous stretch generally contains at least 2, 5, 10, 15, 20, 50, or more amino acids. In general, a characteristic portion of a substance (*e.g.*, of a protein, antibody, *etc.*) is one that, in addition to the sequence and/or structural identity specified above, shares at least one functional characteristic with the relevant intact substance. In some embodiments, a characteristic portion may be biologically active.

"Comparable": The term "comparable", as used herein, refers to two or more agents, entities, situations, sets of conditions, etc. that may not be identical to one another but that are sufficiently similar to permit comparison therebetween so that conclusions may reasonably be drawn based on differences or similarities observed. Those of ordinary skill in the art will understand, in context, what degree of identity is required in any given circumstance for two or more such agents, entities, situations, sets of conditions, etc. to be considered comparable.

"Conjugated": As used herein, the terms "conjugated," "linked," "attached," and "associated with," when used with respect to two or more moieties, means that the moieties are physically associated or connected with one another, either directly or via one or more additional moieties that serves as a linking agent, to form a structure that is sufficiently stable so that the moieties remain physically associated under the conditions in which structure is used, e.g., physiological conditions. Typically the moieties are attached either by one or more covalent bonds or by a mechanism that involves specific binding. Alternately, a sufficient number of weaker interactions can provide sufficient stability for moieties to remain physically associated.

"Corresponding to": As used herein, the term "corresponding to" is often used to designate the position/identity of a residue in a polymer, such as an amino acid residue in a polypeptide or a nucleotide residue in a nucleic acid. Those of ordinary skill will appreciate that, for purposes of simplicity, residues in such a polymer are often designated using a canonical numbering system based on a reference related polymer, so that a residue in a first polymer "corresponding to" a residue at position 190 in the reference polymer, for example, need not actually be the 190th residue in the first polymer but rather corresponds to the residue found at the 190th position in the reference polymer; those of ordinary skill in the art readily appreciate how to identify "corresponding" amino acids, including through use of one or more commercially-available algorithms specifically designed for polymer sequence comparisons.

"Detection entity": The term "detection entity" as used herein refers to any element, molecule, functional group, compound, fragment or moiety that is detectable. In some embodiments, a detection entity is provided or utilized alone. In some embodiments, a detection entity is provided and/or utilized in association with (e.g., joined to) another agent. Examples of detection entities include, but are not limited to: various ligands, radionuclides (e.g., $^3$H, $^{14}$C, $^{18}$F, $^{19}$F, $^{32}$P, $^{35}$S, $^{135}$I, $^{125}$I, $^{123}$I, $^{64}$Cu, $^{187}$Re, $^{111}$In, $^{90}$Y, $^{99m}$Tc, $^{177}$Lu, $^{89}$Zr etc.), fluorescent dyes (for specific exemplary fluorescent dyes, see below), chemiluminescent agents (such as, for example, acridinum esters, stabilized dioxetanes, and the like), bioluminescent agents, spectrally resolvable inorganic fluorescent semiconductors nanocrystals (i.e., quantum dots), metal nanoparticles (e.g., gold, silver, copper, platinum, etc.) nanoclusters, paramagnetic metal ions, enzymes (for specific examples of enzymes, see below), colorimetric labels (such as, for example, dyes, colloidal gold, and the like), biotin, dioxigenin, haptens, and proteins for which antisera or monoclonal antibodies are available.

"Determine": Many methodologies described herein include a step of "determining". Those of ordinary skill in the art, reading the present specification, will appreciate that such "determining" can utilize or be accomplished through use of any of a variety of techniques available to those skilled in the art, including for example specific techniques explicitly referred to herein. In some embodiments, determining involves manipulation of a physical sample. In some embodiments, determining involves consideration and/or manipulation of data or information, for example utilizing a computer or other processing unit adapted to perform a relevant analysis. In some embodiments, determining involves receiving relevant information and/or materials from a source. In some embodiments, determining involves comparing one or more features of a sample or entity to a comparable reference.

"Dosage form": As used herein, the term "dosage form" refers to a physically discrete unit of a therapeutic agent for administration to a subject. Each unit contains a predetermined quantity of active agent. In some embodiments, such quantity is a unit dosage amount (or a whole fraction thereof) appropriate for administration in accordance with a dosing regimen that has been determined to correlate with a desired or beneficial outcome when administered to a relevant population (i.e., with a therapeutic dosing regimen).

"Encapsulated": The term "encapsulated" is used herein to refer to substances that are completely surrounded by another material.

"Functional": As used herein, a "functional" biological molecule is a biological molecule in a form in which it exhibits a property and/or activity by which it is characterized. A biological molecule may have two functions (i.e., bi-functional) or many functions (i.e., multifunctional).

"Graft rejection": The term "graft rejection" as used herein, refers to rejection of tissue transplanted from a donor individual to a recipient individual. In some embodiments, graft rejection refers to an allograft rejection, wherein the donor individual and recipient individual are of the same species. Typically, allograft rejection occurs when the donor tissue carries an alloantigen against which the recipient immune system mounts a rejection response.

"High Molecular Weight Polymer": As used herein, the term "high molecular weight polymer" refers to polymers and/or polymer solutions comprised of polymers (e.g., protein polymers, such as silk) having molecular weights of at least about 200 kDa, and wherein no more than 30% of the silk fibroin has a molecular weight of less than 100 kDa. In some embodiments, high molecular weight polymers and/or polymer solutions have an average molecular weight of at least about 100 kDa or more, including, e.g., at least about 150 kDa, at least about 200 kDa, at least about 250 kDa, at least about 300 kDa, at least about 350 kDa or more. In some embodiments, high molecular weight polymers have a molecular weight distribution, no more than 50 %, for example, including, no more than 40 %, no more than 30 %, no more than 20 %, no more than 10 %, of the silk fibroin can have a molecular weight of less than 150 kDa, or less than 125 kDa, or less than 100 kDa.

"Hydrolytically degradable": As used herein, the term "hydrolytically degradable" is used to refer to materials that degrade by hydrolytic cleavage. In some embodiments, hydrolytically degradable materials degrade in water. In some embodiments, hydrolytically degradable materials degrade in water in the absence of any other agents or materials. In some embodiments, hydrolytically degradable materials degrade completely by hydrolytic cleavage, e.g., in water. By contrast, the term "non-hydrolytically degradable" typically refers to materials that do not fully degrade by hydrolytic cleavage and/or in the presence of water (e.g., in the sole presence of water).

"Hydrophilic": As used herein, the term "hydrophilic" and/or "polar" refers to a tendency to mix with, or dissolve easily in, water.

"Hydrophobic": As used herein, the term "hydrophobic" and/or "non-polar", refers to a tendency to repel, not combine with, or an inability to dissolve easily in, water.

"Identity": As used herein, the term "identity" refers to the overall relatedness between polymeric molecules, *e.g.,* between nucleic acid molecules (*e.g.,* DNA molecules and/or RNA molecules) and/or between polypeptide molecules. In some embodiments, polymeric molecules are considered to be "substantially identical" to one another if their sequences are at least 25%, 30%, 35%, 40%, 45%, 50%, 55%, 60%, 65%, 70%, 75%, 80%, 85%, 90%, 95%, or 99% identical. Calculation of the percent identity of two nucleic acid or polypeptide sequences, for example, can be performed by aligning the two sequences for optimal comparison purposes (*e.g.,* gaps can be introduced in one or both of a first and a second sequences for optimal alignment and non-identical sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or substantially 100% of the length of a reference sequence. The nucleotides at corresponding positions are then compared. When a position in the first sequence is occupied by the same residue (e.g., nucleotide or amino acid) as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleotide sequences can be determined using the algorithm of Meyers and Miller (CABIOS, 1989, 4: 11-17), which has been incorporated into the ALIGN program (version 2.0). In some exemplary embodiments, nucleic acid sequence comparisons made with the ALIGN program use a PAM120 weight residue table, a gap length penalty of 12 and a gap penalty of 4. The percent identity between two nucleotide sequences can, alternatively, be determined using the GAP program in the GCG software package using an NWSgapdna.CMP matrix.

"Insoluble state": As used herein the term insoluble state is used in reference to a silk polymer and refers to the formation of or state of being in a substantially amorphous, primarily β-sheet conformation. The term is intended to reflect the conversion of soluble silk polymer to a water insoluble state. As used herein silk polymer is in an 'insoluble state' if it can be pelleted by centrifugation or if it cannot be dissolved by immersion in or rinsing with water at 37 °C or less.

*"in vitro":* As used herein, the term *"in vitro"* refers to events that occur in an artificial environment, *e.g.,* in a test tube or reaction vessel, in cell culture, *etc.,* rather than within a multi-cellular organism.

*"in vivo":* As used herein, the term *"in vivo"* refers to events that occur within a multi-cellular organism, such as a human and a non-human animal. In the context of cell-based systems, the term may be used to refer to events that occur within a living cell (as opposed to, for example, *in vitro* systems).

"Low Molecular Weight Polymer": As used herein, the term "low molecular weight polymer" refers to polymers and/or polymer solutions, such as silk, comprised of polymers (e.g., protein polymers) having molecular weights within the range of about 20 kDa - about 400 kDa. In some embodiments, low molecular weight polymers (e.g., protein polymers) have molecular weights within a range between a lower bound (e.g., about 20 kDa, about 30 kDa, about 40 kDa, about 50 kDa, about 60 kDa, or more) and an upper bound (e.g., about 400 kDa, about 375 kDa, about 350 kDa, about 325 kDa, about 300 kDa, or less). In some embodiments, low molecular weight polymers (e.g., protein polymers such as silk) are substantially free of, polymers having a molecular weight above about 400 kD. In some embodiments, the highest molecular weight polymers in provided amphiphilic polypeptide materials are less than about 300 - about 400 kD (e.g., less than about 400 kD, less than about 375 kD, less than about 350 kD, less than about 325 kD, less than about 300 kD, etc). In some embodiments, a low molecular weight polymer and/or polymer solution can comprise a population of polymer fragments having a range of molecular weights, characterized in that: no more than 15 % of the total moles of polymer fragments in the population has a molecular weight exceeding 200 kDa, and at least 50 % of the total moles of the silk fibroin fragments in the population has a molecular weight within a specified range, wherein the specified range is between about 3.5 kDa and about 120 kDa or between about 5 kDa and about 125 kDa.

"Marker": A marker, as used herein, refers to an entity or moiety whose presence or level is a characteristic of a particular state or event. In some embodiments, presence or level of a particular marker may be characteristic of presence or stage of a disease, disorder, or condition. To give but one example, in some embodiments, the term refers to a gene expression product that is characteristic of a particular tumor, tumor subclass, stage of tumor, etc. Alternatively or additionally, in some embodiments, a presence or level of a particular marker correlates with activity (or activity level) of a particular signaling pathway, for example that may be characteristic of a particular class of tumors. The statistical significance of the presence or absence of a marker may vary depending upon the particular marker. In some embodiments, detection of a marker is highly specific in that it reflects a high probability that the tumor is of a particular subclass. Such specificity may come at the cost of sensitivity (i.e., a negative result may occur even if the tumor is a tumor that would be expected to express the marker). Conversely, markers with a high degree of sensitivity may be less specific that those with lower sensitivity. According to the present disclosure a useful marker need not distinguish tumors of a particular subclass with 100% accuracy.

"Modulator": The term "modulator" is used to refer to an entity whose presence or level in a system in which an activity of interest is observed correlates with a change in level and/or nature of that activity as compared with that observed under otherwise comparable conditions when the modulator is absent. In some embodiments, a modulator is an activator, in that activity is increased in its presence as compared with that observed under otherwise comparable conditions when the modulator is absent. In some embodiments, a modulator is an antagonist or inhibitor, in that activity is reduced in its presence as compared with otherwise comparable conditions when the modulator is absent. In some embodiments, a modulator interacts directly with a target entity whose activity is of interest. In some embodiments, a modulator interacts indirectly (i.e., directly with an intermediate agent that interacts with the target entity) with a target entity whose activity is of interest. In some embodiments, a modulator affects level of a target entity of interest; alternatively or additionally, in some embodiments, a modulator affects activity of a target entity of interest without affecting level of the target entity. In some embodiments, a modulator affects both level and activity of a target entity of interest, so that an observed difference in activity is not entirely explained by or commensurate with an observed difference in level.

"Nanoparticle": As used herein, the term "nanoparticle" refers to a particle having a diameter of less than 1000 nanometers (nm). In some embodiments, a nanoparticle has a diameter of less than 300 nm, as defined by the National Science Foundation. In some embodiments, a nanoparticle has a diameter of less than 100 nm as defined by the National Institutes of Health. In some embodiments, nanoparticles are micelles in that they comprise an enclosed compartment, separated from the bulk solution by a micellar membrane, typically comprised of amphiphilic entities which surround and enclose a space or compartment (e.g., to define a lumen). In some embodiments, a micellar membrane is comprised of at least one polymer, such as for example a biocompatible and/or biodegradable polymer.

"Nanoparticle composition": As used herein, the term "nanoparticle composition" refers to a composition that contains at least one nanoparticle and at least one additional agent or ingredient. In some embodiments, a nanoparticle composition contains a substantially uniform collection of nanoparticles as described herein.

"Nucleic acid": As used herein, the term "nucleic acid," in its broadest sense, refers to any compound and/or substance that is or can be incorporated into an oligonucleotide chain. In some embodiments, a nucleic acid is a compound and/or substance that is or can be incorporated into an oligonucleotide chain via a phosphodiester linkage. In some embodiments, "nucleic acid" refers to individual nucleic acid residues (e.g., nucleotides and/or nucleosides). In some embodiments, "nucleic acid" refers to an oligonucleotide chain comprising individual nucleic acid residues. As used herein, the terms "oligonucleotide" and "polynucleotide" can be used interchangeably. In some embodiments, "nucleic acid" encompasses RNA as well as single and/or double-stranded DNA and/or cDNA. Furthermore, the terms "nucleic acid," "DNA," "RNA," and/or similar terms include nucleic acid analogs, i.e., analogs having other than a phosphodiester backbone. For example, the so-called "peptide nucleic acids," which are known in the art and have peptide bonds instead of phosphodiester bonds in the backbone, are considered within the scope of the present disclosure. The term "nucleotide sequence encoding an amino acid sequence" includes all nucleotide sequences that are degenerate versions of each other and/or encode the same amino acid sequence. Nucleotide sequences that encode proteins and/or RNA may include introns. Nucleic acids can be purified from natural sources, produced using recombinant expression systems and optionally purified, chemically synthesized, etc. Where appropriate, e.g., in the case of chemically synthesized molecules, nucleic acids can comprise nucleoside analogs such as analogs having chemically modified bases or sugars, backbone modifications, etc. A nucleic acid sequence is presented in the 5' to 3' direction unless otherwise indicated. The term "nucleic acid segment" is used herein to refer to a nucleic acid sequence that is a portion of a longer nucleic acid sequence. In many embodiments, a nucleic acid segment comprises at least 3, 4, 5, 6, 7, 8, 9, 10, or more residues. In some embodiments, a nucleic acid is or comprises natural nucleosides (e.g., adenosine, thymidine, guanosine, cytidine, uridine, deoxyadenosine, deoxythymidine, deoxyguanosine, and deoxycytidine); nucleoside analogs (e.g., 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine); chemically modified bases; biologically modified bases (e.g., methylated bases); intercalated bases; modified sugars (e.g., 2'-fluororibose, ribose, 2'-deoxyribose, arabinose, and hexose); and/or modified phosphate groups (e.g., phosphorothioates and 5'-N-phosphoramidite linkages). In some embodiments, the present disclosure is specifically directed to "unmodified nucleic acids," meaning nucleic acids (e.g., polynucleotides and residues, including nucleotides and/or nucleosides) that have not been chemically modified in order to facilitate or achieve delivery.

"Pharmaceutical composition": As used herein, the term "pharmaceutical composition" refers to an active agent, formulated together with one or more pharmaceutically acceptable carriers. In some embodiments, active agent is present in unit dose amount appropriate for administration in a therapeutic regimen that shows a statistically significant probability of achieving a predetermined therapeutic effect when administered to a relevant population. In some embodiments, pharmaceutical compositions may be specially formulated for administration in solid or liquid form, including those adapted for the following: oral administration, for example, drenches (aqueous or non-aqueous solutions or suspensions), tablets, e.g., those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream, or foam; sublingually; ocularly; transdermally; or nasally, pulmonary, and to other mucosal surfaces.

"Physiological conditions": The phrase "physiological conditions", as used herein, relates to the range of chemical (e.g., pH, ionic strength) and biochemical (e.g., enzyme concentrations) conditions likely to be encountered in the intracellular and extracellular fluids of tissues. For most tissues, the physiological pH ranges from about 6.8 to about 8.0 and a temperature range of about 20-40 degrees Celsius, about 25-40 °C, about 30-40 °C, about 35-40 °C, about 37 °C, atmospheric pressure of about 1. In some embodiments, physiological conditions utilize or include an aqueous environment (e.g., water, saline, Ringers solution, or other buffered solution); in some such embodiments, the aqueous environment is or comprises a phosphate buffered solution (e.g., phosphate-buffered saline).

"Polypeptide": The term "polypeptide", as used herein, generally has its art-recognized meaning of a polymer of at least three amino acids, linked to one another by peptide bonds. In some embodiments, the term is used to refer to specific functional classes of polypeptides. For each such class, the present specification provides several examples of amino acid sequences of known exemplary polypeptides within the class; in some embodiments, such known polypeptides are reference polypeptides for the class. In such embodiments, the term "polypeptide" refers to any member of the class that shows significant sequence homology or identity with a relevant reference polypeptide. In many embodiments, such member also shares significant activity with the reference polypeptide. Alternatively or additionally, in many embodiments, such member also shares a particular characteristic sequence element with the reference polypeptide (and/or with other polypeptides within the class; in some embodiments, with all polypeptides within the class). For example, in some embodiments, a member polypeptide shows an overall degree of sequence homology or identity with a reference polypeptide that is at least about 30-40%, and is often greater than about 50%, 60%, 70%, 80%, 90%, 91%, 92%, 93%, 94%, 95%, 96%, 97%, 98%, 99% or more and/or includes at least one region (i.e., a conserved region that may in some embodiments, may be or comprise a characteristic sequence element) that shows very high sequence identity, often greater than 90% or even 95%, 96%, 97%, 98%, or 99%. Such a conserved region usually encompasses at least 3-4 and often up to 20 or more amino acids; in some embodiments, a conserved region encompasses at least one stretch of at least 2, 3, 4, 5, 6, 7, 8, 9, 10, 11, 12, 13, 14, 15 or more contiguous amino acids. In some embodiments, a useful polypeptide may comprise or consist of a fragment of a parent polypeptide. In some embodiments, a useful polypeptide as may comprise or consist of a plurality of fragments, each of which is found in the same parent polypeptide in a different spatial arrangement relative to one another than is found in the polypeptide of interest (e.g., fragments that are directly linked in the parent may be spatially separated in the polypeptide of interest or vice versa, and/or fragments may be present in a different order in the polypeptide of interest than in the parent), so that the polypeptide of interest is a derivative of its parent polypeptide. In some embodiments, a polypeptide may comprise natural amino acids, non-natural amino acids, or both. In some embodiments, a polypeptide may comprise only natural amino acids or only non-natural amino acids. In some embodiments, a polypeptide may comprise D-amino acids, L-amino acids, or both. In some embodiments, a polypeptide may comprise only D-amino acids. In some embodiments, a polypeptide may comprise only L-amino acids. In some embodiments, a polypeptide may include one or more pendant groups, e.g., modifying or attached to one or more amino acid side chains, and/or at the polypeptide's N-terminus, the polypeptide's C-terminus, or both. In some embodiments, a polypeptide may be cyclic. In some embodiments, a polypeptide is not cyclic. In some embodiments, a polypeptide is linear.

"Polysaccharide": The term "polysaccharide" refers to a polymer of sugars. Typically, a polysaccharide comprises at least three sugars. In some embodiments, a polypeptide comprises natural sugars (e.g., glucose, fructose, galactose, mannose, arabinose, ribose, and xylose); alternatively or additionally, in some embodiments, a polypeptide comprises one or more non-natural amino acids (e.g. modified sugars such as 2'-fluororibose, 2'-deoxyribose, and hexose).

"Porosity": The term "porosity" as used herein, refers to a measure of void spaces in a material and is a fraction of volume of voids over the total volume, as a percentage between 0 and 100%. A determination of a porosity is known to a skilled artisan using standardized techniques, for example mercury porosimetry and gas adsorption (e.g., nitrogen adsorption).

"Protein": As used herein, the term "protein" refers to a polypeptide (i.e., a string of at least two amino acids linked to one another by peptide bonds). Proteins may include moieties other than amino acids (e.g., may be glycoproteins, proteoglycans, etc.) and/or may be otherwise processed or modified. Those of ordinary skill in the art will appreciate that a "protein" can be a complete polypeptide chain as produced by a cell (with or without a signal sequence), or can be a characteristic portion thereof. Those of ordinary skill will appreciate that a protein can sometimes include more than one polypeptide chain, for example linked by one or more disulfide bonds or associated by other means. Polypeptides may contain L-amino acids, D-amino acids, or both and may contain any of a variety of amino acid modifications or analogs known in the art. Useful modifications include, e.g., terminal acetylation, amidation, methylation, etc. In some embodiments, proteins may comprise natural amino acids, non-natural amino acids, synthetic amino acids, and combinations thereof. The term "peptide" is generally used to refer to a polypeptide having a length of less than about 100 amino acids, less than about 50 amino acids, less than 20 amino acids, or less than 10 amino acids. In some embodiments, proteins are antibodies, antibody fragments, biologically active portions thereof, and/or characteristic portions thereof.

"Reference": The term "reference" is often used herein to describe a standard or control agent, individual, population, sample, sequence or value against which an agent, individual, population, sample, sequence or value of interest is compared. In some embodiments, a reference agent, individual, population, sample, sequence or value is tested and/or determined substantially simultaneously with the testing or determination of the agent, individual, population, sample, sequence or value of interest. In some embodiments, a reference agent, individual, population, sample, sequence or value is a historical reference, optionally embodied in a tangible medium. Typically, as would be understood by those skilled in the art, a reference agent, individual, population, sample, sequence or value is determined or characterized under conditions comparable to those utilized to determine or characterize the agent, individual, population, sample, sequence or value of interest.

"Small molecule": As used herein, the term "small molecule" is used to refer to molecules, whether naturally-occurring or artificially created (e.g., via chemical synthesis), having a relatively low molecular weight and being an organic and/or inorganic compound. Typically, a "small molecule" is monomeric and have a molecular weight of less than about 1500 g/mol. In general, a "small molecule" is a molecule that is less than about 5 kilodaltons (kDa) in size. In some embodiments, a small molecule is less than about 4 kDa, 3 kDa, about 2 kDa, or about 1 kDa. In some embodiments, the small molecule is less than about 800 daltons (Da), about 600 Da, about 500 Da, about 400 Da, about 300 Da, about 200 Da, or about 100 Da. In some embodiments, a small molecule is less than about 2000 g/mol, less than about 1500 g/mol, less than about 1000 g/mol, less than about 800 g/mol, or less than about 500 g/mol. In some embodiments, a small molecule is not a polymer. In some embodiments, a small molecule does not include a polymeric moiety. In some embodiments, a small molecule is not a protein or polypeptide (e.g., is not an oligopeptide or peptide). In some embodiments, a small molecule is not a polynucleotide (e.g., is not an oligonucleotide). In some embodiments, a small molecule is not a polysaccharide. In some embodiments, a small molecule does not comprise a polysaccharide (e.g., is not a glycoprotein, proteoglycan, glycolipid, etc.). In some embodiments, a small molecule is not a lipid. In some embodiments, a small molecule is a modulating agent. In some embodiments, a small molecule is biologically active. In some embodiments, a small molecule is detectable (e.g., comprises at least one detectable moiety). In some embodiments, a small molecule is a therapeutic. Preferred small molecules are biologically active in that they produce a local or systemic effect in animals, preferably mammals, more preferably humans. In certain preferred embodiments, the small molecule is a drug. Preferably, though not necessarily, the drug is one that has already been deemed safe and effective for use by the appropriate governmental agency or body. For example, drugs for human use listed by the FDA under 21 C.F.R. §§ 330.5, 331 through 361, and 440 through 460; drugs for veterinary use listed by the FDA under 21 C.F.R. §§ 500 through 589 are all considered acceptable for use in accordance with the present application.

"Solution": As used herein, the term "solution" broadly refers to a homogeneous mixture composed of one phase. Typically, a solution comprises a solute or solutes dissolved in a solvent or solvents. It is characterized in that the properties of the mixture (such as concentration, temperature, and density) can be uniformly distributed through the volume. In the context of the present application, therefore, a "silk fibroin solution" refers to silk fibroin protein in a soluble form, dissolved in a solvent, such as water. In some embodiments, silk fibroin solutions may be prepared from a solid-state silk fibroin material (i.e., silk matrices), such as silk films and other scaffolds. Typically, a solid-state silk fibroin material is reconstituted with an aqueous solution, such as water and a buffer, into a silk fibroin solution. It should be noted that liquid mixtures that are not homogeneous, e.g., colloids, suspensions, emulsions, are not considered solutions.

"Stable": The term "stable," when applied to compositions herein, means that the compositions maintain one or more aspects of their physical structure and/or activity over a period of time under a designated set of conditions. In some embodiments, the period of time is at least about one hour; in some embodiments, the period of time is about 5 hours, about 10 hours, about one (1) day, about one (1) week, about two (2) weeks, about one (1) month, about two (2) months, about three (3) months, about four (4) months, about five (5) months, about six (6) months, about eight (8) months, about ten (10) months, about twelve (12) months, about twenty-four (24) months, about thirty-six (36) months, or longer. In some embodiments, the period of time is within the range of about one (1) day to about twenty-four (24) months, about two (2) weeks to about twelve (12) months, about two (2) months to about five (5) months, etc. In some embodiments, the designated conditions are ambient conditions (e.g., at room temperature and ambient pressure). In some embodiments, the designated conditions are physiologic conditions (e.g., *in vivo* or at about 37 °C for example in serum or in phosphate buffered saline). In some embodiments, the designated conditions are under cold storage (e.g., at or below about 4 °C, -20°C, or -70 °C). In some embodiments, the designated conditions are in the dark.

"Substantially": As used herein, the term "substantially", and grammatic equivalents, refer to the qualitative condition of exhibiting total or near-total extent or degree of a characteristic or property of interest. One of ordinary skill in the art will understand that biological and chemical phenomena rarely, if ever, go to completion and/or proceed to completeness or achieve or avoid an absolute result.

"Sustained release": The term "sustained release" is used herein in accordance with its art-understood meaning of release that occurs over an extended period of time. The extended period of time can be at least about 3 days, about 5 days, about 7 days, about 10 days, about 15 days, about 30 days, about 1 month, about 2 months, about 3 months, about 6 months, or even about 1 year. In some embodiments, sustained release is substantially burst-free. In some embodiments, sustained release involves steady release over the extended period of time, so that the rate of release does not vary over the extended period of time more than about 5%, about 10%, about 15%, about 20%, about 30%, about 40% or about 50%. In some embodiments, sustained release involves release with first-order kinetics. In some embodiments, sustained release involves an initial burst, followed by a period of steady release. In some embodiments, sustained release does not involve an initial burst. In some embodiments, sustained release is substantially burst-free release.

"Therapeutic agent": As used herein, the phrase "therapeutic agent" refers to any agent that elicits a desired pharmacological effect when administered to an organism. In some embodiments, an agent is considered to be a therapeutic agent if it demonstrates a statistically significant effect across an appropriate population. In some embodiments, the appropriate population may be a population of model organisms. In some embodiments, an appropriate population may be defined by various criteria, such as a certain age group, gender, genetic background, preexisting clinical conditions, etc. In some embodiments, a therapeutic agent is any substance that can be used to alleviate, ameliorate, relieve, inhibit, prevent, delay onset of, reduce severity of, and/or reduce incidence of one or more symptoms or features of a disease, disorder, and/or condition.

"Therapeutically effective amount": As used herein, the term "therapeutically effective amount" means an amount that is sufficient, when administered to a population suffering from or susceptible to a disease, disorder, and/or condition in accordance with a therapeutic dosing regimen, to treat the disease, disorder, and/or condition. In some embodiments, a therapeutically effective amount is one that reduces the incidence and/or severity of, and/or delays onset of, one or more symptoms of the disease, disorder, and/or condition. Those of ordinary skill in the art will appreciate that the term "therapeutically effective amount" does not in fact require successful treatment be achieved in a particular individual. Rather, a therapeutically effective amount may be that amount that provides a particular desired pharmacological response in a significant number of subjects when administered to patients in need of such treatment. It is specifically understood that particular subjects may, in fact, be "refractory" to a "therapeutically effective amount." To give but one example, a refractory subject may have a low bioavailability such that clinical efficacy is not obtainable. In some embodiments, reference to a therapeutically effective amount may be a reference to an amount as measured in one or more specific tissues (e.g., a tissue affected by the disease, disorder or condition) or fluids (e.g., blood, saliva, serum, sweart, tears, urine, etc). Those of ordinary skill in the art will appreciate that, in some embodiments, a therapeutically effective amount may be formulated and/or administered in a single dose. In some embodiments, a therapeutically effective amount may be formulated and/or administered in a plurality of doses, for example, as part of a dosing regimen.

"Treating": As used herein, the term "treating" refers to partially or completely alleviating, ameliorating, relieving, inhibiting, preventing (for at least a period of time), delaying onset of, reducing severity of, reducing frequency of and/or reducing incidence of one or more symptoms or features of a particular disease, disorder, and/or condition. In some embodiments, treatment may be administered to a subject who does not exhibit symptoms, signs, or characteristics of a disease and/or exhibits only early symptoms, signs, and/or characteristics of the disease, for example for the purpose of decreasing the risk of developing pathology associated with the disease. In some embodiments, treatment may be administered after development of one or more symptoms, signs, and/or characteristics of the disease.

"Variant": As used herein, the term "variant" refers to an entity that shows significant structural identity with a reference entity but differs structurally from the reference entity in the presence or level of one or more chemical moieties as compared with the reference entity. In many embodiments, a variant also differs functionally from its reference entity. In general, whether a particular entity is properly considered to be a "variant" of a reference entity is based on its degree of structural identity with the reference entity. As will be appreciated by those skilled in the art, any biological or chemical reference entity has certain characteristic structural elements. A variant, by definition, is a distinct chemical entity that shares one or more such characteristic structural elements. To give but a few examples, a small molecule may have a characteristic core structural element (e.g., a macrocycle core) and/or one or more characteristic pendent moieties so that a variant of the small molecule is one that shares the core structural element and the characteristic pendent moieties but differs in other pendent moieties and/or in types of bonds present (single vs double, E vs Z, etc.) within the core, a polypeptide may have a characteristic sequence element comprised of a plurality of amino acids having designated positions relative to one another in linear or three-dimensional space and/or contributing to a particular biological function, a nucleic acid may have a characteristic sequence element comprised of a plurality of nucleotide residues having designated positions relative to on another in linear or three-dimensional space. For example, a variant polypeptide may differ from a reference polypeptide as a result of one or more differences in amino acid sequence and/or one or more differences in chemical moieties (e.g., carbohydrates, lipids, etc.) covalently attached to the polypeptide backbone. In some embodiments, a variant polypeptide shows an overall sequence identity with a reference polypeptide that is at least 85%, 86%, 87%, 88%, 89%, 90%, 91%, 92%, 93%, 94%, 95%, 96%, 97%, or 99%. Alternatively or additionally, in some embodiments, a variant polypeptide does not share at least one characteristic sequence element with a reference polypeptide. In some embodiments, the reference polypeptide has one or more biological activities. In some embodiments, a variant polypeptide shares one or more of the biological activities of the reference polypeptide. In some embodiments, a variant polypeptide lacks one or more of the biological activities of the reference polypeptide. In some embodiments, a variant polypeptide shows a reduced level of one or more biological activities as compared with the reference polypeptide. In many embodiments, a polypeptide of interest is considered to be a "variant" of a parent or reference polypeptide if the polypeptide of interest has an amino acid sequence that is identical to that of the parent but for a small number of sequence alterations at particular positions. Typically, fewer than 20%, 15%, 10%, 9%, 8%, 7%, 6%, 5%, 4%, 3%, 2% of the residues in the variant are substituted as compared with the parent. In some embodiments, a variant has 10, 9, 8, 7, 6, 5, 4, 3, 2, or 1 substituted residue as compared with a parent. Often, a variant has a very small number (e.g., fewer than 5, 4, 3, 2, or 1) number of substituted functional residues (i.e., residues that participate in a particular biological activity). Furthermore, a variant typically has not more than 5, 4, 3, 2, or 1 additions or deletions, and often has no additions or deletions, as compared with the parent. Moreover, any additions or deletions are typically fewer than about 25, about 20, about 19, about 18, about 17, about 16, about 15, about 14, about 13, about 10, about 9, about 8, about 7, about 6, and commonly are fewer than about 5, about 4, about 3, or about 2 residues. In some embodiments, the parent or reference polypeptide is one found in nature. As will be understood by those of ordinary skill in the art, a plurality of variants of a particular polypeptide of interest may commonly be found in nature, particularly when the polypeptide of interest is an infectious agent polypeptide.

## DETAILED DESCRIPTION OF CERTAIN EMBODIMENTS

The present disclosure, among other things, provides solid materials comprised of at least one amphiphilic polypeptide, referred to herein as "amphiphilic polypeptide materials".In some embodiments, provided amphiphilic polypeptide materials are characterized in that when manufactured, they may form three dimensional structures, i.e. blocks or monoliths of any size or shape. Amphiphilic polypeptide materials and methods of preparing such amphiphilic polypeptide materials are provided.

Various embodiments according to the present disclosure are described in detail herein. In particular, the present disclosure describes provided amphiphilic polypeptide materials and their use in various applications, including, for example: anti-counterfeiting materials, biomaterials, biomedical devices, commercial products, controlled degradation applications, controlled release applications, drug delivery, drug release, electronics, extrusion injection molding, materials for tunable degradation, optics, orthopedic devices, orthopedic implants, photonics, preservation of biologically labile compounds, preservation of heat labile compounds, prosthetics, regenerative medicine, robotics, sensing, tissue engineering applications, tissue regeneration, tissue scaffolding, triggered release applications and/or wound clotting.

Polymer blocks or monoliths generally describe materials that are three dimensional and capable of assuming different sizes and shapes. Three dimensional polypeptide structures are known in certain arts to be useful as biomaterials. Fibrous and globular proteins among others are structural proteins that can provide both rigidity and three dimensional shape in biological systems. Fibrous protein for example provide skin, fur, hair, wool, claws, nails, hooves, horns, scales, beaks and feathers in various birds and mammals.

Three dimensional polymer structures have been used for example in the repair or replacement of tissue. Prior polymer and polypeptide materials provide both structural support for tissues and can support regenerative growth. Prior materials however have suffered from drawbacks, including fabrication using materials that lack mechanical strength, fabrication using materials that are not expansion matched to tissues or bone, fabrication using materials that are incompatible because they degraded either too quickly or too slowly. (See for example, Caterson et al., 57 J. Biomed. Mater. Res., 394-403 (2001); Karp et al., 14 J. Craniofacial Surgery 3, 317-323 (2003); Harris et al., 42 J. Biomed. Mater. Res. 396-402 (1998); Holy et al., 65A J. Biomed. Mater. Res., 447-453 (2003); Hutmacher D. 21 Biomaterials, 2529-2543 (2000); Martin et al., 55 J. Biomed. Mater. Res., 229-235 (2001); Perez-Rigueiro et al., 70 Science, 2439-2447 (1998); Petite et al., 18 Nat. Biotechnol., 959-96 (2000); Sofia et al., 54 J. of Biomedical Materials Research, 139-148 (2001)).

More recently, blocks composed of a mixture of silk polymer solution with hydroxyapatite particles were introduced. (See International Patent Publication No.: WO 2009/100280 A2 to Kaplan et al., entitled 3-Dimensional Silk Hydroxyapatite Compositions published on August 13, 2009 (hereafter the "Kaplan Application")). The polymer blocks of the Kaplan Application are formed by combining a paste comprising silk fibroin and hydroxyapatite particles with hexafluoroisopropanol ('HFIP') resulting in a substantially insoluble crystalline material.

The insoluble crystals of the Kaplan Application specifically are formed when the amorphous silk fibroin paste was combined with HFIP and/or exposed to conditions to induce beta-sheet character. The Kaplan Application teaches inducing beta sheet character by direct application of the composition to a defect site or by injecting or pouring the composition into a mold. (See the Kaplan Application at [010]). The Kaplan Application explained that the paste cures to the insoluble crystalline state through either dehydration or shear stress when it is applied during injection. (See the Kaplan Application at [070]). The Kaplan Application further explained that once induced, silk in its insoluble state would result in formation of three dimension structures at the defect site or should the silk solution occupy a mold, an insoluble three dimensional structure would form having about the size and shape of the mold. (See the Kaplan Application at [068]-[070]). The Kaplan Application theorized an all aqueous approach, explaining that such an approach would be useful in order to minimize damage to tissues cause by solvents such as HFIP and methanol. (See the Kaplan Application at [068]-[070]).

Composition and Structure

Polypeptide material monoliths as described herein are or comprise amphiphilic polypeptides.

Aspects of the present disclosure are throughout exemplified by a discussion and/or use of silk fibroin as an amphiphilic polypeptide. Those skilled in the art will readily appreciate that, in many cases, teachings and examples provided with respect to silk are equally applicable to other amphiphilic polypeptides as disclosed herein. For example, amphiphilic polypeptides described herein may comprise an amino acid sequence of polypeptides selected from the following list: actins, catenins, claudins, coilins, collagens, elastins, elaunins, extensins, fibrillins, fibroins, lamins, laminins, keratins, resilins, tublins, viral structural proteins, or zein proteins (seed storage protein).

In some embodiments, provided amphiphilic polypeptide materials are made of a polypeptides corresponding to any one of the above provided list, with or without one or more sequence variations, as compared to the native or wild type counterpart. In some embodiments, for example, such variants may show at least 85% overall sequence identity as compared to a wild type sequence. In some embodiments, for example, such variants may show at least 86%, 87%, 88%, 89%, 90%, 91%, 92%, 93%, 94%, 95%, 96%, 97%, 98%, 99% overall sequence identity.

Indeed, those skilled in the art will understand that the present disclosure is applicable to amalgams, blends, combination, compositions, fusions, or mixtures of such amphiphilic polypeptides.

Moreover, aspects of the present disclosure are below exemplified through discussion and/or use of silk fibroin from the silkworm, *Bombyx mori.* Indeed, those skilled in the art will also appreciate that the teaching and examples provided by the present disclosure are also equally applicable to other forms or types of silk fibroin such as, for example, spider silk such as that from *Nephila clavipes* and/or genetically engineered silk, such as from bacteria, yeast, mammalian cells, transgenic animals or transgenic plants.

Silk fibers spun by spiders and silkworms exhibit exceptional mechanical properties with a unique combination of strength, extensibility and toughness.

In some embodiments, provided amphiphilic polypeptide materials are water-based. In some embodiments, provided amphiphilic polypeptide materials contain residual water. In some embodiments, provided amphiphilic polypeptide materials do not contain residual non-aqueous solvents. In some embodiments, provided amphiphilic polypeptide materials have a lower bound solvent content than prior materials. Provided amphiphilic polypeptide materials have a frozen and non-frozen bound water content of less than 50%.

In some embodiments, when a provided amphiphilic polypeptide material is silk fibroin, such a material has a bound water content (freezing and non-freezing) that measures less than about 50 wt%, about 45 wt%, about 40 wt%, about 39 wt%, about 38 wt%, about 37 wt%, about 36 wt%, about 35 wt%, about 34 wt%, about 33 wt%, about 32 wt%, about 31 wt%, about 30 wt%, about 29 wt%, about 28 wt%, about 27 wt%, about 26 wt%, about 25 wt%, about 24 wt%, about 23 wt%, about 22 wt%, about 21 wt%, about 20 wt%, about 19 wt%, about 18 wt%, about 17 wt%, about 16 wt%, about 15 wt%, about 14 wt%, about 13 wt%, about 12 wt%, about 11 wt%, about 10 wt%, about 9 wt%, about 8 wt%, about 7 wt%, about 6 wt%, about 5 wt%, about 4 wt%, about 3 wt%, about 2 wt%, about 1 wt%, about 0.5 about wt%, or about 0.1 wt%.

In some embodiments, provided amphiphilic polypeptide materials form three-dimensional "blocks" also referred to herein as "monoliths." In some embodiments, as provided herein, a monolith is a three-dimensional block of an amphiphilic polypeptide material.

Amphiphilic polypeptides are structural proteins that are the building blocks for living organisms. Amphiphilic polypeptides are molecules that are composed of multiple domains. Amphiphilic polypeptides typically have three domains: a hydrophilic end, a region comprising amino acids, and a hydrophobic tail.

In some embodiments, an amino acid domain of an amphiphilic polypeptide material is characterized by highly repetitive (i.e. crystalline) amino acid sequences intervaled by disordered (i.e. amorphous) sequences. In some embodiments, a hydrophilic end permits or enhances biological function of an amphiphilic polypeptide material. In some embodiments, a hydrophobic tail of the amphiphilic polypeptide is repelled by water so that such tails aggregate.

In some embodiments, sequences of provided amphiphilic polypeptide materials have the capability to initiate intermolecular interactions and promote self-assembly. In some embodiments, provided amphiphilic polypeptide materials form cross-links which drive the folding of the proteins in long-range ordered molecular secondary, tertiary and quaternary structures (e.g. beta-sheets, coils, helices). As such, in some embodiments, individual domains of amphiphilic polypeptides associate with each other and result in growth of larger assemblies. In some embodiments, provided amphiphilic polypeptide materials comprise amphiphilic polypeptides assemble having a three dimensional monolithic structure. In some embodiments, provided amphiphilic polypeptide materials are characterized by outstanding structural and mechanical properties together with unique biological functionality.

Properties

Provided amphiphilic polypeptide materials are characterized by unique mechanical properties or features that provide advantages over prior materials and/or prior material compositions.

In some embodiments, mechanical properties of amphiphilic polypeptide materials can be tuned through control of the fabrication process.

In some embodiments, provided amphiphilic polypeptide materials are characterized in that such materials are three-dimensional structures, blocks, or monoliths.

In some embodiments, provided amphiphilic polypeptide materials are block or monoliths that characterized in that they are or comprise a single piece of material. In some embodiments, provided amphiphilic polypeptide materials are macrostructures that are both solid and continuous throughout their full size and/or volume.

In some embodiments, provided amphiphilic polypeptide materials are characterized by size. In some embodiments, provided amphiphilic polypeptide materials are characterized by dimensions, such height, width, depth, diameter, or combinations thereof. In some embodiments, provided amphiphilic polypeptide materials are characterized by volume.

In some embodiments, provided amphiphilic polypeptide materials measure less than about 1 nm in each dimension. In some embodiments, provided amphiphilic polypeptide materials measure in each dimension, for example about 0.1 nm, about 0.5 nm, about 1 nm, about 2 nm, about 5 nm, about 10 nm, about 20 nm, about 30 nm, about 40 nm, about 50 nm, about 100 nm, about 250 nm, about 500 nm, about 750 nm, about 1000 nm, about 5 $\mu$m, about 10 $\mu$m, about 50 $\mu$m, about 100 $\mu$m, about 250 $\mu$m, about 500 $\mu$m, about 750 $\mu$m, about 1000 $\mu$m, about 5 mm, about 10 mm, about 20 mm, about 30 mm, about 40 mm, about 50 mm, about 100 mm, about 250 mm, about 500 mm, about 750 mm, about 1000 mm or more. In some embodiments, provided amphiphilic polypeptide materials are or form blocks having dimensions that are only limited according to availability of materials and/or a volume of a template, mold, or substrate that is used to form such materials.

In some embodiments, provided amphiphilic polypeptide materials have dimensions that are only limited according to an availability of materials and/or the volume of a template, mold, or substrate that is used to form these materials.

In some embodiments, provided amphiphilic polypeptide materials are unexpectedly large relative to prior materials. The Kaplan Application describes "a cylindrical mineralized silk biopolymer structure" that took the shape of the container and having a diameter of approximately 6.5 mm by a height of about 4-6 mm. By contrast, in some embodiments, dimensions of provided amphiphilic polypeptide materials are limited only by a supply of material and/or dimensions of a mold or substrate.

In some embodiments, provided amphiphilic polypeptide materials are a single coherent material. In some embodiments, a single coherent material is characterized in that they are not assembled from individual layers or sections of materials.

Materials described in the Kaplan Application and use hexafluoroisopropanol (HFIP), trifluoroisopropanol (TFIP) or formic acid (FA) as protein solvents for the fabrication of fibroin solid structures cannot obtain large structures ($l_0$>5 cm).

Materials described in the Kaplan Application and use hexafluoroisopropanol (HFIP), trifluoroisopropanol (TFIP) or formic acid (FA) as protein solvents for the fabrication of fibroin solid structures allow for the dissolution of silk fibroin in highly concentrated (>15 wt%), viscous solutions where the pre-beta-sheet, helical form of the protein is maintained. Indeed, these solvent-based processes are able to yield small crystalline silk blocks, but fail in the obtainment of large ($l_0$>5 cm) structures due to the instability of the viscous solution when in contact with the coagulation bath (any polar solvent).

In some embodiments, provided amphiphilic polypeptide materials are such that they can exist in any shape that is desirable.

In some embodiments, provided amphiphilic polypeptide materials are in a desirable shape because they were molded using a cast, a frame, a form, a mold, a template during processing. In some embodiments, provided amphiphilic polypeptide materials will assume a desired shape when added, deposited, introduced, and/or poured into a template, mold, or onto a substrate and molded.

In some embodiments, provided amphiphilic polypeptide materials are machined. In some embodiments, provided amphiphilic polypeptide materials are machined using standard machine tools, tooling, and methods. In some embodiments, provided amphiphilic polypeptide materials are mechanically manipulated, for example using hand tools.

In some embodiments, provided amphiphilic polypeptide materials are characterized by their density. In some embodiments, provided amphiphilic polypeptide materials have a density that is comparable to the density of the native polypeptide for example when it is in fibroin or globulin form. Prior materials, for example prior materials described in the Kaplan Application alone cannot achieve such comparable density values.

In contrast to prior materials, in some embodiments, for example, when a provided amphiphilic polypeptide material is silk fibroin, such a material has a density comparable to the density of native silk fibroin. In some embodiments, when a provided amphiphilic polypeptide material is silk fibroin, such a material has a density of about 1.4 kg/dm$^3$, which is comparable to the density of native silk fibroin. In some embodiments, when a provided amphiphilic polypeptide material is silk fibroin, such a material has a density that measures about 0.05 kg/dm$^3$ and about 5.0 kg/dm$^3$, about 0.7 kg/dm$^3$ and about 2.1 kg/dm$^3$, about 1.0 kg/dm$^3$ and about 1.5 kg/dm$^3$, or about 1.1 kg/dm$^3$ and about 1.4 kg/dm$^3$. In some embodiments, for example, when a polypeptide material is silk fibroin, such a material has a density of for example about 0.1 kg/dm$^3$, 0.2 kg/dm$^3$, 0.3 kg/dm$^3$, 0.4 kg/dm$^3$, 0.5 kg/dm$^3$, 0.6 kg/dm$^3$, 0.6 kg/dm$^3$, 0.8 kg/dm$^3$, 0.9 kg/dm$^3$, 1.0 kg/dm$^3$, 1.1 kg/dm$^3$, 1.2 kg/dm$^3$, 1.3 kg/dm$^3$, 1.4 kg/dm$^3$, 1.5 kg/dm$^3$, 1.6 kg/dm$^3$, 1.7 kg/dm$^3$, 1.8 kg/dm$^3$, 1.9 kg/dm$^3$, 2.0 kg/dm$^3$, 2.1 kg/dm$^3$, 2.2 kg/dm$^3$, 2.3 kg/dm$^3$, 2.4 kg/dm$^3$, or at least about 2.5 kg/dm$^3$.

In some embodiments, provided amphiphilic polypeptide materials are characterized by uniform density. In some embodiments, provided amphiphilic polypeptide materials have a micro-density continuity. In some embodiments, provided amphiphilic polypeptide materials have a nano-density continuity.

In some embodiments, provided amphiphilic polypeptide materials are characterized by a porosity. In some embodiments, provided amphiphilic polypeptide materials have a porosity between about 0.5% and 98.5%. In some embodiments, provided amphiphilic polypeptide materials have a porosity, for example, of between about 0.01 and 0.9. In some embodiments, amphiphilic polypeptide materials of the present disclosure are less than about 1 % porous, about 2 % porous, about 3 % porous, about 4 % porous, about 5 % porous, about 6 % porous, about 7 % porous, about 8 % porous, about 9 % porous, about 10 % porous, about 20 % porous, about 30 % porous, about 40 % porous, about 50 % porous, about 60 % porous, about 70 % porous, about 80 % porous, about 90 % porous, at least about 98.5%. For example, in some embodiments, provided amphiphilic polypeptide materials have a porosity comparable to mechanical aerogels.

In some embodiments, provided amphiphilic polypeptide materials have pores with an average pore diameter, including about 0.5 μm, about 1 μm, about 2 μm, about 3 μm, about 4 μm, about 5 μm, about 10 μm, about 15 μm, about 20 μm, about 25 μm, about 30 μm, about 35 μm, about 40 μm, about 45 μm, about 50 μm, about 55 μm, about 60 μm, about 65 μm, about 70 μm, about 75 μm, about 80 μm, about 85 μm, about 90 μm, about 95 μm, about 100 μm, about 125 μm, about 150 μm, about 175 μm, about 200 μm, about 225 μm, about 250 μm, about 275 μm, about 300 μm, about 325 μm, about 350 μm, about 375 μm, about 400 μm, about 450 μm, about 475 μm, or about 500 μm, or more.

In some embodiments, a porosity of provided amphiphilic polypeptide materials is controlled when slow-degrading porogens or porogens that are soluble in polar solvents (like PMMA spheres) are incorporated.

In some embodiments, provided amphiphilic polypeptide materials are substantially amorphous, substantially crystalline, or combinations thereof. In some embodiments, provided amphiphilic polypeptide materials are characterized as exhibiting crystallinity and/or nano-sized crystalline particles.

In some embodiments, provided amphiphilic polypeptide materials are characterized by a secondary structure. In some embodiments, provided amphiphilic polypeptide materials are characterized by helical structures, beta structures, or combinations thereof.

In some embodiments, provided amphiphilic polypeptide materials are characterized by uniform consistency of secondary structures throughout the materials.

In some embodiments, provided amphiphilic polypeptide materials are highly crystalline and surprisingly so when compared to prior materials. In some embodiments, silk fibroin-based materials are characterized by crystalline structure, for example, comprising beta sheet structure and/or hydrogen bonding. In some embodiments, provided amphiphilic polypeptide materials possess at least about five to ten percent more beta sheet content than prior materials. In some embodiments, for example, when a provided amphiphilic polypeptide material is silk fibroin, beta-sheet content is at least about 55%. In some embodiments, beta sheet character of provided amphiphilic polypeptide materials is about 55%, about 60%, about 65%, about 70%, about 75%, about 80%, about 85%, about 90%, about 95%, or about 100%..

Neither the HFIP nor the all aqueous silk-hydroxyapatite compositions described in the Kaplan Application could achieve such values for beta-sheet content. The maximum beta-sheet content the silk-hydroxyapatite blocks were shown to achieve was about 45%. While not wishing to rely on any particular theory, it is believed that passive evaporation of solvent alone or in combination with shear stress as described by the Kaplan Application is insufficient to induce sufficient beta sheet content to form blocks as provided by the present disclosure. Traditional methods for increasing beta-sheet are known, for example, immersion in solvents, such as methanol, annealing, etc.

By contrast, in some embodiments, for example, when a provided amphiphilic polypeptide material is silk fibroin, a percentage beta sheet content, for example, is measured between about 55 % and about 100 %, about 55 % and about 95 %, about 55 % and about 90 %. In some embodiments, for example, when a provided amphiphilic polypeptide material is silk fibroin, a percentage beta sheet content, for example, is measured as about 55 %, about 56 %, about 57 %, about 58 %, about 59 %, about 60 %, about 61 %, about 62 %, about 63 %, about 64 %, about 65 %, about 66 %, about 67 %, about 68 %, about 69 %, about 70 %, about 71 %, about 72 %, about 73 %, about 74 %, about 75 %, about 76 %, about 77 %, about 78 %, about 79 %, about 80 %, about 85 %, about 90 %, about 95 % or more.

In some embodiments, provided amphiphilic polypeptide materials are characterized by a crystalline orientation.

In some embodiments, provided amphiphilic polypeptide materials are crystalline. In some embodiments, atoms and ions of such crystalline polypeptide materials are organized according to a repeating pattern or orientation. In some embodiments, provided amphiphilic polypeptide materials have a structural planes aligned with their orientation. In some embodiments, provided amphiphilic polypeptide materials are characterized by a tendency to cleave or split cleave along these distinct crystallographic structural planes.

In some embodiments, provided amphiphilic polypeptide materials are characterized by a beta sheet crystallinity with crystal orientation. That is, provided amphiphilic polypeptide materials substantially lack polymorphism.

In some embodiments, provided amphiphilic polypeptide materials are substantially polymorphic.

In some embodiments, provided amphiphilic polypeptide materials are characterized crystallography lattice planes or directions.

In some embodiments, provided amphiphilic polypeptide materials are characterized in that they comprise submicron size or nano-sized crystallized spheres and/or particles. In some embodiments, provided amphiphilic polypeptide materials are characterized in that they comprise micron sized spheres and/or particles. In some embodiments, silk fibroin-based materials are characterized in that they comprise crystallized spheres and/or particles that have average dimensions in a ranges of between about 1 nm and about 5 micros. In some embodiments, submicron size or nano-sized crystallized spheres and/or particles have less than 500 nm average diameter, e.g., less than 400 nm, less than 450 nm, less than 400 nm, less than 350 nm, less than 300 nm, less than 250 nm, less than 200 nm, less than 190 nm, less than 180 nm, less than 170 nm, less than 160 nm, less than 150 nm, less than 145 nm, less than 140 nm, less than 135 nm, less than 130 nm, less than 125 nm, less than 120 nm, less than 115 nm, less than 110 nm, less than 100 nm, less than 90 nm, less than 80 nm, less than 70 nm, less than 60 nm, less than 50 nm, less than 40 nm, less than 30 nm, less than 20 nm, less than 15 nm, less than 10 nm, less than 5 nm, or smaller. In some preferred embodiments, submicron size or nano-sized crystallized spheres and/or particles have average diameters of less than 100 nm.

In some embodiments, provided amphiphilic polypeptide materials display anisotropy. In some embodiments, provided amphiphilic polypeptide materials display anisotropy with respect to crystallinity of its amphiphilic polypeptides. In some embodiments, provided amphiphilic polypeptide materials display anisotropy with respect to mechanical properties according to the alignment of the crystal planes. In some embodiments, provided amphiphilic polypeptide materials are characterized in that certain properties of such materials are dependent on a degree of crystallinity or crystal direction.

In some embodiments, provided amphiphilic polypeptide materials are characterized by a network of amphiphilic polypeptide globules.

In some embodiments, microstructures comprise amphiphilic polypeptide globules. In some embodiments, microstructures comprise a plurality of amphiphilic polypeptide globules.

In some embodiments, amphiphilic polypeptide globules are nano-globules. In some embodiments, amphiphilic polypeptide globules have at least one dimension between about 5 nm and about 30 nm.

In some embodiments, a size distribution of amphiphilic polypeptide globules of a plurality of globules may affect mechanical properties of provided amphiphilic polypeptide materials.

In some embodiments, amphiphilic polypeptide globules aggregate with one another. In some embodiments, amphiphilic polypeptide globules aggregate by interlocking with one another. In some embodiments, amphiphilic polypeptide globules aggregate by interacting with one another to form a dense network.

In some embodiments, a plurality of amphiphilic polypeptide globules that are aggregated or interlocked with each other have a packing density. In some embodiments, a packing density may affect mechanical properties of provided amphiphilic polypeptide materials. In some embodiments, tightly packed amphiphilic polypeptide globules have different mechanical properties than loosely packed amphiphilic polypeptide globules.

In some embodiments, amphiphilic polypeptide globules aggregate to form amphiphilic polypeptide microstructures. In some embodiments, globules aggregate with one another so that they form an interlocking microstructure. In some embodiments, amphiphilic polypeptide globules aggregate to form structures of tens to hundreds of nanometers in size. In some embodiments, amphiphilic polypeptide globules aggregate through interactions with one another. In some embodiments, amphiphilic polypeptide globules interact with each other to form a dense network. In some embodiments, a globular packing density may affect mechanical properties of provided materials.

The Kaplan Application does not disclose formation of distinct crystallographic structural planes with mechanical properties that are directionally dependent.

By contrast, in some embodiments, provided amphiphilic polypeptide materials are characterized by anisotropy with respect to properties, for example, including compressive modulus in a range between about 500 Pa and about 3000 kPa.

In some embodiments, provided amphiphilic polypeptide materials are characterized by toughness, a threshold resistance to damage from pressure or an impact.

In some embodiments, provided amphiphilic polypeptide materials are characterized fracture toughness, that is an indication of an amount of stress required to propagate a preexisting flaw.

In some embodiments, provided amphiphilic polypeptide materials are susceptible to compression. In some embodiments, provided amphiphilic polypeptide materials show compression relative to the pre-compression initial volume, up to: at least about 25 % of their original volume after compression, at least about 30 % of their original volume after compression, at least about 35 % of their original volume after compression, at least about 40 % of their original volume after compression, at least about 45 % of their original volume after compression, at least about 50 % of their original volume after compression, at least about 55 % of their original volume after compression, at least about 60 % of their original volume after compression, at least about 65 % of their original volume after compression, at least about 70 % of their original volume after compression, at least about 75 % of their original volume after compression, at least about 80 % of their original volume after compression, at least about 85 % of their original volume after compression, at least about 90 % of their original volume after compression, at least about 95 % of their original volume after compression, or about 100 % of their original volume after compression.

In some embodiments, provided amphiphilic polypeptide materials are characterized by an elastic modulus value in a range between about 1 kPa and about 2500 kPa. In some embodiments, provided amphiphilic polypeptide materials are characterized by an elastic modulus of: less than about 1 kPa, less than about 2 kPa, less than about 3 kPa, less than about 4 kPa, less than about 5 kPa, less than about 6 kPa, less than about 7 kPa, less than about 8 kPa, less than about 9 kPa, less than about 10 kPa, less than about 15 kPa, less than about 20 kPa, less than about 25 kPa, less than about 30 kPa, less than about 35 kPa, less than about 40 kPa, less than about 45 kPa, less than about 50 kPa, less than about 55 kPa, less than about 60 kPa, less than about 65 kPa, less than about 70 kPa, less than about 75 kPa, less than about 80 kPa, less than about 85 kPa, less than about 90 kPa, less than about 95 kPa, less than about 100 kPa, less than about 125 kPa, less than about 150 kPa, less than about 175 kPa, less than about 200 kPa, less than about 225 kPa, less than about 250 kPa, less than about 275 kPa, less than about 300 kPa, less than about 325 kPa, less than about 350 kPa, less than about 375 kPa, less than about 400 kPa, less than about 425 kPa, less than about 450 kPa, less than about 475 kPa, less than about 500 kPa, less than about 600 kPa, less than about 700 kPa, less than about 800 kPa, less than about 900 kPa, less than about 1000 kPa, less than about 1100 kPa, less than about 1200 kPa, less than about 1300 kPa, less than about 1400 kPa, less than about 1500 kPa, less than about 1600 kPa, less than about 1700 kPa, less than about 1800 kPa, less than about 1900 kPa, less than about 2000 kPa, less than about 2100 kPa, less than about 2200 kPa, less than about 2300 kPa, less than about 2400 kPa, or less than about 2500 kPa.

In some embodiments, provided amphiphilic polypeptide materials are characterized by a compressive modulus value in a range between about 500 Pa and about 3000 kPa. In some embodiments, provided amphiphilic polypeptide materials are characterized by a compressive modulus of: less than about 500 Pa, less than about 600 Pa, less than about 700 Pa, less than about 800 Pa, less than about 900 Pa, less than about 1 kPa, less than about 2 kPa, less than about 3 kPa, less than about 4 kPa, less than about 5 kPa, less than about 6 kPa, less than about 7 kPa, less than about 8 kPa, less than about 9 kPa, less than about 10 kPa, less than about 15 kPa, less than about 20 kPa, less than about 25 kPa, less than about 30 kPa, less than about 35 kPa, less than about 40 kPa, less than about 45 kPa, less than about 50 kPa, less than about 55 kPa, less than about 60 kPa, less than about 65 kPa, less than about 70 kPa, less than about 75 kPa, less than about 80 kPa, less than about 85 kPa, less than about 90 kPa, less than about 95 kPa, less than about 100 kPa, less than about 150 kPa, less than about 200 kPa, less than about 250 kPa, less than about 500 kPa, less than about 750 kPa, less than about 1 MPa, less than about 2.5 MPa, less than about 5 MPa, less than about 10 MPa, less than about 20 MPa, less than about 30 MPa, less than about 40 MPa, less than about 50 MPa, less than about 100 MPa, less than about 200 MPa, less than about 300 MPa, less than about 400 MPa, less than about 500 MPa, less than about 600 MPa, less than about 700 MPa, less than about 800 MPa, less than about 900 MPa, less than about 1 GPa, less than about 2.5 MPa, less than about 5 GPa, less than about 10 GPa, less than about 20 GPa, less than about 30 GPa, less than about 40 GPa, less than about 50 GPa, or less than about 100 GPa.

In some embodiments, provided amphiphilic polypeptide materials are characterized by a storage modulus value in a range between about 1 kPa and about 3000 kPa. In some embodiments, provided amphiphilic polypeptide materials are characterized by a compressive modulus of: less than about 1 kPa, less than about 2 kPa, less than about 3 kPa, less than about 4 kPa, less than about 5 kPa, less than about 6 kPa, less than about 7 kPa, less than about 8 kPa, less than about 9 kPa, less than about 10 kPa, less than about 15 kPa, less than about 20 kPa, less than about 25 kPa, less than about 30 kPa, less than about 35 kPa, less than about 40 kPa, less than about 45 kPa, less than about 50 kPa, less than about 55 kPa, less than about 60 kPa, less than about 65 kPa, less than about 70 kPa, less than about 75 kPa, less than about 80 kPa, less than about 85 kPa, less than about 90 kPa, less than about 95 kPa, less than about 100 kPa, less than about 125 kPa, less than about 150 kPa, less than about 175 kPa, less than about 200 kPa, less than about 225 kPa, less than about 250 kPa, less than about 275 kPa, less than about 300 kPa, less than about 325 kPa, less than about 350 kPa, less than about 375 kPa, less than about 400 kPa, less than about 425 kPa, less than about 450 kPa, less than about 475 kPa, less than about 500 kPa, less than about 600 kPa, less than about 700 kPa, less than about 800 kPa, less than about 900 kPa, less than about 1000 kPa, less than about 1100 kPa, less than about 1200 kPa, less than about 1300 kPa, less than about 1400 kPa, less than about 1500 kPa, less than about 1600 kPa, less than about 1700 kPa, less than about 1800 kPa, less than about 1900 kPa, less than about 2000 kPa, less than about 2100 kPa, less than about 2200 kPa, less than about 2300 kPa, less than about 2400 kPa, less than about 2500 kPa, less than about 2600 kPa, less than about 2700 kPa, less than about 2800 kPa, less than about 2900 kPa, or less than about 3000 kPa.

In some embodiments, provided amphiphilic polypeptide materials are characterized by a resistance to deformation from an applied shear stress of about 100 Pa, about 200 Pa, about 300 Pa, about 400 Pa, about 500 Pa, about 1 kPa, about 2 kPa, about 3 kPa, about 4 kPa, about 5 kPa, about 10 kPa, about 15 kPa, about 20 kPa, about 30 kPa, about 40 kPa, about 50 kPa, about 60 kPa, about 70 kPa, about 80 kPa, about 90 kPa, about 100 kPa, about 200 kPa, about 300 kPa, about 400 kPa, about 500 kPa, about 600 kPa, about 700 kPa, about 800 kPa, about 900 kPa, about 1 MPa, about 2 MPa, about 3 MPa, about 4 MPa, about 5 MPa, about 10 MPa, about 20 MPa, about 30 MPa, about 40 MPa, about 50 MPa, about 60 MPa, about 70 MPa, about 80 MPa, about 90 MPa, about 100 MPa, about 200 MPa, about 300 MPa, about 400 MPa, about 500 MPa, about 600 MPa, about 700 MPa, about 800 MPa, about 900 MPa, or about 1 GPa.

In some embodiments, provided amphiphilic polypeptide materials are characterized by a resistance to damage from an applied pull-out force of about 0.1 N, about 0.5 N, about 1 N,. about 2 N, about 3 N, about 4 N, about 5 N, about 6 N, about 7 N, about 8 N, about 9 N, about 10 N, about 11 N, about 12 N, about 13 N, about 14 N, about 15 N, about 16 N, about 17 N, about 18 N, about 19 N, about 20 N, about 21 N, about 22 N, about 23 N, about 24 N, about 25 N, about 30 N, about 35 N, about 40 N, about 45 N, about 50 N, about 55 N, about 60 N, about 65 N, about 70 N, about 75 N, about 80 N, about 85 N, about 90 N, about 95 N, about 100 N, about 105 N,. about 110 N, about 115 N, about 120 N, about 125 N, about 130 N, about 135 N, about 140 N, about 145 N, about 150 N, about 155 N, about 160 N, about 165 N, about 170 N, about 175 N, about 180 N, about 185 N, about 190 N, about 195 N, about 200 N, about 205 N, about 210 N, about 215 N, about 220 N, about 225 N, about 230 N, about 235 N, about 240 N, about 245 N, about 250 N, about 260 N, about 270 N, about 280 N, about 290 N, about 300 N, about 350 N, about 400 N, about 450 N, about 500 N, about 550 N or more.

In some embodiments, provided amphiphilic polypeptide materials are characterized in that they include amphiphilic polypeptides and fragments thereof within a particular molecular weight range so that, as described herein, the materials display one or more desirable characteristics. In some embodiments, provided technologies for selecting, designing, and/or producing amphiphilic polypeptide materials as described herein include selection, design and/or production of amphiphilic polypeptide materials within a particular molecular weight range, so that materials having one or more desired (e.g., pre-determined desired) characteristics are provided. In some embodiments, for example, a response of provided amphiphilic polypeptides materials to an applied compression, expansion, or deformation is directly correlated to a molecular weight of its amphiphilic polypeptides and/or fragments thereof. In some embodiments, when the molecular weight of amphiphilic polypeptides and/or fragments which comprise an amphiphilic polypeptide material increases, a compressive modulus for the material increases. In some embodiments, when the molecular weight of amphiphilic polypeptides and/or fragments which comprise an amphiphilic polypeptide material increases, a shear stress value for the material increases. In some embodiments, when the molecular weight of amphiphilic polypeptides and/or fragments which comprise an amphiphilic polypeptide material increases, a pull out strength for the material increases.

In some embodiments, provided amphiphilic polypeptide materials comprise an exposed surface.

In some embodiments, an exposed surface of provided amphiphilic polypeptide materials is approximately flat.

In some embodiments, an exposed surface of provided amphiphilic polypeptide materials has a surface roughness that is in the order of only few nm. In some embodiments, provided amphiphilic polypeptide materials have a surface roughness that is characterized by small deviations so that such a surface enables integration of electronic component. In some embodiments, provided amphiphilic polypeptide materials have a root mean square ("RMS") surface roughness of less than about 5 nm. In some embodiments, provided amphiphilic polypeptide materials have an RMS surface roughness of about 4.0 nm, about 3.5 nm, about 3.0 nm, about 2.5 nm, about 2.0 nm, about 1.5 nm, or about 1 nm. In some embodiments, provided amphiphilic polypeptide materials have an RMS surface roughness of less than about 1 nm.

In some embodiments, an exposed surface of provided amphiphilic polypeptide materials comprise a pattern formed in or on its surface. In some embodiments, provided amphiphilic polypeptide materials comprise a nanopattern formed in or on its surface. The term "nanopatterned" as used herein refers to patterning provided on a surface having structural features of a size that can be appropriately measured on a nanometer scale, for example, between about a nanometer and a few microns are typical of the patterning used in accordance with the present disclosure.

In some embodiments, a pattern is raised or elevated relative to a surface of an amphiphilic polypeptide material. In some embodiments, a pattern is sunken or recessed relative to a surface of an amphiphilic polypeptide material. In some embodiments, a pattern or a nanopattern is formed in a surface according to means know in the art, for example, e-beam lithography, nanoimprinting, etc.

In some embodiments, provided amphiphilic polypeptide materials are useful as optical devices. In some embodiments, provided amphiphilic polypeptide materials may be molded, machined, or otherwise fabricated as an optical device, including, for example, diffraction gratings, photonic crystals, optofluidic devices, waveguides, and the like. In some embodiments, a nanopattern formed on a surface of provided amphiphilic polypeptide materials is characterized in that when exposed to a specific wavelength of light. (See, for example, WO 2008/127404; WO 2008/118211; WO 2008/127402; WO 2008/127403; WO 2008/127401; WO 2008/140562; WO 2009/061823; WO 2009/155397; WO 2010/126640; WO 2011/046652; WO 2011/026101; WO 2012/054121; WO 2011/130335; WO 2011/112931; WO 2012/047682; WO 2012/031282; WO 2010/088585; WO 2013/130156).

In some embodiments, provided amphiphilic polypeptide materials contain, encompass, include, incorporate, or store additives, agents, and/or functional moieties. In some embodiments, provided amphiphilic polypeptide materials encapsulate, encompass, include, incorporate, and/or store additives, agents, and/or functional moieties within its material monolithic construct.

In some embodiments, provided amphiphilic polypeptide materials are biocompatible.

In some embodiments, provided amphiphilic polypeptide materials additives, agents, and/or functional moieties are or comprise labile or biolabile compounds. In some embodiments, provided amphiphilic polypeptide materials additives, agents, and/or functional moieties are or comprise macromolecules and/or biologically active molecules. In some embodiments, agents, additives, and/or functional moieties are or comprise biological activity or biological functionality. In some embodiments, provided amphiphilic polypeptide materials that comprise biologically functional additives, agents, and/or functional moieties that at the cellular level can be used or support functions such as attack, defense, prevention, and/or protection.

In some embodiments, all aqueous processing under ambient conditions is favorable. In some embodiments, when compared with prior materials that use or require non-aqueous solvents during manufacturing or include or any residual non-aqueous solvents, such as HFIP and/or methanol, provided amphiphilic polypeptide materials show a superior ability to encapsulate, encompass, include, incorporate, and/or store biologically active additives, agents, and/or functional moieties so that they maintain, and/or preserve their biological activity and/or functionality.

In some embodiments, provided amphiphilic polypeptide materials are characterized in that when an additive or agent having biological activity or functionality is added, encapsulated, encompassed, included, incorporated, and/or stored in a provided amphiphilic polypeptide materials, the agent, additive, and/or functional moiety' structure is retained and/or its biological activity is not materially degraded, inhibited, and/or reduced.

In some embodiments, provided amphiphilic polypeptide materials preserve the activity of encapsulated additives, agents, and/or functional moieties that are biologically active. In some embodiments, provided amphiphilic polypeptide materials are arranged and constructed so that when additives, agents, and/or functional moieties that are biologically active are encapsulated in the material, the material is characterized in that the structure and/or biological activity of such additives, agents, and/or functional moieties is retained so that it is not materially degraded, reduced, and/or inhibited. While not wishing to be bound to a specific theory, when an additive, agent and/or functional moiety is encapsulated within a monolith as described herein it is believed that oxidative stress is reduced.

In some embodiments, when a biologically active additive, agent, and/or functional moiety is added, encapsulated, encompassed, included, incorporated, and/or stored in an amphiphilic polypeptide material as described herein, the biologically active material retains its activity such that it is not materially degraded, reduced, and/or inhibited for up to about 1 day, about 2 days, about 3 days, about 4 days, about 5 days, about 6 days, about 7 days, about 8 days, about 9 days, about 10 days, about 11 days, about 12 days, about 13 days, about 14 days, about 3 weeks, about 4 weeks, about 5 weeks, about 6 weeks, about 7 weeks, about 8 weeks, about 9 weeks, about 10 weeks, about 2 months, about 3 months, about 4 months, about 5 months, about 6 months, about 7 months, about 8 months, about 9 months, about 10 months, about 11 months, about 1 year, about 2 years or more.

In some embodiments, provided amphiphilic polypeptide materials are maintained outside 'normal' storage conditions. In some embodiments, normal storage conditions include about ambient temperature, pressure, and/or relative humidity. In some embodiments, when exposed to outside 'normal' storage conditions, provided amphiphilic polypeptide materials preserve the activity of encapsulated additives, agents, and/or functional moieties that are biologically active.

In some embodiments, provided amphiphilic polypeptide materials store macromolecules and/or biologically active molecules in its monolithic construct. In some embodiments, additives, agents, and/or functional moieties are evenly distributed though provided amphiphilic polypeptide materials. In some embodiments, additives, agents, and/or functional moieties are concentrated in a specific portion or area of provided amphiphilic polypeptide materials relative to the whole of such provided amphiphilic polypeptide materials. In some embodiments, additives, agents, and/or functional moieties are concentrated on a surface of such provided amphiphilic polypeptide materials.

In some embodiments, provided amphiphilic polypeptide materials are biodegradable.

In some embodiments, provided amphiphilic polypeptide materials are characterized in that such materials decompose, degrade, delaminate, or dissolve. In some embodiments, silk fibroin-based materials are characterized in that such materials decompose, degrade, delaminate, or dissolve to release an additive, agent, and/or functional moiety.

In some embodiments, provided amphiphilic polypeptide materials are introduced *in vivo*. In some embodiments, provided amphiphilic polypeptide materials decompose, degrade, delaminate, or dissolve when present *in vivo*. In some embodiments, provided amphiphilic polypeptide materials decompose, degrade, delaminate, or dissolve without significant immunological response when present *in vivo*. In some embodiments, provided amphiphilic polypeptide materials exhibit predictable degradation kinetics. In some embodiments, provided amphiphilic polypeptide materials are resorbed in vivo and replaced with natural tissues.

Materials described in the Kaplan Application and use hexafluoroisopropanol (HFIP), trifluoroisopropanol (TFIP) or formic acid (FA) as protein solvents for the fabrication of fibroin solid structures undergo several processes (i.e. freeze-drying, dissolution in the fluorinated solvents and exposure to coagulation bath) to be prepared for the formation of blocks.

All these steps are not only time consuming but also are a hindrance for the incorporation of biologically active molecules or macromolecules in general into the fibroin structure, which is the base for exerting the stabilization and preservation properties of the protein and to provide biochemical and biological features to the protein constructs.

In some embodiments, materials provide osteogenic functionality.

In some embodiments, aqueous processing allows incorporation of bioactive molecules into orthopedic devices. In some embodiments, bioactives functionalize orthopedic devices. In some embodiments, bioactive impart osteoinductive and antimicrobial features.

In some embodiments, biocomposite orthopedic devices are or comprise biocomposite screws. In some embodiments, biocomposite screws comprise silk/hydroxyapatite and silk/silica.

In some embodiments, orthopedic devices improve biocompatibility and provide osteoconductive potential.

In some embodiments, when compared with an organic solvent-based amphiphilic solution, at least one advantage of an aqueous amphiphilic solution is formulation of materials, such as orthopedic devices with similar mechanical properties but eliminating risks associate with the use of harmful solvents. Furthermore, in some embodiments, mild aqueous-based processing facilitates an addition of dopants and bioactive compounds, thus providing additional clinical benefits like osteoinductive, osteoconductive and anti-inflammatory features.

Silk Fibroin-Based Materials

Provided amphiphilic polypeptide materials are or comprise silk fibroin and/or silk fibroin fragments.

Silk fibers spun by spiders and silkworms exhibit exceptional mechanical properties with a unique combination of strength, extensibility and toughness. In some embodiments, mechanical properties of provided silk materials can be tuned through control of its fabrication process.

*Silks*

In some embodiments, an amphiphilic polypeptide is silk. Silk is a natural protein fiber produced in a specialized gland of certain organisms. Silk proteins are fibrous proteins synthesized through a sol-gel-solid transition. In the gland of silkworms and spiders, water-soluble silk fibroin undergoes to a sol-gel transition during the spinning process, which ultimately leads to the formation of a solid, water insoluble, anisotropic, tough and flexible fibre, characterized by an antiparallel beta-sheet structure, with the crystals aligned towards the fibre's long axis. Silk production in organisms is especially common in the Hymenoptera (bees, wasps, and ants), and is sometimes used in nest construction. Other types of arthropod also produce silk, most notably various arachnids such as spiders (e.g., spider silk). Silk fibers generated by insects and spiders represent the strongest natural fibers known and rival even synthetic high performance fibers.

Silk has been a highly desired and widely used textile since its first appearance in ancient China (see for example Elisseeff, "The Silk Roads: Highways of Culture and Commerce," Berghahn Books/UNESCO, New York (2000); see also Vainker, "Chinese Silk: A Cultural History," Rutgers University Press, Piscataway, New Jersey (2004)). Glossy and smooth, silk is favored by not only fashion designers but also tissue engineers because it is mechanically tough but degrades harmlessly inside the body, offering new opportunities as a highly robust and biocompatible material substrate (see for example, Altman et al., Biomaterials, 24: 401 (2003); see also Sashina et al., Russ. J. Appl. Chem., 79: 869 (2006)).

Silk is naturally produced by various species, including, without limitation: Antheraea mylitta; Antheraea pernyi; Antheraea yamamai; Galleria mellonella; Bombyx mori; Bombyx mandarina; Galleria mellonella; Nephila clavipes; Nephila senegalensis; Gasteracantha mammosa; Argiope aurantia; Araneus diadematus; Latrodectus geometricus; Araneus bicentenarius; Tetragnatha versicolor; Araneus ventricosus; Dolomedes tenebrosus; Euagrus chisoseus; Plectreurys tristis; Argiope trifasciata; and Nephila madagascariensis.

In general, silk for use in accordance with the present disclosure may be produced by any such organism, from a recombinant source or may be prepared through an artificial process, for example, involving genetic engineering of cells or organisms to produce a silk protein and/or chemical synthesis. In some embodiments, of the present disclosure, silk is produced by the silkworm, *Bombyx mori.*

As is known in the art, silks are modular in design, with large internal repeats flanked by shorter (~100 amino acid) terminal domains (N and C termini). Naturally-occurring silks have high molecular weight (200 to 350 kDa or higher) with transcripts of 10,000 base pairs and higher and > 3000 amino acids (reviewed in Omenetto and Kaplan (2010) Science 329: 528-531). The larger modular domains are interrupted with relatively short spacers with hydrophobic charge groups in the case of silkworm silk. N- and C-termini are involved in the assembly and processing of silks, including pH control of assembly. The N- and C-termini are highly conserved, in spite of their relatively small size compared with the internal modules. Table 1, below, provides an exemplary list of silk-producing species and silk proteins:

A. Silkworms

| Accession | Species | Producing gland | Protein |
|-----------|---------|-----------------|---------|
| AAN28165 | *Antheraea mylitta* | Salivary | Fibroin |
| AAC32606 | *Antheraea pernyi* | Salivary | Fibroin |
| AAK83145 | *Antheraea yamamai* | Salivary | Fibroin |
| AAG10393 | *Galleria mellonella* | Salivary | Heavy-chain fibroin (N-terminal) |
| AAG10394 | *Galleria mellonella* | Salivary | Heavy-chain fibroin (C-terminal) |
| P05790 | *Bombyx mori* | Salivary | Fibroin heavy chain precursor, Fib-H, H-fibroin |
| CAA27612 | *Bombyx mandarina* | Salivary | Fibroin |
| Q26427 | *Galleria mellonella* | Salivary | Fibroin light chain precursor, Fib-L, L-fibroin, PG-1 |
| P21828 | *Bombyx mori* | Salivary | Fibroin light chain precursor, Fib-L, L-fibroin |

B. Spiders

**Table 1:** An exemplary list of silk-producing species and silk proteins (adopted from Bini et al. (2003), J. Mol. Biol. 335(1): 27-40).

| Accession | Species | Producing gland | Protein |
|-----------|---------|-----------------|---------|
| P19837 | *Nephila clavipes* | Major ampullate | Spidroin 1, dragline silk fibroin 1 |
| P46804 | *Nephila clavipes* | Major ampullate | Spidroin 2, dragline silk fibroin 2 |
| AAK30609 | *Nephila senegalensis* | Major ampullate | Spidroin 2 |
| AAK30601 | *Gasteracantha mammosa* | Major ampullate | Spidroin 2 |
| AAK30592 | *Argiope aurantia* | Major ampullate | Spidroin 2 |
| AAC47011 | *Araneus diadematus* | Major ampullate | Fibroin-4, ADF-4 |
| AAK30604 | *Latrodectus geometricus* | Major ampullate | Spidroin 2 |
| AAC04503 | *Araneus bicentenarius* | Major ampullate | Spidroin 2 |
| AAK30615 | *Tetragnatha versicolor* | Major ampullate | Spidroin 1 |
| AAN85280 | *Araneus ventricosus* | Major ampullate | Dragline silk protein-1 |
| AAN85281 | *Araneus ventricosus* | Major ampullate | Dragline silk protein-2 |
| AAC14589 | *Nephila clavipes* | Minor ampullate | MiSp1 silk protein |
| AAK30598 | *Dolomedes tenebrosus* | Ampullate | Fibroin 1 |
| AAK30599 | *Dolomedes tenebrosus* | Ampullate | Fibroin 2 |
| AAK30600 | *Euagrus chisoseus* | Combined | Fibroin 1 |
| AAK30610 | *Plectreurys tristis* | Larger ampule-shaped | Fibroin 1 |
| AAK30611 | *Plectreurys tristis* | Larger ampule-shaped | Fibroin 2 |
| AAK30612 | *Plectreurys tristis* | Larger ampule-shaped | Fibroin 3 |
| AAK30613 | *Plectreurys tristis* | Larger ampule-shaped | Fibroin 4 |
| AAK30593 | *Argiope trifasciata* | Flagelliform | Silk protein |
| AAF36091 | *Nephila madagascariensis* | Flagelliform | Fibroin, silk protein (N-terminal) |
| AAF36092 | *Nephila madagascariensis* | Flagelliform | Silk protein (C-terminal) |

(continued)

| Accession | Species | Producing gland | Protein |
|---|---|---|---|
| AAC38846 | *Nephila clavipes* | Flagelliform | Fibroin, silk protein (N-terminal) |
| AAC38847 | *Nephila clavipes* | Flagelliform | Silk protein (C-terminal) |

*Silk Fibroin*

Fibroin is a type of structural protein produced by certain spider and insect species that produce silk. Cocoon silk produced by the silkworm, *Bombyx mori,* is of particular interest because it offers low-cost, bulk-scale production suitable for a number of commercial applications, such as textile.

Silkworm cocoon silk contains two structural proteins, the fibroin heavy chain (~ 350 kDa) and the fibroin light chain (~ 25 kDa), which are associated with a family of non-structural proteins termed sericin, which glue the fibroin brings together in forming the cocoon. The heavy and light chains of fibroin are linked by a disulfide bond at the C-terminus of the two subunits (see for example Takei, F., Kikuchi, Y., Kikuchi, A., Mizuno, S. and Shimura, K. (1987) 105 J. Cell Biol., 175-180; see also Tanaka, K., Mori, K. and Mizuno, S. 114 J. Biochem. (Tokyo), 1-4 (1993); Tanaka, K., Kajiyama, N., Ishikura, K., Waga, S., Kikuchi, A., Ohtomo, K., Takagi, T. and Mizuno, S., 1432 Biochim. Biophys. Acta., 92-103 (1999); Y Kikuchi, K Mori, S Suzuki, K Yamaguchi and S Mizuno, "Structure of the Bombyx mori fibroin light-chain-encoding gene: upstream sequence elements common to the light and heavy chain," 110 Gene, 151-158 (1992)). The sericins are a high molecular weight, soluble glycoprotein constituent of silk which gives the stickiness to the material. These glycoproteins are hydrophilic and can be easily removed from cocoons by boiling in water.

Fibroin is maintained water-soluble in the silk gland by a fine regulation of the protein environmental conditions (e.g. pH and concentration of chaotropic ions) similarly crystalline fibroin can be regenerated (i.e. re-solubilized) in vitro by using highly concentrated solutions of chaotropic ions (e.g. lithium bromide), which drive the unfolding of silk fibroin into amorphous form, where the formation of intra-and inter molecular hydrogen bonds is endergonic.

As used herein, the term "silk fibroin" refers to silk fibroin protein, whether produced by silkworm, spider, or other insect, or otherwise generated (see for example, Lucas et al., 13 Adv. Protein Chem., 107-242 (1958)). In some embodiments, silk fibroin is obtained from a solution containing a dissolved silkworm silk or spider silk. For example, in some embodiments, silkworm silk fibroins are obtained, from the cocoon of *Bombyx mori.* In some embodiments, spider silk fibroins are obtained, for example, from *Nephila clavipes.* In the alternative, in some embodiments, silk fibroins suitable for use in the invention are obtained from a solution containing a genetically engineered silk harvested from bacteria, yeast, mammalian cells, transgenic animals or transgenic plants. (See, e.g., WO 97/08315 and U.S. Patent No. 5,245, 012).

Thus, a silk solution is used to fabricate compositions of the present disclosure contains fibroin proteins, essentially free of sericins. In some embodiments, silk solutions used to fabricate various compositions of the present disclosure contain the heavy chain of fibroin, but are essentially free of other proteins. In other embodiments, silk solutions used to fabricate various compositions of the present disclosure contain both the heavy and light chains of fibroin, but are essentially free of other proteins. In certain embodiments, silk solutions used to fabricate various compositions of the present disclosure comprise both a heavy and a light chain of silk fibroin; in some such embodiments, the heavy chain and the light chain of silk fibroin are linked via at least one disulfide bond. In some embodiments, where the heavy and light chains of fibroin are present, they are linked via one, two, three or more disulfide bonds. Although different species of silk-producing organisms, and different types of silk, have different amino acid compositions, various fibroin proteins share certain structural features. A general trend in silk fibroin structure is a sequence of amino acids that is characterized by usually alternating glycine and alanine, or alanine alone. Such configuration allows fibroin molecules to self-assemble into a beta-sheet conformation. These "Alanine-rich" hydrophobic blocks are typically separated by segments of amino acids with bulky side-groups (e.g., hydrophilic spacers).

In some embodiments, core repeat sequences of the hydrophobic blocks of fibroin are represented by the following amino acid sequences and/or formulae:

$(GAGAGS)_{5-15}$ (SEQ **ID** NO: 1);

$(GX)_{5-15}$ (X = V, I, A) (SEQ **ID** NO: 2);

GAAS (SEQ ID NO: 3);

$(S_{1-2}A_{11-13})$

(SEQ ID NO: 4);

GX$_{1-4}$ GGX (SEQ ID NO: 5);

GGGX (X = A, S, Y, R, D V, W, R, D) (SEQ IDNO: 6);

(S1-2A1-4)$_{1-2}$ (SEQ ID NO: 7);

GLGGLG (SEQ ID NO: 8);

GXGGXG (X = L, I, V, P) (SEQ ID NO: 9);

GPX (X = L, Y, I); (GP(GGX)$_{1-4}$ Y)n (X = Y, V, S, A) (SEQ ID NO: 10);

GRGGAn (SEQ ID NO: 11);

GGXn (X = A, T, V, S) ; GAG(A)$_{6-7}$GGA (SEQ ID NO: 12); and

GGX GX GXX (X = Q, Y, L, A, S, R) (SEQ ID NO: 13).

In some embodiments, a fibroin peptide contains multiple hydrophobic blocks, e.g., 3, 4, 5, 6, 7, 8, 9, 10, 11, 12, 13, 14, 15, 16, 17, 18, 19 and 20 hydrophobic blocks within the peptide. In some embodiments, a fibroin peptide contains between 4-17 hydrophobic blocks. In some embodiments, a fibroin peptide comprises at least one hydrophilic spacer sequence ("hydrophilic block") that is about 4-50 amino acids in length. In some embodiments, nonlimiting examples of the hydrophilic spacer sequences include:

TGSSGFGPYVNGGYSG (SEQ ID NO: 14);

YEYAWSSE (SEQ ID NO: 15);

SDFGTGS (SEQ ID NO: 16);

RRAGYDR (SEQ ID NO: 17);

EVIVIDDR(SEQ ID NO: 18);

TTIIEDLDITIDGADGPI (SEQ ID NO: 19) and

TISEELTI (SEQ ID NO: 20).

In some embodiments, a fibroin peptide contains a hydrophilic spacer sequence that is a derivative of any one of the representative spacer sequences listed above. In some embodiments, such derivatives are at least 75%, at least 80%, at least 85%, at least 90%, or at least 95% identical to any one of the hydrophilic spacer sequences.

In some embodiments, a fibroin peptide suitable for the present disclosure contains no spacer.

In some embodiments, silks are fibrous proteins and are characterized by modular units linked together to form high molecular weight, highly repetitive proteins. In some embodiments, modular units or domains, each with specific amino acid sequences and chemistries, are thought to provide specific functions. For example, sequence motifs such as poly-alanine (polyA) and polyalanine-glycine (poly-AG) are inclined to be beta-sheet-forming; GXX motifs contribute to 31-helix formation; GXG motifs provide stiffness; and, GPGXX (SEQ ID NO: 22) contributes to beta-spiral formation. These are examples of key components in various silk structures whose positioning and arrangement are intimately tied with the end material properties of silk-based materials (reviewed in Omenetto and Kaplan (2010) Science 329: 528-531).

It has been observed that the beta-sheets of fibroin proteins stack to form crystals, whereas the other segments form amorphous domains. It is the interplay between the hard crystalline segments, and the strained elastic semi amorphous regions, that gives silk its extraordinary properties. An exemplary list of hydrophobic and hydrophilic components of fibroin sequences may be found for example in International Patent Publication Number WO 2011/130335.

Silk materials explicitly exemplified herein were typically prepared from material spun by silkworm, *Bombyx mori.* Typically, cocoons are boiled in an aqueous solution of 0.02 M Na$_2$CO$_3$, then rinsed thoroughly with water to extract the glue-like sericin proteins. Extracted silk is then dissolved in a solvent, for example, LiBr (such as 9.3 M) solution at room temperature. A resulting silk fibroin solution can then be further processed for a variety of applications as described elsewhere herein.

In some embodiments, polymers refers to peptide chains or polypeptides having an amino acid sequence corresponding to fragments derived from silk fibroin protein or variants thereof. In the context of silk fibroin-based materials of the present disclosure, silk fibroin fragments generally refer to silk fibroin peptide chains or polypeptides that are smaller than naturally occurring full length silk fibroin counterpart, such that one or more of the silk fibroin fragments within a population or composition. In some embodiments, for example, silk fibroin-based materials comprise silk fibroin polypeptides having an average molecular weight of between about 3.5 kDa and about 400 kDa. In some embodiments, suitable ranges of silk fibroin fragments include, but are not limited to: silk fibroin polypeptides having an average molecular weight of between about 3.5 kDa and about 200 kDa; silk fibroin polypeptides having an average molecular weight of between about 3.5 kDa and about 150 kDa; silk fibroin polypeptides having an average molecular weight of between about 3.5 kDa and about 120 kDa. In some embodiments, silk fibroin polypeptides have an average molecular weight of: about 3.5 kDa, about 4 kDa, about 4.5 kDa, about 5 kDa, about 6 kDa, about 7 kDa, about 8 kDa, about 9 kDa, about 10 kDa, about 15 kDa, about 20 kDa, about 25 kDa, about 30 kDa, about 35 kDa, about 40 kDa, about 45 kDa, about 50 kDa, about 55 kDa, about 60 kDa, about 65 kDa, about 70 kDa, about 75 kDa, about 80 kDa, about 85 kDa, about 90 kDa, about 95 kDa, about 100 kDa, about 105 kDa, about 110 kDa, about 115 kDa, about 120 kDa, about 125 kDa, about 150 kDa, about 200 kDa, about 250 kDa, about 300 kDa, about 350 kDa, or about 400 kDa. In some preferred embodiments, silk fibroin polypeptides have an average molecular weight of about 100 kDa.

In some embodiments, silk fibroin-based materials are or comprise silk fibroin and/or silk fibroin fragments. In some embodiments, silk fibroin and/or silk fibroin fragments of various molecular weights may be used. In some embodiments, silk fibroin and/or silk fibroin fragments of various molecular weights are silk fibroin polypeptides. In some embodiments, silk fibroin polypeptides are "reduced" in size, for instance, smaller than the original or wild type counterpart, may be referred to as "low molecular weight silk fibroin." For more details related to low molecular weight silk fibroins, see: U.S. provisional application concurrently filed herewith, entitled "LOW MOLECULAR WEIGHT SILK FIBROIN AND USES THEREOF,". In some embodiments, silk fibroin polypeptides have an average molecular weight of: less than 350 kDa, less than 300 kDa, less than 250 kDa, less than 200 kDa, less than 175 kDa, less than 150 kDa, less than 120 kDa, less than 100 kDa, less than 90 kDa, less than 80 kDa, less than 70 kDa, less than 60 kDa, less than 50 kDa, less than 40 kDa, less than 30 kDa, less than 25 kDa, less than 20 kDa, less than 15 kDa, less than 12 kDa, less than 10 kDa, less than 9 kDa, less than 8 kDa, less than 7 kDa, less than 6 kDa, less than 5 kDa, less than 4 kDa, less than 3.5 kDa, less than 3 kDa, less than 2.5 kDa, less than 2 kDa, less than 1.5 kDa, or less than about 1.0 kDa, etc.

*Degradation Properties of Silk and Silk-based Materials*

Additionally, as will be appreciated by those of skill in the art, much work has established that researchers have the ability to control the degradation process of silk. According to the present disclosure, such control can be particularly valuable in the fabrication of electronic components, and particularly of electronic components that are themselves and/or are compatible with biomaterials. Degradability (e.g., bio-degradability) is often essential for biomaterials used in tissue engineering and implantation. The present disclosure encompasses the recognition that such degradability is also relevant to and useful in the fabrication of silk electronic components.

According to the present disclosure, one particularly desirable feature of silk-based materials is the fact that they can be programmably degradable. That is, as is known in the art, depending on how a particular silk-based material is prepared, it can be controlled to degrade at certain rates. Degradability and controlled release of a substance from silk-based materials have been published (see, for example, WO 2004/080346, WO 2005/012606, WO 2005/123114, WO 2007/016524, WO 2008/150861, WO 2008/118133).

Control of silk material production methods as well as various forms of silk-based materials can generate silk compositions with known degradation properties. For example, using various silk fibroin-based materials entrapped agents such as therapeutics can be loaded in active form, which is then released in a controlled fashion, e.g., over the course of minutes, hours, days, weeks to months. It has been shown that layered silk fibroin coatings can be used to coat substrates of any material, shape and size, which then can be used to entrap molecules for controlled release, e.g., 2-90 days.

Additives, Agents, and/or Functional Moieties

In any of the embodiments embraced by the present invention, silk fibroin-based materials may further include one or more additives, agents, and/or functional moieties and other active or inactive agents, depending on particular use.

In some embodiments, provided silk fibroin-based materials can comprise one or more (e.g., one, two, three, four, five or more) additives, agents, and/or functional moieties. Without wishing to be bound by a theory, additives, agents, and/or functional moieties can provide or enhance one or more desirable properties, e.g., strength, flexibility, ease of processing and handling, biocompatibility, bioresorbability, surface morphology, release rates and/or kinetics of one or more active agents present in the composition, and the like. In some embodiments, one or more such additives, agents, and/or functional moieties can be covalently or non-covalently linked with the silk fibroin-based material (e.g., with a polymer such as silk fibroin that makes up the material) and can be integrated homogenously or heterogeneously within the silk composition.

In some embodiments, additives, agents, and/or functional moieties are or comprises a moiety covalently associated (e.g., via chemical modification or genetic engineering) with a polymer. In some embodiments, an additive is non-covalently associated with a silk fibroin-based material or silk fibroin-based material component.

In some embodiments, provided silk fibroin-based materials comprise additives, agents, and/or functional moieties at a total amount from about 0.01 wt% to about 99 wt%, from about 0.01 wt% to about 70 wt%, from about 5 wt% to about 60 wt%, from about 10 wt% to about 50 wt%, from about 15 wt% to about 45 wt%, or from about 20 wt% to about 40 wt%, of the total silk composition. In some embodiments, ratio of silk fibroin to additive in the composition can range from about 1000:1 (w/w) to about 1:1000 (w/w), from about 500:1 (w/w) to about 1:500 (w/w), from about 250:1 (w/w) to about 1:250 (w/w), from about 200:1 (w/w) to about 1:200 (w/w), from about 25:1 (w/w) to about 1:25 (w/w), from about 20:1 (w/w) to about 1:20 (w/w), from about 10:1 (w/w) to about 1:10 (w/w), or from about 5:1 (w/w) to about 1:5 (w/w).

In some embodiments, provided silk fibroin-based materials include one or more additives, agents, and/or functional moieties at a molar ratio relative to polymer (i.e., a silk:additive ratio) of, e.g., at least 1000:1, at least 900:1, at least 800:1, at least 700:1, at least 600:1, at least 500:1, at least 400:1, at least 300:1, at least 200:1, at least 100:1, at least 90:1, at least 80:1, at least 70:1, at least 60:1, at least 50:1, at least 40:1, at least 30:1, at least 20: 1, at least 10:1, at least 7:1, at least 5:1, at least 3:1, at least 1:1, at least 1:3, at least 1:5, at least 1:7, at least 1:10, at least 1:20, at least 1:30, at least 1:40, at least 1:50, at least 1:60, at least 1:70, at least 1:80, at least 1:90, at least 1:100, at least 1:200, at least 1:300, at least 1:400, at least 1:500, at least 600, at least 1:700, at least 1:800, at least 1:900, or at least 1:100.

In some embodiments, moiety silk:additive ratio is, e.g., at most 1000:1, at most 900: 1, at most 800:1, at most 700:1, at most 600:1, at most 500:1, at most 400: 1, at most 300:1, at most 200:1, 100: 1, at most 90:1, at most 80:1, at most 70:1, at most 60:1, at most 50:1, at most 40:1, at most 30:1, at most 20:1, at most 10:1, at most 7:1, at most 5:1, at most 3:1, at most 1:1, at most 1:3, at most 1:5, at most 1:7, at most 1:10, at most 1:20, at most 1:30, at most 1:40, at most 1:50, at most 1:60, at most 1:70, at most 1:80, at most 1:90, at most 1:100, at most 1:200, at most 1:300, at most 1:400, at most 1:500, at most 1:600, at most 1:700, at most 1:800, at most 1:900, or at most 1:1000.

In some embodiments, moiety silk:additive ratio is, e.g., from about 1000:1 to about 1:1000, from about 900:1 to about 1:900, from about 800:1 to about 1:800, from about 700:1 to about 1:700, from about 600:1 to about 1:600, from about 500:1 to about 1:500, from about 400:1 to about 1:400, from about 300:1 to about 1:300, from about 200:1 to about 1:200, from about 100:1 to about 1:100, from about 90:1 to about 1:90, from about 80:1 to about 1:80, from about 70:1 to about 1:70, from about 60:1 to about 1:60, from about 50:1 to about 1:50, from about 40:1 to about 1:40, from about 30:1 to about 1:30, from about 20:1 to about 1:20, from about 10:1 to about 1:10, from about 7:1 to about 1:7, from about 5:1 to about 1:5, from about 3:1 to about 1:3, or about 1:1.

In some embodiments, provided silk fibroin-based materials comprise additives, agents, and/or functional moieties, for example, therapeutic, preventative, and/or diagnostic agents.

In some embodiments, an additives, agents, and/or functional moieties is or comprises one or more therapeutic agents. In general, a therapeutic agent is or comprises a small molecule and/or organic compound with pharmaceutical activity (e.g., activity that has been demonstrated with statistical significance in one or more relevant pre-clinical models or clinical settings). In some embodiments, a therapeutic agent is a clinically-used drug. In some embodiments, a therapeutic agent is or comprises an cells, proteins, peptides, nucleic acid analogues, nucleotides, oligonucleotides, nucleic acids (DNA, RNA, siRNA), peptide nucleic acids, aptamers, antibodies or fragments or portions thereof, anesthetic, anticoagulant, anticancer agent, inhibitor of an enzyme, steroidal agent, anti-inflammatory agent, anti-neoplastic agent, antigen, vaccine, antibody, decongestant, antihypertensive, sedative, birth control agent, progestational agent, anticholinergic, analgesic, anti-depressant, anti-psychotic, β-adrenergic blocking agent, diuretic, cardiovascular active agent, vasoactive agent, anti-glaucoma agent, neuroprotectant, angiogenesis inhibitor, hormones, hormone antagonists, growth factors or recombinant growth factors and fragments and variants thereof, cytokines, enzymes, antibiotics or antimicrobial compounds, antifungals, antivirals, toxins, prodrugs, chemotherapeutic agents, small molecules, drugs (e.g., drugs, dyes, amino acids, vitamins, antioxidants), pharmacologic agents, and combinations thereof.

In some embodiments, provided silk fibroin-based materials comprise additives, agents, and/or functional moieties, for example, cells. Cells suitable for use herein include, but are not limited to, progenitor cells or stem cells, smooth muscle cells, skeletal muscle cells, cardiac muscle cells, epithelial cells, endothelial cells, urothelial cells, fibroblasts, myoblasts, chondrocytes, chondroblasts, osteoblasts, osteoclasts, keratinocytes, hepatocytes, bile duct cells, pancreatic islet cells, thyroid, parathyroid, adrenal, hypothalamic, pituitary, ovarian, testicular, salivary gland cells, adipocytes, and precursor cells.

In some embodiments, provided silk fibroin-based materials comprise additives, agents, and/or functional moieties, for example, organisms, such as, a bacterium, fungus, plant or animal, or a virus. In some embodiments, an active agent may include or be selected from neurotransmitters, hormones, intracellular signal transduction agents, pharmaceutically active agents, toxic agents, agricultural chemicals, chemical toxins, biological toxins, microbes, and animal cells such as neurons, liver cells, and immune system cells. The active agents may also include therapeutic compounds, such as pharmacological materials, vitamins, sedatives, hypnotics, prostaglandins and radiopharmaceuticals.

In some embodiments, provided silk fibroin-based materials comprise additives, agents, and/or functional moieties, for example, antibiotics. Antibiotics suitable for incorporation in silk fibroin-based materials include, but are not limited to, aminoglycosides (e.g., neomycin), ansamycins, carbacephem, carbapenems, cephalosporins (e.g., cefazolin, cefaclor, cefditoren, cefditoren, ceftobiprole), glycopeptides (e.g., vancomycin), macrolides (e.g., erythromycin, azithromycin), monobactams, penicillins (e.g., amoxicillin, ampicillin, cloxacillin, dicloxacillin, flucloxacillin), polypeptides (e.g., bacitracin, polymyxin B), quinolones (e.g., ciprofloxacin, enoxacin, gatifloxacin, ofloxacin, etc.), sulfonamides (e.g., sulfasalazine, trimethoprim, trimethoprim-sulfamethoxazole (co-trimoxazole)), tetracyclines (e.g., doxycyline, minocycline, tetracycline, etc.), chloramphenicol, lincomycin, clindamycin, ethambutol, mupirocin, metronidazole, pyrazinamide, thiamphenicol, rifampicin, thiamphenicl, dapsone, clofazimine, quinupristin, metronidazole, linezolid, isoniazid, fosfomycin, fusidic acid, β-lactam antibiotics, rifamycins, novobiocin, fusidate sodium, capreomycin, colistimethate, gramicidin, doxycycline, erythromycin, nalidixic acid, and vancomycin. For example, β-lactam antibiotics can be aziocillin, aztreonam, carbenicillin, cefoperazone, ceftriaxone, cephaloridine, cephalothin, moxalactam, piperacillin, ticarcillin and combination thereof.

In some embodiments, provided silk fibroin-based materials comprise additives, agents, and/or functional moieties, for example, anti-inflammatories. Anti-inflammatory agents may include corticosteroids (e.g., glucocorticoids), cycloplegics, non-steroidal anti-inflammatory drugs (NSAIDs), immune selective anti-inflammatory derivatives (ImSAIDs), and any combination thereof. Exemplary NSAIDs include, but not limited to, celecoxib (Celebrex®); rofecoxib (Vioxx®), etoricoxib (Arcoxia®), meloxicam (Mobic®), valdecoxib, diclofenac (Voltaren®, Cataflam®), etodolac (Lodine®), sulindac (Clinori®), aspirin, alclofenac, fenclofenac, diflunisal (Dolobid®), benorylate, fosfosal, salicylic acid including acetylsalicylic acid, sodium acetylsalicylic acid, calcium acetylsalicylic acid, and sodium salicylate; ibuprofen (Motrin), ketoprofen, carprofen, fenbufen, flurbiprofen, oxaprozin, suprofen, triaprofenic acid, fenoprofen, indoprofen, piroprofen, flufenamic, mefenamic, meclofenamic, niflumic, salsalate, rolmerin, fentiazac, tilomisole, oxyphenbutazone, phenylbutazone, apazone, feprazone, sudoxicam, isoxicam, tenoxicam, piroxicam (Feldene®), indomethacin (Indocin®), nabumetone (Relafen®), naproxen (Naprosyn®), tolmetin, lumiracoxib, parecoxib, licofelone (ML3000), including pharmaceutically acceptable salts, isomers, enantiomers, derivatives, prodrugs, crystal polymorphs, amorphous modifications, co-crystals and combinations thereof.

In some embodiments, provided silk fibroin-based materials comprise additives, agents, and/or functional moieties, for example, antibodies. Suitable antibodies for incorporation in silk fibroin-based materials include, but are not limited to, abciximab, adalimumab, alemtuzumab, basiliximab, bevacizumab, cetuximab, certolizumab pegol, daclizumab, eculizumab, efalizumab, gemtuzumab, ibritumomab tiuxetan, infliximab, muromonab-CD3, natalizumab, ofatumumab omalizumab, palivizumab, panitumumab, ranibizumab, rituximab, tositumomab, trastuzumab, altumomab pentetate, arcitumomab, atlizumab, bectumomab, belimumab, besilesomab, biciromab, canakinumab, capromab pendetide, catumaxomab, denosumab, edrecolomab, efungumab, ertumaxomab, etaracizumab, fanolesomab, fontolizumab, gemtuzumab ozogamicin, golimumab, igovomab, imciromab, labetuzumab, mepolizumab, motavizumab, nimotuzumab, nofetumomab merpentan, oregovomab, pemtumomab, pertuzumab, rovelizumab, ruplizumab, sulesomab, tacatuzumab tetraxetan, tefibazumab, tocilizumab, ustekinumab, visilizumab, votumumab, zalutumumab, and zanolimumab.

In some embodiments, provided silk fibroin-based materials comprise additives, agents, and/or functional moieties, for example, polypeptides (e.g., proteins), including but are not limited to: one or more antigens, cytokines, hormones, chemokines, enzymes, and any combination thereof as an agent and/or functional group. Exemplary enzymes suitable for use herein include, but are not limited to, peroxidase, lipase, amylose, organophosphate dehydrogenase, ligases, restriction endonucleases, ribonucleases, DNA polymerases, glucose oxidase, laccase, and the like.

In some embodiments, provided silk fibroin-based materials comprise additives, agents, and/or functional moieties, for example, particularly useful for wound healing. In some embodiments, agents useful for wound healing include stimulators, enhancers or positive mediators of the wound healing cascade which 1) promote or accelerate the natural wound healing process or 2) reduce effects associated with improper or delayed wound healing, which effects include, for example, adverse inflammation, epithelialization, angiogenesis and matrix deposition, and scarring and fibrosis.

In some embodiments, provided silk fibroin-based materials comprise additives, agents, and/or functional moieties, for example, an optically or electrically active agent, including but not limited to, chromophores; light emitting organic compounds such as luciferin, carotenes; light emitting inorganic compounds, such as chemical dyes; light harvesting compounds such as chlorophyll, bacteriorhodopsin, protorhodopsin, and porphyrins; light capturing complexes such as phycobiliproteins; and related electronically active compounds; and combinations thereof. In some embodiments, inorganic fillers, include for example: β-TCP and biphasic calcium phosphate (BCP).

In some embodiments, provided silk fibroin-based materials comprise additives, agents, and/or functional moieties, for example, particularly useful for chemical modification of silk fibroin-based material. In some embodiments, provided silk fibroin-based materials comprise additives, agents, and/or functional moieties, for example, particularly to improve hydrophilicity, absorbency of water, shape retention, or hydrophobicity, including but not limited to: 4-sulfanilic acid, poly-lysine, 4-(heptyloxy)aniline, 4'-aminoacetophenone, superabsorbent polymers; examples include but are not limited to sodium polyacrylate, crosslinked polyvinyl alcohol (PVA), polyethylene oxide (PEO), polyacrylic acid, hygroscopic materials, cellulose and starch (e.g. modified or unmodified), nylon, polycarbonate, polyethylene glycol, or combinations thereof.

*Nucleic Acids*

In some embodiments, provided silk fibroin-based materials comprise additives, agents, and/or functional moieties, for example, nucleic acid agents. In some embodiments, a silk fibroin-based materials may release nucleic acid agents. In some embodiments, a nucleic acid agent is or comprises a therapeutic agent. In some embodiments, a nucleic acid agent is or comprises a diagnostic agent. In some embodiments, a nucleic acid agent is or comprises a prophylactic agent.

It would be appreciated by those of ordinary skill in the art that a nucleic acid agent can have a length within a broad range. In some embodiments, a nucleic acid agent has a nucleotide sequence of at least about 40, for example at least about 60, at least about 80, at least about 100, at least about 200, at least about 500, at least about 1000, or at least about 3000 nucleotides in length. In some embodiments, a nucleic acid agent has a length from about 6 to about 40 nucleotides. For example, a nucleic acid agent may be from about 12 to about 35 nucleotides in length, from about 12 to about 20 nucleotides in length or from about 18 to about 32 nucleotides in length.

In some embodiments, nucleic acid agents may be or comprise deoxyribonucleic acids (DNA), ribonucleic acids (RNA), peptide nucleic acids (PNA), morpholino nucleic acids, locked nucleic acids (LNA), glycol nucleic acids (GNA), threose nucleic acids (TNA), and/or combinations thereof.

In some embodiments, a nucleic acid has a nucleotide sequence that is or comprises at least one protein-coding element. In some embodiments, a nucleic acid has a nucleotide sequence that is or comprises at least one element that is a complement to a protein-coding sequence. In some embodiments, a nucleic acid has a nucleotide sequence that includes one or more gene expression regulatory elements (e.g., promoter elements, enhancer elements, splice donor sites, splice acceptor sites, transcription termination sequences, translation initiation sequences, translation termination sequences, etc.). In some embodiments, a nucleic acid has a nucleotide sequence that includes an origin of replication. In some embodiments, a nucleic acid has a nucleotide sequence that includes one or more integration sequences. In some embodiments, a nucleic acid has a nucleotide sequence that includes one or more elements that participate in intra- or inter-molecular recombination (e.g., homologous recombination). In some embodiments, a nucleic acid has enzymatic activity. In some embodiments, a nucleic acid hybridizes with a target in a cell, tissue, or organism. In some embodiments, a nucleic acid acts on (e.g., binds with, cleaves, etc.) a target inside a cell. In some embodiments, a nucleic acid is expressed in a cell after release from a provided composition. In some embodiments, a nucleic acid integrates into a genome in a cell after release from a provided composition.

In some embodiments, nucleic acid agents have single-stranded nucleotide sequences. In some embodiments, nucleic acid agents have nucleotide sequences that fold into higher order structures (e.g., double and/or triple-stranded structures). In some embodiments, a nucleic acid agent is or comprises an oligonucleotide. In some embodiments, a nucleic acid agent is or comprises an antisense oligonucleotide. Nucleic acid agents may include a chemical modification at the individual nucleotide level or at the oligonucleotide backbone level, or it may have no modifications.

In some embodiments, of the present disclosure, a nucleic acid agent is an siRNA agent. Short interfering RNA (siRNA) comprises an RNA duplex that is approximately 19 basepairs long and optionally further comprises one or two single-stranded overhangs. An siRNA may be formed from two RNA molecules that hybridize together, or may alternatively be generated from a single RNA molecule that includes a self-hybridizing portion. It is generally preferred that free 5' ends of siRNA molecules have phosphate groups, and free 3' ends have hydroxyl groups. The duplex portion of an siRNA may, but typically does not, contain one or more bulges consisting of one or more unpaired nucleotides. One strand of an siRNA includes a portion that hybridizes with a target transcript. In certain preferred embodiments of the invention, one strand of the siRNA is precisely complementary with a region of the target transcript, meaning that the siRNA hybridizes to the target transcript without a single mismatch. In other embodiments of the invention one or more mismatches between the siRNA and the targeted portion of the target transcript may exist. In most embodiments of the invention in which perfect complementarity is not achieved, it is generally preferred that any mismatches be located at or near the siRNA termini.

Short hairpin RNA refers to an RNA molecule comprising at least two complementary portions hybridized or capable of hybridizing to form a double-stranded (duplex) structure sufficiently long to mediate RNAi (typically at least 19 base pairs in length), and at least one single-stranded portion, typically between approximately 1 and 10 nucleotides in length that forms a loop. The duplex portion may, but typically does not, contain one or more bulges consisting of one or more unpaired nucleotides. As described further below, shRNAs are thought to be processed into siRNAs by the conserved cellular RNAi machinery. Thus shRNAs are precursors of siRNAs and are, in general, similarly capable of inhibiting expression of a target transcript.

In describing siRNAs it will frequently be convenient to refer to sense and antisense strands of the siRNA. In general, the sequence of the duplex portion of the sense strand of the siRNA is substantially identical to the targeted portion of the target transcript, while the antisense strand of the siRNA is substantially complementary to the target transcript in this region as discussed further below. Although shRNAs contain a single RNA molecule that self-hybridizes, it will be appreciated that the resulting duplex structure may be considered to comprise sense and antisense strands or portions. It will therefore be convenient herein to refer to sense and antisense strands, or sense and antisense portions, of an shRNA, where the antisense strand or portion is that segment of the molecule that forms or is capable of forming a duplex and is substantially complementary to the targeted portion of the target transcript, and the sense strand or portion is that segment of the molecule that forms or is capable of forming a duplex and is substantially identical in sequence to the targeted portion of the target transcript.

For purposes of description, the discussion below may refer to siRNA rather than to siRNA or shRNA. However, as will be evident to one of ordinary skill in the art, teachings relevant to the sense and antisense strand of an siRNA are generally applicable to the sense and antisense portions of the stem portion of a corresponding shRNA. Thus in general the considerations below apply also to shRNAs.

An siRNA agent is considered to be targeted to a target transcript for the purposes described herein if 1) the stability of the target transcript is reduced in the presence of the siRNA or shRNA as compared with its absence; and/or 2) the siRNA or shRNA shows at least about 90%, more preferably at least about 91%, 92%, 93%, 94%, 95%, 96%, 97%, 98%, 99%, or 100% precise sequence complementarity with the target transcript for a stretch of at least about 15, more preferably at least about 17, yet more preferably at least about 18 or 19 to about 21-23 nucleotides; and/or 3) one strand of the siRNA or one of the self-complementary portions of the shRNA hybridizes to the target transcript under stringent conditions for hybridization of small (<50 nucleotide) RNA molecules *in vitro* and/or under conditions typically found within the cytoplasm or nucleus of mammalian cells. Since the effect of targeting a transcript is to reduce or inhibit expression of the gene that directs synthesis of the transcript, an siRNA, shRNA, targeted to a transcript is also considered to target the gene that directs synthesis of the transcript even though the gene itself (i.e., genomic DNA) is not thought to interact with the siRNA, shRNA, or components of the cellular silencing machinery. Thus in some embodiments, an siRNA, shRNA, that targets a transcript is understood to target the gene that provides a template for synthesis of the transcript.

In some embodiments, an siRNA agent can inhibit expression of a polypeptide (e.g., a protein). Exemplary polypeptides include, but are not limited to, matrix metallopeptidase 9 (MMP-9), neutral endopeptidase (NEP) and protein tyrosine phosphatase 1B (PTP1B).

*Growth Factor*

In some embodiments, provided silk fibroin-based materials comprise additives, agents, and/or functional moieties, for example, growth factor. In some embodiments, silk fibroin-based materials may release growth factor. In some embodiments, silk fibroin-based materials may release multiple growth factors. In some embodiments, growth factor known in the art include, for example, adrenomedullin, angiopoietin, autocrine motility factor, basophils, brain-derived neurotrophic factor, bone morphogenetic protein, colony-stimulating factors, connective tissue growth factor, endothelial cells, epidermal growth factor, erythropoietin, fibroblast growth factor, fibroblasts, glial cell line-derived neurotrophic factor, granulocyte colony stimulating factor, granulocyte macrophage colony stimulating factor, growth differentiation factor-9, hepatocyte growth factor, hepatoma-derived growth factor, insulin-like growth factor, interleukins, keratinocyte growth factor, keratinocytes, lymphocytes, macrophages, mast cells, myostatin, nerve growth factor, neurotrophins, platelet-derived growth factor, placenta growth factor, osteoblasts, platelets, proinflammatory, stromal cells, T-lymphocytes, thrombopoietin, transforming growth factor alpha, transforming growth factor beta, tumor necrosis factor-alpha, vascular endothelial growth factor and combinations thereof.

In some embodiments, provided silk fibroin-based materials comprise additives, agents, and/or functional moieties, for example, that are particularly useful for healing. Exemplary agents useful as growth factor for defect repair and/or healing can include, but are not limited to, growth factors for defect treatment modalities now known in the art or later-developed; exemplary factors, agents or modalities including natural or synthetic growth factors, cytokines, or modulators thereof to promote bone and/or tissue defect healing. Suitable examples may include, but not limited to 1) topical or dressing and related therapies and debriding agents (such as, for example, Santyl® collagenase) and Iodosorb® (cadexomer iodine); 2) antimicrobial agents, including systemic or topical creams or gels, including, for example, silver-containing agents such as SAGs (silver antimicrobial gels), (CollaGUARD™, Innocoll, Inc) (purified type-I collagen protein based dressing), CollaGUARD Ag (a collagen-based bioactive dressing impregnated with silver for infected wounds or wounds at risk of infection), DermaSIL™ (a collagen-synthetic foam composite dressing for deep and heavily exuding wounds); 3) cell therapy or bioengineered skin, skin substitutes, and skin equivalents, including, for example, Dermograft (3-dimensional matrix cultivation of human fibroblasts that secrete cytokines and growth factors), Apligraf® (human keratinocytes and fibroblasts), Graftskin® (bilayer of epidermal cells and fibroblasts that is histologically similar to normal skin and produces growth factors similar to those produced by normal skin), TransCyte (a Human Fibroblast Derived Temporary Skin Substitute) and Oasis® (an active biomaterial that comprises both growth factors and extracellular matrix components such as collagen, proteoglycans, and glycosaminoglycans); 4) cytokines, growth factors or hormones (both natural and synthetic) introduced to the wound to promote wound healing, including, for example, NGF, NT3, BDGF, integrins, plasmin, semaphoring, blood-derived growth factor, keratinocyte growth factor, tissue growth factor, TGF-alpha, TGF-beta, PDGF (one or more of the three subtypes may be used: AA, AB, and B), PDGF-BB, TGF-beta 3, factors that modulate the relative levels of TGFβ3, TGFβ1, and TGFβ2 (e.g., Mannose-6-phosphate), sex steroids, including for example, estrogen, estradiol, or an oestrogen receptor agonist selected from the group consisting of ethinyloestradiol, dienoestrol, mestranol, oestradiol, oestriol, a conjugated oestrogen, piperazine oestrone sulphate, stilboestrol, fosfesterol tetrasodium, polyestradiol phosphate, tibolone, a phytoestrogen, 17-beta-estradiol; thymic hormones such as Thymosin-beta-4, EGF, HB-EGF, fibroblast growth factors (e.g., FGF1, FGF2, FGF7), keratinocyte growth factor, TNF, interleukins family of inflammatory response modulators such as, for example, IL-10, IL-1, IL-2, IL-6, IL-8, and IL-10 and modulators thereof; INFs (INF-alpha, -beta, and -delta); stimulators of activin or inhibin, and inhibitors of interferon gamma prostaglandin E2 (PGE2) and of mediators of the adenosine 3',5'-cyclic monophosphate (cAMP) pathway; adenosine A1 agonist, adenosine A2 agonist or 5) other agents useful for wound healing, including, for example, both natural or synthetic homologues, agonist and antagonist of VEGF, VEGFA, IGF; IGF-1, proinflammatory cytokines, GM-CSF, and leptins and 6) IGF-1 and KGF cDNA, autologous platelet gel, hypochlorous acid (Sterilox® lipoic acid, nitric oxide synthase3, matrix metalloproteinase 9 (MMP-9), CCT-ETA, alphavbeta6 integrin, growth factor-primed fibroblasts and Decorin, silver containing wound dressings, Xenaderm™, papain wound debriding agents, lactoferrin, substance P, collagen, and silver-ORC, placental alkaline phosphatase or placental growth factor, modulators of hedgehog signaling, modulators of cholesterol synthesis pathway, and APC (Activated Protein C), keratinocyte growth factor, TNF, Thromboxane A2, NGF, BMP bone morphogenetic protein, BMP-2, P24 peptides, CTGF (connective tissue growth factor), wound healing chemokines, decorin, modulators of lactate induced neovascularization, cod liver oil, placental alkaline phosphatase or placental growth factor, and thymosin beta 4. In certain embodiments, one, two three, four, five or six agents useful for wound healing may be used in combination. More details can be found in US Patent No. 8,247,384.

It is to be understood that agents useful for growth factor for healing (including for example, growth factors and cytokines) encompass all naturally occurring polymorphs (for example, polymorphs of the growth factors or cytokines). Also, functional fragments, chimeric proteins comprising one of said agents useful for wound healing or a functional fragment thereof, homologues obtained by analogous substitution of one or more amino acids of the wound healing agent, and species homologues are encompassed. It is contemplated that one or more agents useful for wound healing may be a product of recombinant DNA technology, and one or more agents useful for wound healing may be a product of transgenic technology. For example, platelet derived growth factor may be provided in the form of a recombinant PDGF or a gene therapy vector comprising a coding sequence for PDGF.

*Diagnostic Agents*

In some embodiments, provided silk fibroin-based materials comprise additives, agents, and/or functional moieties, for example, that are particularly useful as diagnostic agents. In some embodiments, diagnostic agents include gases; commercially available imaging agents used in positron emissions tomography (PET), computer assisted tomography (CAT), single photon emission computerized tomography, x-ray, fluoroscopy, and magnetic resonance imaging (MRI); and contrast agents. Examples of suitable materials for use as contrast agents in MRI include gadolinium chelates, as well as iron, magnesium, manganese, copper, and chromium. Examples of materials useful for CAT and x-ray imaging include iodine-based materials.

In some embodiments, provided silk fibroin-based materials comprise additives, agents, and/or functional moieties, for example, radionuclides that are particularly useful as therapeutic and/or diagnostic agents. Among the radionuclides used, gamma-emitters, positron-emitters, and X-ray emitters are suitable for diagnostic and/or therapy, while beta emitters and alpha-emitters may also be used for therapy. Suitable radionuclides for forming thermally-responsive conjugates in accordance with the invention include, but are not limited to, $^{123}$I, $^{125}$I, $^{130}$I, $^{131}$I, $^{133}$I, $^{135}$I, $^{47}$Sc, $^{72}$As, $^{72}$Se, $^{90}$Y, $^{88}$Y, $^{97}$Ru, $^{100}$Pd, $^{101}$mRh, $^{119}$Sb, $^{128}$Ba, $^{197}$Hg, $^{211}$At, $^{212}$Bi, $^{212}$Pb, $^{109}$Pd, $^{111}$In, $^{67}$Ga, $^{68}$Ga, $^{67}$Cu, $^{75}$Br, $^{77}$Br, $^{99}$mTc, $^{14}$C, $^{13}$N, $^{15}$O, $^{32}$P, $^{33}$P, and $^{18}$F. In some embodiments, a diagnostic agent may be a fluorescent, luminescent, or magnetic moiety.

Fluorescent and luminescent moieties include a variety of different organic or inorganic small molecules commonly referred to as "dyes," "labels," or "indicators." Examples include fluorescein, rhodamine, acridine dyes, Alexa dyes, cyanine dyes, *etc.* Fluorescent and luminescent moieties may include a variety of naturally occurring proteins and derivatives thereof, e.g., genetically engineered variants. For example, fluorescent proteins include green fluorescent protein (GFP), enhanced GFP, red, blue, yellow, cyan, and sapphire fluorescent proteins, reef coral fluorescent protein, *etc.* Luminescent proteins include luciferase, aequorin and derivatives thereof. Numerous fluorescent and luminescent dyes and proteins are known in the art (see, *e.g.,* U.S. Patent Application Publication No.: 2004/0067503; Valeur, B., "Molecular Fluorescence: Principles and Applications," John Wiley and Sons, 2002; Handbook of Fluorescent Probes and Research Products, Molecular Probes, 9th edition, 2002; and The Handbook - A Guide to Fluorescent Probes and Labeling Technologies, Invitrogen, 10th edition, available at the Invitrogen web site).

Method of Manufacturing Amphiphilic Polypeptide Materials

The present disclosure provides methods of forming, manufacturing, providing, and/or preparing amphiphilic polypeptide materials. The present invention provides technologies for achieving a solution to solid transition. The present invention provides technologies for achieving a solution to gel to solid transition. As described herein below, it is contemplated that achieving such a transition will result in the formation of an amphiphilic polypeptide material as provided by the present disclosure.

Fabrication of provided amphiphilic polypeptide materials comprises a solution to solid transition.

Fabrication of provided amphiphilic polypeptide materials comprises a solution to gel to solid transition. Methods of manufacturing provided amphiphilic polypeptide materials includes providing an amphiphilic polypeptide solution. Methods include conformally changing an amphiphilic polypeptide solution to an amphiphilic polypeptide gel. Methods include providing an amphiphilic polypeptide gel. Methods include transitioning an amphiphilic polypeptide gel to an amphiphilic polypeptide solid.

In some embodiments, steps of disclosed methods are all aqueous. In some embodiments, steps of disclosed methods do not include non-aqueous solvents.

The present disclosure recognizes that provided amphiphilic polypeptide materials are manufactured from aqueous solutions. In some embodiments, methods of manufacturing provided amphiphilic polypeptide materials do not comprise, use, or require organic solvents and/or non-aqueous solvents in their fabrication. In some embodiments, methods of manufacturing provided amphiphilic polypeptide materials do not comprise, use, or require removing organic solvents and/or non-aqueous solvents from such materials. In some embodiments, all-water based processing does not compromise the biocompatibility of the material.

In some embodiments, the present disclosure recognizes that provided amphiphilic polypeptide materials are manufactured under ambient conditions.

In some embodiments, material and mechanical properties of amphiphilic polypeptide materials formed by provided methods are susceptible to conditions, such as, for example, temperature, pressure, air/gas flow, and /or humidity, as described herein.

Methods comprising an amphiphilic polypeptide solution are or comprise silk fibroin.

*Regeneration of Amphiphilic Polypeptides in a Water Solution*

Provided amphiphilic polypeptide materials for use in the practice of the present disclosure are provided, prepared, and/or manufactured by forming an amphiphilic polypeptide in water solution.

As provided above, the present methods are useful for any amphiphilic polypeptide, for example, as described in detail herein, silk fibroin.

In some embodiments, amphiphilic polypeptide solutions for use in the practice of the present disclosure are provided, prepared, and/or manufactured from an aqueous solution of polypeptide (e.g., silk polymer) where the solvent is water, PBS and combinations thereof. In some embodiments, amphiphilic polypeptide solutions for use in accordance with the present disclosure are provided, prepared, and/or manufactured from an aqueous polypeptide solution in a solvent other than PBS. In some embodiments, amphiphilic polypeptide solutions for use in accordance with the present disclosure are provided, prepared, and/or manufactured from a solution of polypeptide in water. In some embodiments, amphiphilic polypeptide solutions for use in accordance with the present disclosure is provided, prepared, and/or manufactured from a solution of polypeptide in DMEM. In some embodiments, amphiphilic polypeptide solutions for use in accordance with the present disclosure are provided, prepared, and/or manufactured from an aqueous polypeptide solution that is not buffered.

In some embodiments, provided amphiphilic polypeptide materials can be made from amphiphilic polypeptide solution concentrations in a range between about 0.1 % and about 30%.

In some embodiments, high molecular weight silk can be used to form provided amphiphilic polypeptide materials according to the present methods. In some embodiments, low molecular weight silk can be made into provided silk fibroin materials/monoliths using the present methods. For example, in some embodiments, silk fibroin having a molecular weight in a range between about 50 kDa and about 400 kDa can form provided silk fibroin materials/monoliths. In some embodiments, very low molecular weight silk (90 mb) can be made into provided silk fibroin materials/monoliths. In some embodiments, provided silk fibroin materials are comprised of silk fibroin having molecular weights within a range between a lower bound (e.g., about 20 kDa, about 30 kDa, about 40 kDa, about 50 kDa, about 60 kDa, or more) and an upper bound (e.g., about 400 kDa, about 375 kDa, about 350 kDa, about 325 kDa, about 300 kDa, or less). In some embodiments, silk fibroin materials/monoliths provided, prepared, and/or manufactured according to the disclosed methods are or comprise silk fibroin having a molecular weight around 60 kDa.

In some embodiments, providing a silk fibroin solutions having high molecular weight silk fibroin fragments will produce a silk fibroin polypeptide material/monoliths that is both more crystalline and more brittle.

In some embodiments, providing a silk fibroin solution having low molecular weight amphiphilic polypeptide fragments will produce a silk fibroin material that is both less crystalline and less brittle.

In some embodiments, boiling and degumming an amphiphilic polypeptide, such as silk fibroin, is in a solution for example of in $Na_2CO_3$.

In some embodiments, provided amphiphilic polypeptide materials of the present disclosure, such as a silk, are produced from an amphiphilic polypeptide by degumming silk cocoons in an aqueous solution at temperatures of: about 30 °C, about 35 °C, about 40 °C, about 45 °C, about 50 °C, about 55 °C, about 60°C, about 65 °C, about 70 °C, about 75 °C, about 80 °C, about 85 °C, about 90 °C, about 95 °C, about 100 °C, about 105 °C, about 110 °C, about 115 °C, about 120 °C, about 125 °C, about 130 °C, about 135 °C, about 140 °C, about 145 °C, or about at least 150 °C. In some embodiments, methods involve extraction of polypeptides (such as silk fibroin) under high temperature, such as between about 101 °C and about 135 °C, between about 105 °C and about 130 °C, between about 110 °C and about 130 °C, between about 115 °C and about 125 °C, between about 118 °C and about 123 °C, e.g., about 115 °C, 116 °C, 117°C, 118 °C, 119°C, 120 °C, 121 °C, 122 °C, 123 °C, 124 °C, 125 °C. In some embodiments, boiling is performed at a temperature below about 65 °C. In some embodiments, boiling is performed at a temperature of about 60 °C or less.

Additionally or alternatively, in some embodiments, provided methods involve extraction of amphiphilic polypeptides (such as silk fibroin) under elevated pressure, such as about 34.5 kPa, 41.4 kPa, 48.3 kPa, 55.2 kPa, 61.2 kPa, 69.0 kPa, 75.8 kPa, 82.7 kPa, 89.6 kPa, 96.5 kPa, 103.4 kPa, 110.3 kPa, 117.2 kPa, 124.1 kPa, 131.0 kPa, 137.9 kPa, 144.8 kPa, 151.7 kPa, 158.6 kPa, 165.5 kPa, 172.4 kPa, 179.3 kPa, 186.2 kPa, 193.1 kPa, 200.0 kPa, 206.9 kPa, 213.7 kPa, 220.6 kPa, 227.5 kPa, 234.4 kPa, and 241.3 kPa(5 psi, 6 psi, 7 psi, 8 psi, 9 psi, 10 psi, 11 psi, 12 psi, 13 psi, 14 psi, 15 psi, 16 psi, 17 psi, 18 psi, 19 psi, 20 psi, 21 psi, 22 psi, 23 psi, 24 psi, 25 psi, 30 psi, 31 psi, 32 psi, 33 psi, 34 psi and 35 psi). In some embodiments, amphiphilic polypeptides (such as silk fibroin) are extracted under high temperature and under elevated pressure, e.g., at about 110 and about 130 °C and about 69 kPa (10 psi) and about 137.9 kPa (20 psi) for a duration suitable to produce an amphiphilic polypeptide solution that would easily go through a 0.2 $\mu$m filter. In some embodiments, amphiphilic polypeptides (such as silk fibroin) are extracted under high temperature and under elevated pressure, e.g., at about 110 °C and about 130 °C and about 10 to about 20 psi for about 60 to about 180 minutes. In some embodiments, amphiphilic polypeptides (such as silk fibroin) are extracted under high temperature and under elevated pressure.

In some embodiments, amphiphilic polypeptide fragments for use with the present disclosure are produced having a molecular weight inversely related to a length of boiling time. In some embodiments, amphiphilic polypeptide materials for use in accordance with the present disclosure are provided, prepared, and/or manufactured from a polypeptide solution (e.g. silk fibroin) that has been boiled for at least about 5, 10, 15, 20, 25, 30, 35, 40, 45, 50, 55, 60, 70, 80, 90, 100, 110, 120, 150, 180, 210, 240, 270, 310, 340, 370, 410 minutes or more. In some embodiments, amphiphilic polypeptides, such as, silk fibroin fragments can be derived by degumming silk cocoons at or close to (e.g., within 5% around) an atmospheric boiling temperature for at least about: 1 minute of boiling, 2 minutes of boiling, 3 minutes of boiling, 4 minutes of boiling, 5 minutes of boiling, 6 minutes of boiling, 7 minutes of boiling, 8 minutes of boiling, 9 minutes of boiling, 10 minutes of boiling, 11 minutes of boiling, 12 minutes of boiling, 13 minutes of boiling, 14 minutes of boiling, 15 minutes of boiling, 16 minutes of boiling, 17 minutes of boiling, 18 minutes of boiling, 19 minutes of boiling, 20 minutes of boiling, 25 minutes of boiling, 30 minutes of boiling, 35 minutes of boiling, 40 minutes of boiling, 45 minutes of boiling, 50 minutes of boiling, 55 minutes of boiling, 60 minutes or longer, including, e.g., at least 70 minutes, at least 80 minutes, at least 90 minutes, at least 100 minutes, at least 110 minutes, at least about 120 minutes or longer. As used herein, the term "atmospheric boiling temperature" refers to a temperature at which a liquid boils under atmospheric pressure.

In some embodiments, provided materials are prepared and/or manufactured from silk fibers were solubilized in LiBr and then dialyzed against water.

In some embodiments, amphiphilic polypeptides (e.g. silk fibroin) for use in accordance with the present disclosure are provided, prepared, and/or manufactured from a silk solution adjusted and/or maintained at a sub-physiological pH. For example, in some embodiments, amphiphilic polypeptides for use in accordance with the present disclosure are provided, prepared, and/or manufactured from a solution of an amphiphilic polypeptide that is adjusted to and/or maintained at a pH near or below about 6. In some embodiments, amphiphilic polypeptides are provided, prepared, and/or manufactured from a solution of an amphiphilic polypeptide with a pH for instance about 6 or less, about 5 or less, about 4 or less, about 3 or less, about 2 or less, about 1.5 or less, or about 1 or less. However, in some alternative embodiments, amphiphilic polypeptides are provided, prepared, and/or manufactured from a solution of an amphiphilic polypeptide with a pH in a range for example of at least 6, at least 7, at least 8, at least 9, and at least about 10.

In some embodiments, provided amphiphilic polypeptide materials for use in accordance with the present

EP 3 328 438 B1

disclosure are provided, prepared, and/or manufactured from a amphiphilic polypeptide solution, such as a silk fibroin solution of about 0.5 wt% polypeptide to about 30 wt% polypeptide. In some embodiments, provided amphiphilic polypeptide materials for use in accordance with the present disclosure are provided, prepared, and/or manufactured from an amphiphilic polypeptide solution, such as a silk fibroin solution that is less than about 30 wt% polypeptide. In some embodiments, provided amphiphilic polypeptide materials for use in accordance with the present disclosure are provided, prepared, and/or manufactured from a polypeptide solution, such as a silk fibroin solution that is less than about 20 wt% polypeptide. In some embodiments, provided amphiphilic polypeptide materials for use in accordance with the present invention are provided, prepared, and/or manufactured from a amphiphilic polypeptide solution, such as a silk fibroin solution that is less than about 10 wt% amphiphilic polypeptide. In some embodiments, provided amphiphilic polypeptide materials for use in accordance with the present invention are provided, prepared, and/or manufactured from a amphiphilic polypeptide solution, such as a silk fibroin solution that is less than about 10 wt% amphiphilic polypeptide, or even that is about 5% wt%, about 4 wt%, about 3 wt%, about 2 wt%, about 1 wt% amphiphilic polypeptide or less.

In some embodiments, provided amphiphilic polypeptide materials, such as silk fibroin-based materials, are modulated by controlling a silk concentration. In some embodiments, a weight percentage of silk fibroin can be present in the solution at any concentration suited to the need. In some embodiments, a silk fibroin solution can have silk fibroin at a concentration of about 0.1 mg/mL to about 50 mg/mL. In some embodiments, a silk fibroin solution can comprise silk fibroin at a concentration of about less than 1 mg/mL, about less than 1.5 mg/mL, about less than 2 mg/mL, about less than 2.5 mg/mL, about less than 3 mg/mL, about less than 3.5 mg/mL, about less than 4 mg/mL, about less than 4.5 mg/mL, about less than 5 mg/mL, about less than 5.5 mg/mL, about less than 6 mg/mL, about less than 6.5 mg/mL, about less than 7 mg/mL, about less than 7.5 mg/mL, about less than 8 mg/mL, about less than 8.5 mg/mL, about less than 9 mg/mL, about less than 9.5 mg/mL, about less than 10 mg/mL, about less than 11 mg/mL, about less than 12 mg/mL, about less than 13 mg/mL, about less than 14 mg/mL, about less than 15 mg/mL, about less than 16 mg/mL, about less than 17 mg/mL, about less than 18 mg/mL, about less than 19 mg/mL, about less than 20 mg/mL, about less than 25 mg/mL, about less than 30 mg/mL, about less than 35 mg/mL, about less than 40 mg/mL, about less than 45 mg/mL, or about less than 50 mg/mL.

Among naturally derived amphiphilic polypeptides, silk fibroin protein has been studied because of its excellent mechanical properties, biocompatibility, controllable degradation rates, and inducible formation of crystalline β-sheet structure networks. (See for example Altman et al., 24 Biomats. 401-16 (2003); Jin & Kaplan, 424 Nature 1057-61 (2003); Horan et al., 26 Biomats. 3385-93 (2005); Kim et al., 26 Biomats. 2775-85 (2005); Ishida et al., 23 Macromolecules 88-94 (1990); Nazarov et al., 5 Biomacromolecules 718-26 (2004)). Silk fibroin has been fabricated into various material formats including films, three dimensional porous scaffolds, electrospun fibers and microspheres for both tissue engineering and controlled drug release applications (Jin et al., 5 Biomacromolecules 711-7 (2004); Jin et al., 3 Biomacromolecules, 1233-39 (2002); Hino et al., 266 J. Colloid Interface Sci. 68-73 (2003); Wang et al., 117 J. Control Release, 360-70 (2007); see also U.S. patent application Ser. No. 11/020,650; No. 10/541,182; No. 11/407,373; and No. 11/664,234; PCT/US07/020,789; PCT/US08/55072).

*Sol-gel Transition*

An amphiphilic polypeptide material is formed by a solution to gel to solid transition.

In some embodiments, an amphiphilic polypeptide gel may be formed by any methods already reported in the literature.

In some embodiments, gelation occurs when polymer chains crosslink either chemically or physically into networks, triggered by chemical reagents (e.g., cross-linkers) or physical stimulants (e.g., pH and/or temperature).

Generally, gelation of naturally derived polymers is less controllable, although they tend to be useful as carriers of cell and bioactive molecules for tissue engineering and implantable medical devices because their macromolecular properties are more closely aligned to the extracellular matrix and the degradation products are nontoxic. (See Lee et al., 221 Int'l J. Pharma. 1-22 (2001); Smidsrød et al., 8 Trends Biotech. 71-78 (1990)).

*In vitro,* purified silk fibroin aqueous solutions undergo self-assembly into β-sheet structures and form hydrogels. This sol-gel transition is influenced by temperature, pH, and ionic strength. (See Wang et al., 36 Int'l J. Biol. Macromol. 66-70 (2005); Kim et al., 5 Biomacromolecules 786-92 (2004); Matsumoto et al., 110 J. Phys. Chem. B 21630-38 (2006),).

In some embodiments, an amphiphilic polypeptide sol-gel transition is achieved with solutions having a concentration between about 0.5 wt% and about 30 wt% through: electrogelation of a solution, decrease in solution pH, sonication of a solution, vortexing of a solution, exposure to polar solvent (e.g. acetone, ethanol, methanol), self-gelation at various temperatures (2 < T < 90 °C). Alternatively, in some embodiments, an aquagel may form by concentrating a solution in water at concentration > 20 wt%.

In some embodiments, applying an electric field to an amphiphilic polypeptide solution converts a solution into a gel. In some embodiments, when an amphiphilic polypeptide is silk, forming a silk fibroin gel occurs with direct application of voltage to a silk fibroin solution.

In some embodiments, an amphiphilic polypeptide gel may be formed by electrogelation of an amphiphilic polypeptide solution. Electrogelling is known in the art, (see for example, WO 2010/036992 A2 Bernhard et al., Active Silk Muco-Adhesives, Silk Electrogelation Process, and Devices, published April 1, 2010).

In some embodiments, applying an electric field to a silk fibroin solution may be performed, for example, by submerging electrodes in a silk fibroin solution and applying a constant voltage across the electrodes. In some embodiments, an electric field created by voltage causes a conformational change of silk fibroin from random coil to the silk I formation. In some embodiments, an increase in the meta- stable silk I conformation of silk fibroin is achieved with electrogelation. Additionally, in some embodiments, silk I structures exhibited in gel form can be transformed back to liquid form (random coil form); or through minimal additional molecular alignment, can be converted to β-sheet structures.

In some embodiments, other methods of applying an electric field to the silk solution may also be used, such as current sources, antennas, lasers, and other generators.

In some embodiments, vortexing as used in the present disclosure includes applying a shear-gradient to an amphiphilic polypeptide solution to induce sol-gel transition.

In some embodiments, an amphiphilic polypeptide gel may be formed by vortexing an amphiphilic polypeptide solution. Vortexing is known in the art (see for example, WO 2011/005381 A2 Kaplan et al., Vortex-Induced Silk Fibroin Gelation for Encapsulation and Delivery, published January 13, 2011).

In some embodiments, when an amphiphilic polypeptide is silk, forming a silk fibroin gel occurs when a vortex treatment is applied to a silk fibroin solution. In some embodiments, methods comprise exposing a silk fibroin solution to a treatment comprising vortexing for a sufficient period of time to initiate gelation. In some embodiments, vortexing of a silk fibroin solution is for a time sufficient to initiate intermolecular self-assembly of silk fibroin β-sheet structure. In some embodiments, a vortexing technique induces change in silk fibroin structure and solution viscoelastic properties to cause shear gradient and control the post-vortex self-assembly and kinetics of silk fibroin.

In some embodiments, sonication of an amphiphilic polypeptide solution induces a sol-gel transition.

In some embodiments, an amphiphilic polypeptide gel may be formed by sonicating an amphiphilic polypeptide solution. Sonication is known in the art, (see for example, WO 2008/150861 A1 Wang et al., Method for Silk Fibroin Gelation Using Sonication, published December 11, 2008).

Generally, in sonication, mechanical vibration causes the formation and collapse of bubbles in a solution, known as cavitation. As a result of this cavitation, solutions may experience extreme local effects: heating (10,000 K), high pressure (200 bar) and high strain rates ($10^7$ s$^{-1}$). (See Paulusse & Sijbesma, 44 J. Polym. Sci.-Polym. Chem. 5445-53 (2006); Kemmere et al., 290 Macromol. Mater. Eng. 302-10 (2005)). Mechanistically, in some embodiments, sonication induces physical β-sheet crosslinking via alteration in hydrophobic hydration of the fibroin protein chains.

In some embodiments, when an amphiphilic polypeptide is silk, forming a silk, unique hydrophobic block sequences featured in silk fibroin chains are particularly suitable for this type of technique due to the critical role of water in the control of intra- and inter-chain interactions. (See for example Jin & Kaplan, 424 Nature 1057-61 (2003)).

In some embodiments, when an amphiphilic polypeptide is silk, gelation occurs when silk fibroin is exposed to a treatment comprising ultrasonication for a sufficient period of time to initiate gelation. In some embodiments, ultrasonication rapidly forms a silk fibroin gel and substantial gelation usually occurs within twenty-four hours after the ultrasonication treatment. While not wishing to be bound by any theory it is likely that several physical factors related to sonication, including local temperature increases, mechanical/shear forces, and increased air-liquid interfaces affect the process of rapid gelation of silk fibroin.

In some embodiments, methods further provide for manipulation of the pH and salt concentration effects on gelation.

In some embodiments, an amphiphilic polypeptide gel may be formed by changing a pH of an amphiphilic polypeptide solution. Such a technique is known in the art, (see for example, WO 2012/087823 A2 Kaplan et al., PH Induced Silk Fibroin Gels and Uses Thereof, published June 28, 2012).

In some embodiments, methods include preparing an amphiphilic polypeptide gel by reducing a pH level of an amphiphilic polypeptide solution.

In some embodiments, when an amphiphilic polypeptide is silk, forming a silk fibroin gel occurs by induced by reducing pH. In some embodiments, reducing pH is due to direction pH titration. In some embodiments, reducing pH is due to indirect pH change. In some embodiments, reducing pH results in an increased proton concentration of silk fibroin solution, either locally or in bulk solution. In some embodiments, a silk gel is formed by reducing the local pH or bulk pH of a silk fibroin solution to an acidic pH. In some embodiments, a step of reducing pH of a silk fibroin solution comprises titrating a silk fibroin solution with an acid to an acidic pH.

In some embodiments, an amphiphilic polypeptide gel may be formed by exposing an amphiphilic polypeptide solution to a polar solvent (e.g. acetone or ethanol). Such a technique is known in the art, (see for example, WO 2010/042798 A2 Kaplan et al., Modified Silk Films Containing Glycerol, published April 15, 2010). In some embodiments, methods include preparing an amphiphilic polypeptide gel by immersion in a polar solvent. In some embodiments, a silk II structure can be obtained by solvent treatment, such as with acetone, methanol, and/or ethanol.

In some embodiments, for example, many cell-based applications, gelation must be induced under mild conditions in a relatively short period of time (within hours). Silk gelation time may be prohibitively long, however, unless non-physiological treatments are considered (such as low pH, high temperature, additives) in the absence of chemical modifications to the native silk fibroin protein. For silk fibroin concentrations from 0.6% to 15% (w/v), days to weeks were required for the sol-gel transition at room temperature or 37° C. (See for example Kim et al., 5 Biomacromolecules 786-92 (2004); Matsumoto et al., 110 J. Phys. Chem. B 21630-38 (2006)). Adding salts at concentrations above physiological levels does not significantly alter the gelation kinetics (Kim et al., 2004),. Lowering pH (pH<5) or increasing temperature (>60° C) could reduce the gelation time to a few hours (See Kim et al., 5 Biomacromolecules 786-92 (2004); Motta et al., 15 J. Biomater. Sci. Polymer. Edu. 851-64 (2004)), but these conditions could potentially alter cell function and affect cell viability. In some embodiments, a sol-gel transition may be achieved at any temperature. In some embodiments, higher temperatures results in a shorter transition time. In some embodiments, lower temperatures are favorable for stability of additives, agents, and/or functional moieties that are encapsulated, encompassed, included, incorporated, and/or stored.

In some embodiments, provided methods further include encapsulating, encompassing, including, incorporating, and/or storing at least one additives, agents, and/or functional moieties in a provided amphiphilic polypeptide material.

In some embodiments, at least one additives, agents, and/or functional moieties is encapsulated, encompassed, included, incorporated, and/or stored in an amphiphilic polypeptide solution. In some embodiments, at least one additives, agents, and/or functional moieties is encapsulated, encompassed, included, incorporated, and/or stored prior to a sol-gel transition. In some embodiments, at least one additives, agents, and/or functional moieties is encapsulated, encompassed, included, incorporated, and/or stored during a sol-gel transition. In some embodiments, at least one additives, agents, and/or functional moieties is encapsulated, encompassed, included, incorporated, and/or stored before substantial gelation occurs in the silk fibroin solution. In some embodiments, at least one additives, agents, and/or functional moieties is encapsulated, encompassed, included, incorporated, and/or stored after substantial gelation has occurred in the silk fibroin solution. In some embodiments, at least one additives, agents, and/or functional moieties is encapsulated, encompassed, included, incorporated, and/or stored after a sol-gel transition. In some embodiments, at least one additives, agents, and/or functional moieties is encapsulated, encompassed, included, incorporated, and/or stored in an amphiphilic polypeptide gel.

Indeed, for example, human bone marrow derived mesenchymal stem cells (hMSCs) have been encapsulated in silk gels. hMSCs have been successfully encapsulated in a variety of hydrogel systems, such as polyethylene glycol, agarose, collagen and alginate, because of the potential of these cells for tissue repair or regeneration and long-term drug release. (See for example Nuttelman et al., 24 Matrix Biol. 208-18 (2005); Nuttelman et al., 27 Biomats. 1377-86 (2006); Mauck et al., 14 Osteoarthr. Cartilage 179-89 (2006); Lewus & Nauman, 11 Tissue Eng. 1015-22 (2005); Majumdar et al., 185 J. Cell Physiol. 98-106 (2000); Boison, 27 Trends Pharmacol. Sci. 652-58 (2006)).

In some embodiments, a gel is injected, inserted, placed, poured, pressed, and/or transferred to a mold and/or a substrate.

In some embodiments, a mold and/or a substrate maintains and/or retains a defined shape for an amphiphilic polypeptide gel.

In some embodiments, crystallinity of provided amphiphilic polypeptide materials is tailored. In some embodiments, crystallinity of provided amphiphilic polypeptide materials is tailored *a priori,* during gel fabrication by regulating sol-gel transition. In some embodiments, crystallinity is tailored using methods known to those of skill, for example, heat, treatment with polar solvents, etc.

In some embodiments, anisotropy is induced in provided amphiphilic polypeptide materials. In some embodiments, anisotropy is induced during a sol-gel transition. In some embodiments, anisotropy is induced through application of electric fields.

*Solid Formation from a Gel*

Provided amphiphilic polypeptide materials are formed when an amphiphilic polypeptide gel transitions to a solid.

In some embodiments, spontaneous self-assembly of molecules of an amphiphilic polypeptide occurs in amphiphilic polypeptide gels and/or gels including a blend of different amphiphilic polypeptides.

In some embodiments, a gel-solid transition is achieved by fusing gel particles into polypeptide materials.

In some embodiments, fusing gel particles includes removing free water from a gel.

In some embodiments, fusing gel particles includes removing at least some freezing-bound water. Methods comprise removing freezing-bound water is at a temperature of at least 0 °C.

In some embodiments, removing free water from a gel through controlled evaporation techniques. In some embodiments, a gel-solid transition is achieved by dehydrating an amphiphilic polypeptide gel. In some embodiments, dehydrating is accomplished by any means known to reduce moisture.

In some embodiments, dehydrating an amphiphilic polypeptide results in a self-assembling of a solid amphiphilic polypeptide material. In some embodiments, self-assembling occurs by a combination of hydrogen-bonding between beta-sheet forming amino acids and hydrophobic collapse of the tails to yield micelles.

In some embodiments, a gel-solid transition is achieved by dehydrating an amphiphilic polypeptide through formation of a liquid-liquid interface, where an amphiphilic polypeptide material forms by quasi-static removing of solvent by diffusion in a coagulation bath.

In some embodiments, dehydrating and forming a solid from a gel occurs at ambient conditions. In some embodiments, dehydrating requires time to remove water.

In some embodiments, time dehydrating an amphiphilic polypeptide gel depends on its size and geometry. In some embodiments, larger gels results in longer dehydrating time. In some embodiments, gels having a geometry with low exposed surface area results in longer dehydrating time.

In some embodiments, a time dehydrating an amphiphilic polypeptide gel depends on concentration. In some embodiments, for example, low concentration and higher solvent content results in longer dehydrating times.

In some embodiments, dehydrating may be accelerated by changing conditions, such as, for example, temperature, pressure, humidity, adding a flow of forced gas, increasing a flow-rate of a forced gas, etc. In some embodiments, gel-solid transition conditions are held constant, for example, a temperature is set and held constant through a gel-solid transition. In some embodiments, gel-solid transition conditions vary either randomly or according to a set ramp rate.

In some embodiments, a gel-solid transition is achieved by dehydrating an amphiphilic polypeptide gel at a temperature between about 0 °C and about 90 °C. In some embodiments, a gel-solid transition is faster at a higher temperature. In some embodiments, a gel-solid transition is achieved by dehydrating an amphiphilic polypeptide gel at room temperature. In some embodiments, dehydrating temperature is about 5 °C, about 10 °C, about 15 °C, about 20 °C, about 25 °C, about 30 °C, about 35 °C, about 40 °C, about 45 °C, about 50 °C, about 55 °C, about 60 °C, about 65 °C, about 70 °C, about 75 °C, about 80 °C, about 85 °C, about 90 °C or higher. In some embodiments, when encapsulating, encompassing, including, incorporating, and/or storing at least one additives, agents, and/or functional moieties that are heat-labile molecules, a temperature is about room temperature.

In some embodiments, a gel-solid transition is achieved by dehydrating an amphiphilic polypeptide gel at atmospheric pressure. In some embodiments, a gel-solid transition is achieved by dehydrating an amphiphilic polypeptide gel at a pressure above atmospheric pressure. In some embodiments, a gel-solid transition is faster at higher pressure.

In some embodiments, a gel-solid transition is achieved by dehydrating an amphiphilic polypeptide gel at a relative humidity between about 0 % and about 70%. In some embodiments, a gel-solid transition is faster at a lower humidity.

In some embodiments, a gel-solid transition is achieved by dehydrating an amphiphilic polypeptide gel by exposing an amphiphilic polypeptide gel a forced flow of a gas.

In some embodiments, air is or comprises compressed dried air. In some embodiments, a gas is or comprises nitrogen, argon, carbon dioxide, compressed dried air, oxygen, neon, helium, among others known in the art.

In some embodiments, a gel-solid transition is achieved by dehydrating an amphiphilic polypeptide through sealing an amphiphilic polypeptide gel in a desiccator with a desiccant. In some embodiments, a desiccant is a hygroscopic substance that induces or sustains a dry state within its vicinity.

In some embodiments, a desiccant is or comprises alumina, aluminum amalgam, barium oxide, barium perchlorate, boric anhydride, calcium chloride (anhydrous), calcium oxide, calcium sulphate (anhydrous), copper (II) sulphate (anhydrous), magnesium amalgam, magnesium perchlorate (anhydrous), magnesium sulphate (anhydrous), phosphorus pentoxide, potassium (metal), potassium carbonate (anhydrous), silica gel, sodium (metal), sodium hydroxide, sodium potassium alloy, sodium sulfate (anhydrous), sulfuric acid (concentrated).

*Solid Formation from a Solution*

In nature, silk fibroin aqueous solutions are produced for example in the posterior section of silkworm gland and then stored in the middle section at a concentration up to 30% (w/v) and contains a high content of random coil or alpha helical structure. During fiber spinning into air, high shear force and elongational flow induces self-assembly and a structural transition to the $\beta$-sheet structure, leading to the formation of solid fibers. (See for example Vollrath & Knight, 410 Nature, 541-48 (2001)). The presence of metallic ions and pH changes in different sections of the gland influence this transition. (See for example Chen et al., 3 Biomacromolecules 644-8 (2002); Zhou et al., 109 J. Phys. Chem. B 16937-45 (2005); Dicko et al., 5 Biomacromolecules 704-10 (2004); Terry et al., 5 Biomacromolecules 768-72 (2004)).

The outstanding mechanical properties of natural silk fibers derive from their unique structure and spinning process. In some embodiments, a biomimetic, all-aqueous process is used to obtain amphiphilic polypeptide materials. In some embodiments, amphiphilic polypeptide materials generated by provided methods replicate the nano-scale structure of natural silk fibers.

Provided amphiphilic polypeptide materials are formed when an amphiphilic polypeptide solution transitions to a solid.

In some embodiments, by mimicking the native spinning process used by the silkworm, an all-green, aqueous process to generate amphiphilic materials, such as compact crystalline silk block materials.

In some embodiments, spontaneous self-assembly of molecules of an amphiphilic polypeptide, which may include a blend of different amphiphilic polypeptides.

In some embodiments, a solution to solid transition is achieved by a slow dehydration process. In some embodiments, dehydrating and forming a solid from a solution occurs at ambient conditions. In some embodiments, dehydrating requires time to remove water. In some embodiments, methods include dehydrating at a temperature of at least 10 °C.

In some embodiments, for example, a silk solution pH is controlled at 8.0 to prevent hydrogel formation during a dehydration process.

In some embodiments, removing free water from a gel through controlled evaporation techniques. In some embodiments, a solution to solid transition is achieved by dehydrating an amphiphilic polypeptide solution. In some embodiments, dehydrating is accomplished by any means known to reduce moisture.

In some embodiments, as moisture is drawn from an amphiphilic polypeptide solution, amphiphilic polypeptide globules form. In some embodiments, amphiphilic polypeptide globules have at least one dimension between about 5 nm and about 30 nm. In some embodiments, when amphiphilic polypeptide globules form interactions with one another so that they aggregate.

In some embodiments, time dehydrating an amphiphilic polypeptide solution depends on its volume. In some embodiments, larger volumes or lower exposed surface area results in longer dehydrating times. In some embodiments, a time dehydrating an amphiphilic polypeptide gel depends on concentration. In some embodiments, for example, low concentration and higher solvent content results in longer dehydrating times.

In some embodiments, dehydrating may be accelerated by changing conditions, such as, for example, temperature, pressure, humidity, adding a flow of forced gas, increasing a flow-rate of a forced gas, etc. In some embodiments, solution to solid conditions are held constant, for example, a temperature is set and held constant through a solution to solid transition. In some embodiments, solution to solid transition conditions vary either randomly or according to a set ramp rate.

In some embodiments, a solution to solid transition is achieved by dehydrating an amphiphilic polypeptide gel at a temperature between about 0 °C and about 90 °C. In some embodiments, a solution to solid transition is faster at a higher temperature. In some embodiments, a solution to solid transition is achieved by dehydrating an amphiphilic polypeptide gel at room temperature. In some embodiments, dehydrating temperature is about 5 °C, about 10 °C, about 15 °C, about 20 °C, about 25 °C, about 30 °C, about 35 °C, about 40 °C, about 45 °C, about 50 °C, about 55 °C, about 60 °C, about 65 °C, about 70 °C, about 75 °C, about 80 °C, about 85 °C, about 90 °C or higher. In some embodiments, when encapsulating, encompassing, including, incorporating, and/or storing at least one additives, agents, and/or functional moieties that are heat-labile molecules, a temperature is about room temperature.

In some embodiments, a gel-solid transition is achieved by dehydrating an amphiphilic polypeptide gel at atmospheric pressure. In some embodiments, a gel-solid transition is achieved by dehydrating an amphiphilic polypeptide gel at a pressure above atmospheric pressure. In some embodiments, a gel-solid transition is faster at higher pressure.

In some embodiments, a gel-solid transition is achieved by dehydrating an amphiphilic polypeptide gel at a relative humidity between about 0 % and about 70 %. In some embodiments, a gel-solid transition is faster at a lower humidity.

In some embodiments, a solution to solid transition is achieved by dehydrating an amphiphilic polypeptide gel by exposing an amphiphilic polypeptide gel a forced flow of a gas.

In some embodiments, air is or comprises compressed dried air. In some embodiments, a gas is or comprises nitrogen, argon, carbon dioxide, compressed dried air, oxygen, neon, helium, among others known in the art.

In some embodiments, a solution to solid transition is achieved by dehydrating an amphiphilic polypeptide through sealing an amphiphilic polypeptide gel in a desiccator with a desiccant. In some embodiments, a desiccant is a hygroscopic substance that induces or sustains a dry state within its vicinity.

In some embodiments, a desiccant is or comprises alumina, aluminum amalgam, barium oxide, barium perchlorate, boric anhydride, calcium chloride (anhydrous), calcium oxide, calcium sulphate (anhydrous), copper (II) sulphate (anhydrous), magnesium amalgam, magnesium perchlorate (anhydrous), magnesium sulphate (anhydrous), phosphorus pentoxide, potassium (metal), potassium carbonate (anhydrous), silica gel, sodium (metal), sodium hydroxide, sodium potassium alloy, sodium sulfate (anhydrous), sulfuric acid (concentrated).

<u>Materials</u>

*Additives Introduced to Affect Porosity and Degradation Profile*

In some embodiments, a porosity of provided amphiphilic polypeptide materials may be tailored. In some embodiments, processing of provided amphiphilic polypeptide materials comprises a step of incorporating porogens at the point of material fabrication. Porosity can be controlled in the same way it can be controlled for silk gels, by incorporating slow-degrading porogens or porogens that are soluble in polar solvents.

In some embodiments, a degradation profile of an amphiphilic polypeptide material is similar and/or predictable based on a degradation profile of other materials and based on a degradation profile of other material formats. In some embodiments, lipid vesicle-loaded proteases (the release of which can be triggered or regulated by temperature) will affect a degradation profile. In some embodiments, vesicles are incorporated in amphiphilic polypeptide materials at the point of solution formation. In some embodiments, modulation of a molecular weight of amphiphilic polypeptide fragments may also result in a controlled degradation profile.

*Machinability*

In some embodiments, provided amphiphilic polypeptide materials are machinable. In some embodiments, processing of provided amphiphilic polypeptide materials comprises a step of machining.

In some embodiments, provided amphiphilic polypeptide materials are characterized in that machining reduces their volume of relative to its initial volume. In some embodiments, machining reduces a volume provided amphiphilic polypeptide materials to form a structure having a definite shape. For example, in some embodiments, provided amphiphilic polypeptide materials are machined on a lathe. In some embodiments, when worked on via a lathe, a lathe rotates such a material on its axis while performing operations selected from the group consisting of cutting, sanding, knurling, drilling, deformation, facing, or turning, to create an object which has symmetry about an axis of rotation.

In some embodiments, processing of provided amphiphilic polypeptide materials comprises a step of manipulating by use of hand tools rather than a machine tool.

In some embodiments, methods include steps that alter an amphiphilic polypeptide material and/or a surface of an amphiphilic polypeptide material after the material is formed.

In some embodiments, crystallinity of provided amphiphilic polypeptide materials is tailored *a posteriori,* after solid formation with post-processing techniques known to those skilled in the art. In some embodiments, *a posteriori* techniques include for example heat, immersion in polar solvent treatment, etc.

In some embodiments, anisotropy is used to tailor properties and/or mechanics of provided amphiphilic polypeptide materials. In some embodiments, anisotropy is induced using post-processing techniques. In some embodiments, provided methods include inducing anisotropy in provided amphiphilic polypeptide materials at a material drying stage (i.e. a solution to solid transition). In some embodiments, inducing anisotropy in provided amphiphilic polypeptide materials occurs through semi-confinement of a gel in non-water permeable environments, which results in preferential water evaporation.

In some embodiments, provided amphiphilic polypeptide materials are embossed. In some embodiments, processing of provided amphiphilic polypeptide materials comprises a step of embossing. In some embodiments, provided amphiphilic polypeptide materials are nanoimprinted. In some embodiments, processing of provided amphiphilic polypeptide materials comprises a step of nanoimprinting. In some embodiments, a step of nanoimprinting or embossing changes surface features (e.g. Imprint diffraction grating). In some embodiments, steps of imprinting or embossing include forming structures between a couple of nanometers to several hundred millimeters.

The present disclosure provides an unprecedented method to rapidly fabricate amphiphilic polypeptide materials that have the capability to form large monoliths, with the possibility to store and stabilize macromolecules and bioactive molecules in the monolithic construct. Additionally, the anisotropy of the material can be controlled together with the crystallinity degree of the material.

Protein concentration, geometrical factors, environmental conditions and methodology used to induce protein dehydration are then the critical factors that direct the formation of intra-molecular or inter-molecular hydrogen bonds and ultimately regulate the size and the material properties of the resulting silk fibroin material.

In some embodiments, provided amphiphilic polypeptide materials are manufactured according to present methods, a time to fabricate an amphiphilic polypeptide material is less than 1 week.

EXEMPLIFICATION

The following examples illustrate some embodiments and aspects of the invention. It will be apparent to those skilled in the relevant art that various modifications, additions, substitutions, and the like can be performed without altering the scope of the invention, and such modifications and variations are encompassed within the scope of the invention as defined in the claims which follow

**Example 1**

The present Example describes synthesis and characterization of a silk polypeptide materials as provided herein.

## Materials and Methods

In some embodiments, the all-water fabrication of silk fibroin monoliths includes a Solution-Gel-Solid transition. FIG. 1 shows an all-water process by which silk fibroin monoliths form. FIG. 1 at panel (a) shows a physical transition from a silk solution to a gel to a solid. A Solution-Gel-Solid transition comprises three steps, including: i) regeneration of silk fibroin in water solution; ii) sol-gel transition of silk solution with any of the methods reported in literature but preferably with e-gel formation or spontaneous self-assembly; and iii) gel-solid transition (i.e. fusion of the particles composing silk gel into silk monoliths) by removal of free water and of part of the freezing-bound water at T ≥ 0 °C.

### Silk Fibroin Regeneration

Cocoon from *Bombyx Mori* were used as source of fibroin. Extraction of silk fibroin was achieved by standard degumming process, which involved boiling (t= 5 to 60 minutes) 2.5 g of chopped silk cocoons per liter of 0.02 M sodium carbonate solution. Silk fibroin was then solubilized in 9.3 M lithium bromide for 4 hours in a 60 °C oven. The chaotropic salt was subsequently removed through dialysis (3.5 kDa MWCO) against Milli-Q water for a total of 72 hours, yielding an 8 wt% silk fibroin solution. The resulting SF solution was then purified by centrifugation at 9000 rpm (~ 12,700 g) over two 25 minute-long periods, at a constant of 4 °C.

FIG. 1 at panel (b) shows a silk solution. In a solution state, silk is characterized by micelles that are substantially in particle form. In solution, silk-fibroin heavy chain is present in an amorphous configuration showing no intermolecular interactions and lacking in crystalline structure. Indeed, such silk solutions are characterized by electrostatic repulsions and as a consequence solutions lack any conformational stability.

### Sol-Gel Transition

Silk fibroin sol-gel transition was achieved with methods already reported in literature. In particular, the formation of silk fibroin hydrogel starting from solutions at 2-18 wt% was achieved through: electrogelation, decrease in solution pH, sonication of silk solution, vortexing of silk solution, exposure to polar solvent (e.g. acetone or ethanol), self-gelation at various temperatures (2 < T < 90 °C). Alternatively, silk aquagel were achieved by concentrating silk solution in water at concentration > 20 wt%.

FIG. 1 at panel (c) shows a state of transition from a silk solution to a silk gel. A sol-gel transition is characterized by intermicellar interactions. During a sol-gel transition, silk-fibroin heavy chain of silk gels is present in helical and beta-sheet configuration showing intermolecular interactions and showing some evidence of intermolecular crosslinks. During a sol-gel transition, silk gels are characterized by a metastable confirmation.

### Gel-Solid Transition

Gel-solid transition was achieved by exposing gels (either hydrogel or aquagel) to air (either forced or still or forced, 0 < RH < 70) at several temperatures (0 < T < 90 °C). In some cases (particularly for aquagel) a mold made of semipermeable membrane (3.5 kDa MWCO) was used to maintain the silk gel with a defined shape.

FIG. 1 at panel (d) shows a state of transition from a silk gel to solid silk. A gel-solid transition is characterized by a fusion of micelles that forms crystalline silk fibroin constructs. During a gel-sol transition, silk-fibroin heavy chain is present in beta-sheet configuration showing intermolecular crosslinks. During a gel-sol transition, hard silk or solid silk is characterized by a stable confirmation.

## Material Characterization

Morphologically, silk fibroin monoliths were analyzed through scanning electron microscopy (SEM) and atomic force microscopy (AFM). Fibroin monolith residual water content (non-bound and bound freezing water) was analyzed through thermogravimetric analysis (TGA) at RH=30%. Silk fibroin conformation in the monolithic structure was analyzed through ATR-FTIR, μ-Raman and XRD analysis. The mechanical properties of silk fibroin were assessed through unconfined compression tests.

## Results and Discussion

The quasi-static removal of water from the gels resulted in the formation of silk monolithic blocks of defined dimensions, crystallinity and mechanical properties. The all-water based green processing does not compromise the biocompatibility of the material.

All the blocks were translucent with an amber-like color. The block density was equal to 1.4 Kg/dm$^3$, equal to the estimated bulk density of silk fibroin. Silk blocks exhibited a bound-water content (freezing and non-freezing) of less than 20 wt%, as measured from thermogravimetric analysis. In addition, silk fibroin monoliths presented all the advantageous characteristics of silk films in a 3D structure. FIG. 2 shows a morphological characterization of silk fibroin monoliths. FIG. 2 at panel (a) shows an Scanning Electron Microscope (SEM) image of a silk fibroin monolith. FIG. 2 at panel (b) shows an Atomic Force Microscope (AFM) image of a silk fibroin monolith. FIG. 2 at panel (c) shows an AFM analyses of formation of smooth surfaces, with an RMS of few nm. Surface roughness was in the order of nanometers (RMS= 21 nm as from AFM), which enables the integration of electronic component on the monoliths' surface. In addition, the all-water based processing allows for the incorporation of labile compound (antibiotic, vaccines, growth factors, ...) in the monoliths, which preserved their activity outside 'normal' storage conditions, as previously seen in other silk formats (still under experimentation).

FIG. 3 shows spectroscopic characterization of silk fibroin monoliths obtained through electrogelation. FIG. 3 at panel (a) shows a μRaman analysis was used to monitor silk fibroin conformational changes during the process of monolith formation. Amorphous silk fibroin was converted to a helical protein during the e-gel formation (sol-gel transition). Silk gel was converted by dehydration of the protein during the formation of silk fibroin monolith (gel-solid transition). Solid formation corresponded with the formation of highly crystalline, antiparallel beta-sheeted structures. FIG. 3 at panel (b) shows an ATR-FTIR with an Amide I peak centered at 1621 cm$^{-1}$ that were used to silk fibroin monoliths with highly crystalline, antiparallel beta-sheeted structures. Polarized ATR-FTIR showed a preferential orientation of the silk fibroin crystals along the direction of the electric field used during the electrogelation process. FIG. 3 at panel (c) shows X-ray diffraction (peak around 8 and 21 2theta) measurements that were used to show silk fibroin monoliths with highly crystalline, antiparallel beta-sheeted structures.

FIG. 4 shows mechanical properties of silk fibroin monoliths. Mechanically, silk monoliths displayed outstanding mechanical properties (compressive modulus = 97.65±15.26 MPa, yield strength = 15.32±4.21 MPa). In addition, anisotropy can be induced in the material at the point of gel formation, which can be used to tailor the mechanics of these structures. Anisotropy can be induced during sol-gel transition through electric fields or at the point of material drying (gel-solid transition), through semi-confinement of the gel in non-water permeable environments, which results in preferential water evaporation. Silk monoliths are machinable (e.g. with a lathe), compostable and their crystallinity can be tailored a priori during gel fabrication (by regulating sol-gel transition) and a posteriori with already developed post-processing techniques (e.g. heat, polar solvent treatment...).

**Example 2**

The present Example provides amphiphilic polypeptide materials and properties thereof are described. This Example demonstrates how such amphiphilic polypeptide materials could be manufactured and/or machined into structures. Additionally, the present Example also describes amphiphilic polypeptide materials provide functional silk fibroin monoliths.

Amphiphilic polypeptide materials in this Example were prepared according to a sol-gel-solid transformation as described in Example 1.

Results and Discussion

*Light Induced Heating and/or Thermolysis*

FIG. 5 at panel (a) and (b) show a silk polypeptide material bulk-loaded with Au nanorods (AuNR) and machined into a 1 cm screw.

The silk polypeptide screw was exposed to about 1 W of 850 nm light. Upon illumination with an LED, the AuNR of silk polypeptide screw absorbed energy. FIG. 5 at panel (c) shows that absorption by the Au-NRs caused the screw to heat up to about 158 °C.

When the LED power increases, the mechanical component combusts, via thermolysis. Components made from provided amphiphilic polypeptide materials, for example, degradable screws, nuts, bolts, and hardware pieces would fall apart on command with exposure. Such functional materials can be machined into multiple forms, such as mechanical containers. Such a material container could carry heat activated chemicals to be triggered remotely to dissolve the device.

*Thermoplasticity*

Upon heating to a temperature of about 70 °C - about 80 °C, a silk polypeptide material as provided herein shows thermoplastic behavior.

FIG. 6 illustrates a silk polypeptide material, a monolith. The silk fibroin monolith was sized to be about 50 mm x 50 mm x 5 mm.

The silk fibroin monolith superficially was patterned during the gel-solid transition at room temperature. The monolith was patterned through embossing at about 100 kPa at Room Temperature. One skilled in the art would expect effective patterning of polypeptide materials in a range between about 10 Pa and about 500 MPa. That is, upon crystallization, the surface of the material can be plastically modified via a low-pressure (10 Pa < P < 500 MPa), low-heat (25 °C < T < 170 °C).

While not wishing to be bound to a specific theory, it is believed that a silk fibroin surface is patterned by a combination of temperature and pressure plastically deforming a silk fibroin material surface. Plastically deforming a silk fibroin material surface occurs at the point of water evaporation.

A size of patterned structures is only limited by the embossing. The pattern shown in FIG. 6 at panel (a) clearly shows "Tufts University." The minimum feature size as shown in FIG. 6 at panel (b) is approximately 250 nm.

The plastic deformation is stable over time and with temperature. The patterned surface has remained stable for in excess of 2 months. The patterned surface has remained stable at temperatures between -80 °C and about 220 °C.

Amphiphilic polypeptide materials having such surface thermoplasticity would benefit anti-counterfeiting, photonic materials, cell guidance, etc.

A silk polypeptide monolith was formed into a pin shape. FIG. 7 illustrates a silk fibroin pin ($\bigcirc \cong 1$ mm, length = 60 mm). As formed, the silk fibroin pin was substantially straight.

As shown in FIG. 7 at panel (a), is a photograph showing one end of the pin as it was gripped by an extremity in a vice. A load of 200 g was applied to the opposite and protruding end of the pin. FIG. 7 at panel (b) shows an infrared thermography (IRT) image of the pin. The image shows the temperature of the silk fibroin pin to be about 18 °C to about 25 °C, i.e. approximately room temperature. FIG. 7 at panels (a) and (b) show that the 200 g weight did not cause the pin to bend. The pin remained substantially straight when the weight was applied without temperature.

The pin then was heated with a heat gun. FIG. 7 at panel (c) shows a photographic image of the pin under heat. The pin appeared slightly bent with increasing temperature. FIG. 7 at panel (d) shows that the temperature of the pin was at least 70 °C. The angle of the IRT images also confirms that the pin was at least slightly bent from substantially straight.

FIG. 7 at panel (e) shows a photographic image of the pin under additional heat. FIG. 7 at panel (f) shows that the temperature of the pin was at least 70 °C. The pin clearly appears bent. The angle of the pin in IRT images confirms that the pin was bent from substantially straight.

The silk fibroin pin plastically deformed with temperature and pressure. As shown in FIG. 7 at panels (a) and (b), the pin did not deform with the pressure of a 200 g alone. Although the data is not shown herein, the pin did not deform with temperature alone.

While not wishing to be bound to a specific theory, it is believed that the silk fibroin material deformation occurred by a plastic deformation induced with pressure and heat. It is believed that in some embodiments, application of temperature and heat results in a local conformational change of the material from amorphous to crystalline. Upon partial crystallization via a combination of the downward pressure from the 200 g weight and the heat (25 °C < T < 170 °C), the material shows a partial thermoplastic behavior as its shape was (partially) plastically modified. One skilled in the art would appreciate that varying temperatures and pressures achieve conformational change of the material from amorphous to crystalline and a plastic deformation corresponding thereto.

Amphiphilic polypeptide materials having such bulk thermoplasticity would benefit in fabrication with techniques that use thermoplastic behavior of polymers (e.g. extrusion, injection molding).

*Preservation and Release of Heat-labile Molecules*

FIG. 8 shows silk fibroin monoliths and their ability to preserve and release heat-labile molecules. FIG. 8 at panel (a) shows a photographic image of four silk fibroin monoliths machined into screws. FIG. 8 at panel (a) at the far left and annotated "No HRP", shows a silk fibroin monolith machined into a screw in which no horse radish peroxidase ("HRP") has been bulk loaded into the monolith. FIG. 8 at panel (a) at second from the left and annotated "Low HRP", shows a silk fibroin monolith machined into a screw in which 0.2U/10 mg$_{silk}$ HRP has been bulk loaded into the monolith. FIG. 8 at panel (a) at second from the right and annotated "Mid HRP", shows a silk fibroin monolith machined into a screw in which 1U/10 mg$_{silk}$ HRP has been bulk loaded into the monolith. FIG. 8 at panel (a) at the far right and annotated "High HRP", shows a silk fibroin monolith machined into a screw in which 2U/10mg$_{silk}$HRP has been bulk loaded into the monolith.

The silk fibroin screws were left at room temperature for 14 days. It is noted that HRP not encapsulated in silk is not shelf-stable under these storage conditions.

The HRP was released from the screws by exposure to a wet environment.

On release, HRP was visualized via a reaction with a chromogenic substrate, in this case 3,3',5,5'-Tetramethylbenzidine (TMB). Upon exposure to a wet environment containing TMB, the silk fibroin screws containing HRP, released the HRP producing a blue color.

FIG. 8 at panel (b) shows a photographic images of silk fibroin monoliths machined into screws and submerged in a solvent containing TMB. The images show the screws at T=0s or concurrent with submersion. FIG. 8 at panel (b) on the left and annotated "No HRP" shows a silk fibroin monolith machined into a screw in which no HRP has been bulk loaded into the monolith and submerged in a solvent, T=20s. The submerged "No HRP" silk fibroin screws not display blue coloring.

FIG. 8 at panel (b) on the right and annotated "Mid HRP" shows a silk fibroin monolith machined into a screw in which 1U/10 mg$_{silk}$ HRP has been bulk loaded into the monolith and submerged in a solvent, T = 0 s. FIG. 8 at panel (c) shows a photographic images of silk fibroin monoliths machined into screws and submerged in a solvent. The images show the screws at T = 20 s. FIG. 8 at panel (c) on the left and annotated "No HRP" shows a silk fibroin monolith machined into a screw in which no HRP has been bulk loaded into the monolith and submerged in a solvent, T = 20 s. FIG. 8 at panel (c) on the right and annotated "Mid HRP" shows a silk fibroin monolith machined into a screw in which 1U/10 mg$_{silk}$ HRP has been bulk loaded into the monolith and submerged in a solvent, T = 20 s. The submerged "Mid HRP" silk fibroin screws visually showed blue coloring migrating into the solution indicating HRP release. The HRP was sustainably released for a period of 48 hours.

While not wishing to be bound to a specific theory, it is believed that the silk fibroin materials provided protection to the HRP against oxidative stress thereby preserving HRP in for extended periods. Moreover, exposure of silk fibroin materials containing HRP (or other labile materials) to solvents have predictable degradation and release profiles.

Amphiphilic polypeptide materials having such preservation and release profiles would benefit in fabrication with cell instruction, drug release, preservation of heat-labile compounds, and sensing applications to name a few.

## Example 3

The present Example provides amphiphilic polypeptide materials. Amphiphilic polypeptide materials in this Example were prepared according to a sol-gel-solid transformation as described in Example 1. This Example demonstrates mechanical properties of such materials. This Example also demonstrates a comparison of materials (e.g. Monoliths) fabricated with silk solutions of HFIP with aqueous silk solutions.

Results and Discussion

*Compressive modulus*

FIG. 9 shows analysis of the compressive modulus of silk fibroin materials as a function of molecular weight. Modulus is a measure that characterizes that softness or hardness of a material. Compressive modulus is a resistance to permanent deformation after a force is applied. Modulus is equal to stress divided by strain. The stress is the pressure. The strain is the ratio of the change in thickness to the original thickness. As shown in FIG. 9, compressive modulus is directly correlated with increasing molecular weight of silk fibroin. Silk fibroin materials having a molecular weight between about 350 kDa and 230 kDa had a compressive modulus of about 9 GPa. Silk fibroin materials having a molecular weight between about 310 kDa and 150 kDa had a compressive modulus of about 8 GPa. Silk fibroin materials having a molecular weight between about 220 kDa and 95 kDa had a compressive modulus of about 6 GPa. Silk fibroin materials having a molecular weight between about 130 kDa and 75 kDa had a compressive modulus of about 2 GPa. Silk fibroin materials formulated from silk fibroin having a molecular weight of between about 220 kDa and about 95 kDa in aqueous solutions were compared with monoliths formulated from silk fibroin having a molecular weight of between about 220 kDa and about 95 kDa in HFIP. Compressive modulus silk fibroin materials made from aqueous solutions was shown to be about three times that of silk fibroin materials made from solutions containing HFIP as a solvent.

*Shear Stress*

FIG. 10 shows analysis of the shear stress of silk fibroin materials as a function of molecular weight. Shear stress is an external force acting on a material surface parallel to the plane in which it lies. The shear stress acts in plane to the stressed area at right-angles to compressive stress as above addressed. The shear stress is the stress that tends to shear the material. As shown in FIG. 10, shear stress is directly correlated with increasing molecular weight of silk fibroin. Silk fibroin materials having a molecular weight between about 350 kDa and 230 kDa had a shear stress of about 120 MPa. Silk fibroin materials having a molecular weight between about 310 kDa and 150 kDa had a shear stress of about 100 MPa. Silk fibroin materials having a molecular weight between about 220 kDa and 95 kDa had a shear stress of about 90 MPa. Silk fibroin materials having a molecular weight between about 130 kDa and 75 kDa had a shear stress of about 80 MPa. Silk fibroin materials formulated from silk fibroin having a molecular weight of between about 220 kDa and about 95 kDa in aqueous solutions were compared with monoliths formulated from silk fibroin having a molecular weight of between about 220 kDa and about 95 kDa in HFIP. Shear stress for silk fibroin materials made from aqueous solutions were slightly higher but comparable to silk fibroin materials made from solutions containing HFIP as a solvent.

*Pull-Out Strength*

FIG. 11 shows analysis of the pull-out strength of silk fibroin materials as a function of molecular weight. As shown in FIG. 11, pull-out strength is directly correlated with increasing molecular weight of silk fibroin. Silk fibroin materials having a molecular weight between about 350 kDa and 230 kDa had a pull-out strength of about 160 N. Silk fibroin materials having a molecular weight between about 310 kDa and 150 kDa had a pull-out strength of about 150 N. Silk fibroin materials having a molecular weight between about 220 kDa and 95 kDa had a pull-out strength of about 130 N. Silk fibroin materials having a molecular weight between about 130 kDa and 75 kDa had a pull-out strength of about 90 N. Silk fibroin materials formulated from silk fibroin having a molecular weight of between about 220 kDa and about 95 kDa in aqueous solutions were compared with monoliths formulated from silk fibroin having a molecular weight of between about 220 kDa and about 95 kDa in HFIP. Pull-out strength for silk fibroin materials made from aqueous solutions was more than 50% greater than that of silk fibroin materials made from solutions containing HFIP as a solvent.

**Example 4**

The present Example describes synthesis of a silk polypeptide materials as provided herein. Such silk polypeptide materials in this Example were prepared according to a solution to solid transformation.

Materials and Methods

*Preparation of aqueous silk solution:*

In some embodiments, the all-water fabrication of silk fibroin monoliths includes a Solution to Solid transition. Silk fibroin solution was prepared from *B. mori* cocoons using our established protocols with some modifications. (See D. N. Rockwood, 6 Nat. Protoc., 1612 (2011)). First, sericin was removed by boiling the cocoon pieces in 0.02 M aqueous $Na_2CO_3$ solution followed by extensive rinses in distilled water. The degummed silk was then dried overnight and dissolved in 9.3 M LiBr at 60 °C for 4 hours, yielding a 20% (w/v) solution. The pH of LiBr solution was adjusted by adding 1 M LiOH solution so that the pH of the final silk solution after dialysis was 8.0. The silk/LiBr solution was dialyzed against distilled water for 2 days with 10 changes of water. The solution was centrifuged for 2 x 20 min at 9,000 rpm. The silk concentration was determined by evaporating water from a solution of known weight and weighing the remaining solid using an analytical balance.

*Preparation of Biomimetic Materials from an Aqueous Silk Solution:*

Provided biomimetic, all-aqueous regenerated silk-based materials are useful in fabrication of orthopedic devices. Silk materials generated replicate the nano-scale structure of natural silk fibers and resulting materials and devices possess excellent mechanical properties. Further, provided silk materials are machinable, providing a path towards the fabrication of a new family of resorbable orthopedic devices where organic solvents are avoided, thus allowing functionalization with bioactive molecules to promote bone remodeling and integration.

The outstanding mechanical properties of natural silk fibers derive from their unique structure and spinning process. FIG. 12 shows a schematic illustration of the fabrication process and characterization of the biomimetic silk materials. FIG. 12 at panel (a) is a schematic of the natural spinning process used by the silkworm, *Bombyx mori.* In such process, the silk fibroin (hereafter referred to as silk) concentration increases gradually and controllably from ~12% to 30% as the silk molecules move from the posterior to the anterior region of the silk glands. Meanwhile, the chains assemble to micelles, follow by arrange and stack them together in a step-by-step manner, and form the compact solid architecture under regulation of external environments, such as pH, ion concentration, physical shear and/or elongational flow. Many efforts have been explored to regenerate silk materials with mechanical properties comparable to or exceeding those of natural silk fibers. (See J. Luo, 66 Int. J. Biol. Macromol., 319 (2014); see also M. Sun, 22 J. Mater. Chem. 18372 (2012); and G. Zhou, 21 Adv. Mater., 366 (2009)). One common feature of those studies is a spinning dope of highly concentrated silk solutions, which is a prerequisite to form a dense, compact solid structure. However, the focus has mainly been limited to the generation one-dimensional silk fibers in the micrometer range, which significantly limits their application. There is need to develop three-dimensional silk materials in larger dimensions that can be used to fabricate devices with high mechanical demands, such as orthopedic devices.

Metals like titanium alloys and stainless steel remain the gold standard for orthopedic devices due to their robust mechanical properties and ease of fabrication and implantation, while limitations of stress shielding, infections, bone remodeling, and second surgical removal have shifted significant interest to degradable devices. (See D. S. Goldberg, 6 J.

Craniofac. Surg. 301 (1995); and J. Zhang, 72 J. Trauma Acute Care Surg., E106 (2012)). Resorbable orthopedic devices composed of poly-L-lactic acid and polyglycolic acid reduces the need for hardware removal and improved bone remodeling. However, the degradation of these resorbable devices is associated with inflammatory foreign body reactions due to the acidic degradation products. (See D. Eglin, 16 European cells & materials, 80 (2008)). Therefore, there remains need to develop fully degradable orthopedic devices that can overcome these limitations.

Silk is an unique candidate to address this issue due to its excellent mechanical properties and biocompatibility as well as tunable degradability. (See G. S. Perrone, Nat. Commun. 5 (2014); and C. Vepari, 32 Prog. Polym. Sci. 991 (2007)). Inspired by the natural spinning process, provided materials are formed by an all aqueous, 'green' and controllable strategy to create silk materials with unique structure and excellent mechanical properties in large dimensions. FIG. 12 at panel (b) shows an aqueous process to create silk materials with unique structure and excellent mechanical properties in large dimensions. Silk blanks were prepared by a four-stage dehydration process. Aqueous silk solution of 25-30%, matching the silk concentration in the anterior region of silkworm gland, was first obtained by a slow dehydration process. The pH of the silk solution was controlled at 8.0 to prevent the hydrogel formation during dehydration. The concentrated silk solution was then loaded into molds to allow further water removal at 10 °C without forming a hydrogel, resulting in solid silk blanks. The remaining water was removed by further dehydration at elevated temperatures. A remarkable feature of the silk blanks is their machinability. The silk blanks can be machined into various types of orthopedic devices, such as bone screws, plates and intramedullary (IM) nails. FIG. 12 at panel (c) shows that silk blanks can readily be machined into other desired shapes, such as a cylinder, a triangular prism, a star and a flash sign. Therefore, this new, bio-friendly silk biomimetic material may find use in the fabrication of engineered components with complex shapes and desired functionalities. We previously demonstrated the feasibility of silk protein as a molding and machinable biomaterial system for the preparation of devices for craniofacial repairs, based on the use of an organic solvent-based process (1,1,1,3,3,3 hexafluro-2-propanol (HFIP). (See G. S. Perrone, Nat. Commun. 5 (2014)). In comparison to the solvent-based system, the advent of an aqueous silk formulation to generate orthopedic devices with improved mechanical properties eliminates the risks with the use of toxic organic solvents. Furthermore, the mild aqueous-based process facilitates the addition of dopants and bioactive compounds, thus providing additional clinical benefits like osteoinductive, osteoconductive and anti-inflammatory features.

Structural insight into the self-assembled biomimetic materials was obtained by scanning electron microscopy (SEM) and atomic force microscopy (AFM). FIG. 12 at panels (d), (e), and (f) show the combination of the two techniques revealed a uniform and dense structure composed of silk globules of about 15 nm to about 30 nm in size, reminiscent of the globular structure of the native silk fiber. (See J. Pérez-Rigueiro, 40 Macromolecules, 5360 (2007)). Spider silk showed isotropic ~10 nm globules, whereas silkworm silk generates globules that are anisotropic ~23 nm x 16 nm. Another unique feature of the regenerated silk materials is the hierarchical organization of the material. FIG. 12 at panel (e) shows silk nano-globules that were formed from aggregates of tens to hundreds of nanometer in size and these aggregates interacted with each other to form a dense network. As shown in FIG. 12 at panel (f), resulting materials exhibit a microstructure composed of interlocked nano-globules, which is also the fundamental structures of nanofibrils in natural silk fiber. These nano-globules of different sizes, which are responsible for the nonslip kinematics, restricted shearing between fibrils, allowing controlled local slipping and energy dissipation without bulk fracturing. (See C. P. Brown, 6 ACS Nano, 1961 (2012)).

FIG. 12 at panel (g) shows Fourier transform infrared spectroscopy (FTIR) that was used to monitor the structural changes during the assembly process of the silk materials. The 1515 cm$^{-1}$ band in amide II region, assigned to the aromatic ring C-C stretching of the tyrosine (Tyr) side chain, is a local monitor for the conformational change and hydrophilicity of the Tyr microenvironment. (See D. Reinstädler, 34 Proteins: Struct. Funct. Bioinform., 303 (1999)). A decrease in the wavenumber to 1512 cm$^{-1}$ was observed with dehydration, which could be related to a more hydrophobic microenvironment induced by peptide folding. (See A. Barth, 74 Progress in Biophysics and Molecular Biology, 141 (2000)). Figure 12 at panel (g) shows that control of solution pH and slow dehydration process allows sufficient time for fibroin chains to self-assemble and thus β-sheet content increases over time.

As shown in FIG. 12 at panel (g, at plot A), silk molecules are present as oligomeric aggregates in concentrated silk solutions adopting mainly random coil structure, similar to the silk molecules in spinning duct. As the local protein concentration increases with further dehydration, crystal nucleation between protein chains is trigged, leading to the formation of ordered fibrous/globular intermediate structures, consisting of nano-sized globules. While water molecules continue to leave the system, these intermediate structures formed a network *via* hydrogen bonds and eventually a densely-packed solid silk material. FIG. 12 at panel (g) (plot B) shows that after dehydration at 10 °C for 3 days and room temperature for 4 days, absorbance bands appeared around 1632 and 1697 cm$^{-1}$, indicating the formation of intramolecular and intermolecular β-sheet structure, respectively. (See X. Hu, 39 Macromolecules, 6161 (2006)). FIG. 12 at panel (g) (plot C) shows that after further dehydration at 45 °C did not induce significant changes in silk structure.

Considering the highly structural similarity with natural silks as well as superb mechanical performance of natural silk fibers, we sought to evaluate the mechanical properties of the biomimetic silk materials. The compressive stress-strain profile was similar to that of natural bone: an elastic region followed by a plastic deformation phase. FIG. 12 at panel (h) shows compressive yield strength of 123.6 ± 8.6 MPa and modulus of 4.2 ± 0.4 GPa (silk) were well above the mechanical tolerance of cancellous bone (2-12 MPa and 50-500 MPa, respectively) and approached that of cortical bone (100-230 MPa and 7-30 GPa, respectively). Remarkably, the mechanical properties of biomimetic silk materials can be further improved by adding inorganic fillers. For example, when 20% nano-hydroxyapatite (HAP), a natural component of bone, was included in the silk materials (Silk/HAP), the compressive yield strength and modulus increased to 164.0 ± 5.9 MPa and 6.4 ± 0.7 GPa, respectively. FIG. 12 at panel (i) shows a comparison of specific strength and modulus of the biomimetic silk material with natural silk materials, bone, polymer, ceramic and metal/alloys. (Adapted with permission from nature publishing group, see U. G. K. Wegst, 14 Nature Mater., 23 (2015)). The yellow ellipse represents the silk and silk/HAP materials in this study. FIG. 12 at panel (j) shows the structural evolution of silk molecules in our designed a biomimetic fabrication process as provided herein, according to the aforementioned structural evidences. Protein molecules tend to further form reversible cluster/aggregates due to the contribution of attractive forces and repulsive excluded volume effect. (See A. Saluja, 358 Int. J. Pharm., 1 (2008); A. Stradner, 432 Nature, 492 (2004); E.J. Yearley, 106 Biophys. J., 1763 (2014)).

## Example 5

The present Example describes formation of orthopedic devices from silk polypeptide materials as provided herein. Such silk polypeptide materials in this Example were prepared according to a solution to solid transformation as described in Example 4.

<u>Materials and Methods</u>

*P24 Synthesis*

The BMP-2-related peptide P24 (SKIPKASSVPTEL-SAISTLYLDDD) was synthesized by Tufts university core facility. The peptides were made on an ABI 431 Peptide Synthesizer using Fmoc chemistry and HBTU activation. The purity of the peptides were greater than 90%.

*Preparation of Silk-based Blanks for Machining:*

Silk solution of 6-8 % (wt/wt) was subjected to forced airflow and water was slowly removed at 10 °C until the silk concentration reached 25-30%. The concentration of the solution was monitored by weighing the remaining solid after drying. Rectangular molds with water-permeable membranes were used to prepare silk blanks for machining. As an example, 3-12 ml Slide-A-Lyzer dialysis cassette (Thermo Fisher, USA) with inner chamber dimensions of 65 mm × 28 mm × 6.5 mm was loaded with concentrated silk solution and placed into a refrigerated incubator with forced air flow at 10 °C for 3 to 4 days. Water evaporated through the porous water-permeable membrane from both sides of the cassette and resulted in solid silk materials. The materials were then left in a fume hood for 4 days followed by another 4 days in a 45 °C oven to remove the remaining water. To produce silk-based composite materials, silica (20-200 nm, Sigma, USA) and hydroxyapatite (200 nm, Sigma, USA) were first dispersed in water by sonication and mixed with silk fibroin solution to obtain a suspension with desired amount of $SiO_2$ or HAP. The same dehydration procedure as mentioned above was conducted to obtain silk/$SiO_2$ and silk/HAP blanks. To produce antibiotic-containing silk materials, a suspension of ciprofloxacin·HCl in water was mixed with 26.5% (wt/wt) silk solution to a final ciprofloxacin content of 5 % (wt/wt). The mixture was loaded into molds and dehydrated at 10 °C for 3 days followed by room temperature drying in a fume hood for 2 weeks. Osteoinductive silk materials were obtained in a similar way except that BMP-2 (Wyeth, USA) and P24 solution was mixed with concentrated silk solution at a concentration of 30 μg BMP-2/g silk and 1.0 mg P24/g silk, respectively.

*Machining of Silk Orthopedic Devices:*

The silk blanks were machined into screws and IM nails using a CNC lathe (Trak TRL 1440 EX). For IM nails, the nail blank was left on the CNC lather once the desired diameter was reached and a needle type tip was placed on the end for insertion. For screws with bone screw threads, a custom single point external cutter (Vargus, USA) was used on the CNC lathe to cut screw threads by matching turning speed with horizontal speed of the cutter to cut a desired pitch length (outer diameter ~ 1.8 mm, pitch = 600 μm). The screw heads were machined to have a cylindrical heads by use of the CNC lathe. Once machined, the screw or IM nail was then cut off behind the head or length of nail. A diamond cutter was mounted to the lathe and used to cut a slot in the screw head for screw insertion. For plates, rectangular silk blanks were machined using a CNC milling machine (Trak DPM). The milling machine was used both to cut the desired shape of the mold and thickness. Once the plate was shaped and sized, cylindrical or conical screw holes were cut using a 90 degree Ford countersink.

*Characterization:*

The morphology of the biomimetic silk materials and silk-based orthopedic devices were characterized by scanning electron microscopy (SEM) and atomic force microcopy (AFM). The structure was analyzed with a JASCO 6200 FTIR spectrometer .

SEM: For morphological analysis, samples were sputtered coated with platinum and imaged with a Zeiss Supra 55VP SEM (Cal Zeiss, Germany). Silk rod of 1.5 mm was fractured in liquid nitrogen and fracture surface was imaged under SEM.

AFM: AFM was performed on a MFP-3D-BIO AFM (Asylum Research, USA) with tapping mode. Cr/Au(5/45) coated silicon cantilever with a tip radius below 10 nm was used (k = 2 N/m, v ~ 70 kHz, Asylum Research, USA).

ATR-FTIR: SF solution of 26.5% was flash frozen in liquid nitrogen and freeze dried to preserve original structure before FTIR analysis. FTIR spectra were gathered utilizing FT/IR-6200 Spectrometer (Jasco, USA), equipped with a triglycine sulfate detector in attenuated total reflection (ATR) mode. The measurements were taken in the range of 4000-400 cm$^{-1}$ at a resolution of 2 cm$^{-1}$ with 128 scans. The background spectra were collected under the same conditions and subtracted from the scan for each sample.

*Mechanical Testing:*

All mechanical tests were performed in the dry state under ambient conditions.

Compression tests: Silk blanks were machined into cylinders of 4 mm in diameter and 4 mm in height. The compressive strength and modulus of the cylinders were measured using an Instron 3366 (Instron USA) frame at a crosshead speed of 5 mm/min.

Shear test: The double lap shear test was performed on silk pins of 1.5 mm diameter. FIG. 19 at panel (a) shows a fixture that consists of three adjacent stainless steel plates with aligned holes of 1.5 mm that allow the tight fitting of the silk pins. The bottom fixture remained stationary and the top fixture was mounted to an Instron 3366 test frame and compressed at a rate of 5 mm/min until fracture of the pins (ASTM standard F2502-11). (See K. J. Bozic, 26 J. Hand Surg. Am., 755 (2001)). Maximum shear stress was determined from the maximum load and the cross section area of the pins.

Bending test: FIG. 18 shows silk plates with a width of 7 mm, length of 29 mm and thickness of 2 mm were tested for bending strength. The plates were 4-hole plates with 2 mm diameter holes. Holes were placed 3.5 mm from the ends of the plates with the nearest hole separated by 4.5 mm. The 2 center holes were separated by 13 mm. FIG. 19 at panel (b) shows a 4-point bending fixture was machined from stainless steel with brass loading rollers and support rollers, allowing rolling during testing to avoid frictional forces. The fixture has a loading span and center span of 6 mm with support rollers placed directly between the 2 outer holes. The bottom fixture remained stationary while the top fixture was mounted to an Instron 3366 test frame and compressed at a rate of 5 mm/min until fracture or complete compression to the bottom fixture. The bending stiffness, bending structural stiffness and bending strength were determined from the load vs. load-point displacement curve using the methods described in ASTM standard F2502-11.

Bending modulus was determined using equation (1):

$$(1)\ E_{4p} = \frac{m_{4p}L_0^3}{8wt^3}\text{q}$$

(See F. Mujika, 25 Polym. Test., 214 (2006)).
Where:

E$_{4p}$ = 4-point bending modulus (MPa)

M$_{4p}$ = 4-point bending stiffness (N/mm)

L$_0$ = length between support rollers (mm)

t = thickness of plate (mm)

w = width of plate (mm).

*In Vitro Zone of Inhibition Testing and Antibiotics Release:*

In vitro antibacterial effect of the ciprofloxacin-loaded silk orthopedic device was estimated by using the Kirby-Bauer susceptibility test. Briefly, 50 $\mu$l of the *S. aureus* (ATCC 25923) overnight culture with O.D. of 0.8-1.0 were plated on Tryptic Soy Agar plates. After 24 hours, the zone of inhibition was measured using image J software. Ciprofloxacin-containing silk pins (1.5 mm in diameter and 3-4 mm in length) were placed on the plates and the incubated overnight at 37 °C to allow bacteria to grow. The ciprofloxacin release was determined by immersing drug-loaded silk pins in Dulbecco's PBS (0.2 mL) at 37 °C. At desired time points, the buffer was removed and replaced with fresh buffer. The amount of release was determined using a direct zone of inhibition assay. The active antibiotic was quantified by comparing zone of clearance in bacteria lawns with zones of clearance generated by standards of known antibiotic concentration.

*Human Mesenchymal Stem Cell (hMSCs) Isolation and Expansion:*

hMSCs were isolated from total bone marrow aspirate from a healthy, non-smoking male order under the age of 25 (Lonza, USA). Whole bone marrow was diluted in expansion medium, plated in 175 cm$^2$ flasks (Corning, USA) at a density of 200,000 cells/cm$^2$ and cultured at 37 °C with 5% $CO_2$ in a humidified environment. Expansion media consisted of Dulbecco's modified eagle medium (DMEM), 10% fetal bovine serum (FBS), 1% non-essential amino acids, 1 ng/ml bFGF, and 1% antibiotic/antimycotic (ThermoFisher Scientific, NY, USA). Each flask contained a final volume of 35 ml, which was rocked daily to allow hemopoietic cells to remain in suspension and the stromal cells to adhere to the flask. The adherent cells were allowed to reach 80% confluence, after which they were trypsinized, suspended in FBS containing 10% dimethyl sulfoxide (DMSO) and stored in liquid nitrogen. Second passage cells were used for experiments.

*BMP-2 Staining:*

BMP-2 incorporated silk rods were incubated in 10% goat serum at room temperature for 1 hour to block non-specific protein-protein interactions. The rods were then incubated with anti-BMP-2 antibody (1x) overnight at 4 °C. The samples were washed with PBS three times and incubated in Alexa Fluor 488 goat anti-rabbit IgG at a 1/200 dilution at room temperature for 1 hour. The samples were then washed with PBS for three times and images with a Keyence BZ-X710 fluorescent microscope (Keyence, USA).

*Bioactivity of Released BMP-2 from Silk Rods*

The hMSCs were seeded at a density of 1x10$^4$ cells/well in 24-well plates containing BMP-2 incorporated silk rods and maintained in osteogenic medium (DMEM, 10% FBS, 100 nM dexamethasone, 10 mM $\beta$-glycerol phosphate, 0.05 mM ascorbic acid, 1% NEAA, 1% antibiotic/antimycotic). Cells grown in growth medium (DMEM, 10% FBS, 1% NEAA, 1% antibiotic/antimycotic) and osteogenic medium were used as controls. After 4 weeks, the cells were stained for alkaline phosphatase (ALP) and hydroxyapatite formation. ALP was detected using BCIP/NBT (Sigma, USA) as a substrate. OsteoImage mineralization assay kit (Lonza, USA) were used to stain hydroxyapatite deposits according to manufacturer's instructions. The samples were then observed under a Keyence BZ-X710 fluorescent microscope (Keyence, USA).

*Osteogenic Differentiation of hMSCs induced by BMP2/P24-incorporated screws:*

Silk sponge of pore size of 400-500 $\mu$m was prepared as previously described. (See D. N. Rockwood, 6 Nat. Protoc., 1612 (2011)). Tubular silk sponge (inner diameter of 2 mm, outer diameter of 4 mm, height of 4 mm) were cut from the sponge with dermal punch. Ethylene oxide sterilized BMP-2 and P24-loaded silk screws were then inserted into the tubular sponge and one million hMSCs were seeded in the sponge. The construct was then maintained in osteogenic medium for 6 weeks. The harvested constructs were cut in half along the longitudinal axis in order to expose the interface of the screw and sponge. The samples were then stained with OsteoImage and observed under Keyence BZ-X710 fluorescent microscope.

## Example 6

The present Example also provides silk polypeptide materials and properties thereof are described. This Example demonstrates how such silk polypeptide materials could be manufactured and/or machined into structures. Additionally, the present Example also describes silk polypeptide materials provide functional silk fibroin monoliths. Amphiphilic polypeptide materials in this Example were prepared according to a solution to solid transformation as described in Example 4.

Results and Discussion

*Amphiphilic Polypeptide Materials: Orthopedic Devices*

In order to meet the morphology and structure requirements of orthopedic devices, we explored the machinability of the regenerated silk materials generated by the biomimetic aqueous process. FIG. 13 shows silk screws, pins and plates were successfully machined from silk blanks.

FIG. 13 at panel (a) shows the resultant silk screws (major diameter ~1.8 mm) appeared a glossy surface without any defects, and more importantly, showed distinct threads with sharp edges. FIG. 13 at panel (b) shows the screw threads having a relatively homogeneous appearance at low magnification. Refined features were revealed by SEM image, which showed uniform depth of threads (about 100 $\mu$m) with thread pitch of 600 $\mu$m.

FIG. 17 shows a rougher surface at high magnification. In addition, the screw threads displayed a relatively homogeneous appearance at low magnification, (a scale of few hundreds of microns), while a rougher surface at high magnification scale of several microns. A high roughness profile at several microns scale should improve early fixation and long-term stability due to the mechanical interlocking between the implants and bone ingrowth. (See M. M. Shalabi, 85 J. Dent. Res., 496 (2006)). Moreover, this rough topographic features play a decisive role on the biological response to orthopedic implants. (See M. Jager, J. Biomed. Biotechnol. (2007)). Shear forces are also prominently experienced by bone fixation devices. Silk pins with a diameter of 1.5 mm, instead of screws, were used to determine the maximum shear stress. FIG. 13 at panel (g) shows silk pins reached a maximum shear stress of around 80.2 $\pm$ 8.5 MPa, which is comparable to that of the solvent-based systems (~ 90 MPa). (See G. S. Perrone, Nat. Commun. 5 (2014)). These values are also comparable with the current resorbable systems which have a shear force of 100 -185 MPa. (See N. Ashammakhi, 17 J. Mater. Sci. Mater. Med. 1275 (2006); and S. Leinonen, 13 J. Craniofac. Surg. 212 (2002)). FIG. 13 at panel (h) shows the mechanical properties of silk plates were measured by a 4-point bending test. The mechanics of aqueous plates were significantly improved compared to solvent - based plates. The mechanical properties of silk plates were measured by a four-point bending test using four-hole plates of 29 mm length and 2 mm thickness. The mechanics of aqueous plates (bending stiffness of 206.3 $\pm$ 13.8 N/mm, structural stiffness of 18574.4 $\pm$ 1242.8 N/mm$^2$, bending strength of 469.6 $\pm$ 92.8 N·mm, and bending modulus of 2.7 $\pm$ 0.2 GPa) were significantly improved relative to those of solvent-based plates (bending stiffness of 162.8 $\pm$ 23.0 N/mm, structural stiffness of 11621.7 $\pm$ 2608.7 N/mm$^2$, bending strength of 389.6 $\pm$ 56.4 N·mm, and bending modulus of 1.7 $\pm$ 0.3 GPa).

The tight packing of silk nano-globules is useful in determining the mechanical properties and machinability of the regenerated silk materials. Silk blanks from fast dehydration process were not machinable, which could be due to the faster formation of fibers/globules of $\beta$-sheet structure by methanol treatment and the resulting more loosely packed structures.

To demonstrate the versatility of the biomimetic silk materials, several functionalized silk orthopedic devices were prepared. One main appeal of the aqueous-based process lies in the development of osteoinductive orthopedic devices. Extensive work has been reported on the ability to functionalize various silk formats with various bioactive compounds. (See A. B. Li, 219 J. Controlled Release, 416 (2015)). Silk orthopedic device was functionalized with osteoinductive factors through incorporation of bone morphogenetic protein 2 (BMP-2) or P24 peptides. P24 is a synthetic BMP-2-related peptide corresponding to residues of the knuckle epitope of BMP-2, which could regulate adhesion and differentiation of bone marrow stromal cells and induce ectopic osteogenesis. FIG. 20 shows immunostaining of BMP-2 demonstrated the uniform incorporation of BMP-2 in silk pins and the bioactivity of the released BMP-2 was verified by improved ALP activity and calcification.

*In vitro* cell culture systems were created to mimic the screw-bone interface and the hydroxyapatite formation was stained with OsteoImage after 6 week of cell culture. FIG. 14 at panel (e) - FIG. 14 at panel (j) shows positive OsteoImage stain was detected at the screw / sponge interface and inside the sponge in BMP-2- and P24-incorporated silk screw groups. In contrast, FIG. 14 at panel (b) - FIG. 14 at panel (d) shows mineralization only occurred at the periphery of the silk sponge in the osteogenic control without BMP-2 or P24. Notably, the deposition of mineralized tissue followed the contour of the screw and was observed in direct with the screw surface in the presence of BMP2, which might imply the good osteointegeration of the screw with the bony tissue when implanted *in vivo.* Neither current metallic nor resorbable orthopedic fixation systems provide osteogenic functionality. The ability to stabilize and control the release of osteoinductive compound from silk could modulate the concentrations to local microenvironments, avoiding ectopic bone complications and optimizing sustained functional features as needed. This control would offer a distinct advantage of not only being biodegradable and resorbable but actually promoting and inducing bone growth and remodeling only locally.

A further advantage of the aqueous-based biomimetic process is to add antimicrobial functionality to the system. Incorporation of antibiotics within biodegradable orthopedic implants provides an approach for local antimicrobial prophylaxis of biomaterial-related infections without significant systematic exposure. This is a major need for orthopedic repairs. (See T. J. Mäkinen, 36 Bone, 292 (2005)). For this purpose, silk devices containing ciprofloxacin were prepared. Ciprofloxacin was selected in this study as it possesses a broad spectrum of activity, including most osteomyelitis-causing pathogens, such as *S. aureus.* FIG. 14 at panel (l) shows the antimicrobial effect of the silk/ciprofloxacin pins was demonstrated by a zone of inhibition assay, and a clear zone lacking bacteria growth formed around the ciprofloxacin-containing silk rods placed on the *S. aureus* bacterial lawn. Release studies were carried out in PBS as well as in the presence of protease IXV to model the potential *in vivo* proteolytic degradation of the silk. FIG. 14 at panel (m) shows the continuous release of ciprofloxacin was sustained for at least 36 days, suggesting that the bioactivity of the sensitive therapeutics was preserved during the mild, aqueous processing fabrication process. The stabilization effect of silk on antibiotic activity was consistent with previous findings on antibiotics stabilization within silk materials in various formats. (See E. M. Pritchard, 23 Adv. Funct. Mater. 854 (2013); and J. Zhang, 109 Proc. Natl. Acad. Sci. USA, 11981 (2012)). In addition, ciprofloxacin release in protease solution was significantly faster than that in PBS, which suggested that the release profile can be tuned by silk degradation.

Composite screws were previously developed by the addition of inorganic fillers such as HAP and β-tricalcium phosphate due to their proven osteoconductivity. (See M. Johnston, 27 Arthroscopy, 1671 (2011); and Arthrex Research and Development, Arthrex BioComposite Interference Screws for ACL and PCL Reconstruction (2010)). Studies have shown that silica-containing materials, such as silica nanoparticle, could stimulate the differentiation and activity of bone-building osteoblast but suppress bone-resorbing osteoclast *in vitro.* (See L. Macarini, 113 La radiologia medica, 1185 (2008)). FIG. 14 at panels (n), (o), (q), and (r) show incorporated HAP and silica nanoparticles in the silk blanks and composite blanks were the resulting composite blanks into orthopedic devices. The composite materials showed similar machinability to silk alone. For example, silk/HAP and silk/SiO$_2$ biocompsite screws showed a smooth surface finish after machining. FIG. 14 at panel (q) and panel (s) show that the FTIR analysis revealed the presence of HAP and silica nanoparticles in the screw.

We have demonstrated the feasibility of generating machinable silk materials via a biomimetic, all-aqueous process. We focused on the dehydration of silk solutions and the assembly of silk molecules to generate compact crystalline structures, as a route to generate highly dense, organized building blocks from which to generate mechanically robust silk-based orthopedic devices. The mild, all-aqueous process provides a wide range of opportunities by combining silk with other biomolecules (e.g., antibiotics, growth factors, cytokines), thus providing useful avenues for functionalized orthopedic devices.

**Claims**

1. A solid material comprising:

    at least one amphiphilic polypeptide **characterized by** an ability to adopt at least one of a beta-sheet secondary structure form and a globular structure form, wherein the at least one amphiphilic polypeptide comprises silk fibroin,
    wherein the solid material is **characterized by** a bound water content of less than about 50 wt%; the presence of a plurality of the globular structures, each having at least one dimension between about 5 nm and about 250 nm, wherein individual globular structures are aggregated with one another; and optionally a beta sheet content of at least about 40%;
    wherein the solid material is arranged and constructed so that when a biologically active molecule or macromolecule is encapsulated within the solid material, one or both of its structure and its biological activity is not materially degraded, reduced, and/or inhibited by its encapsulation,
    wherein the solid material is free of sericin, and
    wherein the solid material exhibits thermoplasticity.

2. The solid material of claim 1, wherein the biologically active molecules or macromolecules comprise members selected from the group consisting of: antibodies, growth factors, hormones, nucleic acids, peptides, proteins, or vaccines.

3. The solid material of claims 1 or 2, wherein the solid material is substantially free of hexafluoroisopropanol.

4. The solid material of any preceding claim, wherein the at least one amphiphilic polypeptide is a blend of at least two amphiphilic polypeptides.

5. The solid material of any preceding claim, wherein the solid material is characterized as sufficiently physically robust to withstand mechanical manipulation so that when machined the solid materials adopt and retain a desired shape.

6. The solid material of claim 5, wherein mechanical manipulation includes use or application of hand and/or machine tools.

7. The solid material of any of claims 1 to 3 and 5 and 6 when not dependent on claim 4, wherein the at least one amphiphilic polypeptide is silk fibroin.

8. The solid material of claim 7, **characterized by** a bulk density of between about 1.1 kg/dm$^3$ and about 1.4 kg/dm$^3$.

9. The solid material of any preceding claim, wherein the solid material is **characterized by** a bulk density substantially equivalent to a density of the amphiphilic polypeptide.

10. The solid material of any preceding claim, wherein the solid material is translucent or amber in colour.

11. The solid material of any preceding claim, further comprising an agent, additive, and/or functional moiety.

12. The solid material of claim 11, wherein the solid material is **characterized by** degradation such that the agent, additive, and/or functional moiety is released over time; and/or wherein the agent, additive, and/or functional moiety is present in the solid material so that the agent, additive, and/or functional moiety is released from the solid material when the solid material decomposes, degrades, denatures and/or delaminates.

13. The solid material of any preceding claim, wherein the solid material is **characterized by** a compressive modulus of greater than about 60 MPa; and/or wherein the solid material is **characterized by** a yield strength of greater than about 10 Mpa; and/or

    wherein the solid material is **characterized by** an elastic modulus in a range of between about 100 Pa and about 5000 kPa; and/or
    wherein the solid material is **characterized by** a compressive modulus in a range of between about 500 Pa and about 1 Gpa; and/or
    wherein the solid material is **characterized by** a resistance to damage from an applied pull-out force of about 500 N; and/or
    wherein the solid material is **characterized by** a shear stress up to about 100 Mpa.

14. A method for manufacturing a solid material, the method comprising (a) the steps of:

    providing an aqueous solution comprising an amphiphilic polypeptide, wherein the at least one amphiphilic polypeptide comprises silk fibroin;
    conformally changing the aqueous solution to a gel; and
    inducing a transition in the gel to form the solid material,
    wherein the step of inducing comprises removing water from the gel, freezing bound water in the gel at a temperature of at least 0 °C, or combinations thereof,
    wherein the amphiphilic polypeptide is **characterized by** an ability to adopt a beta-sheet secondary structure form,
    wherein the solid material exhibits thermoplasticity,
    wherein the solid material is free of sericin,
    wherein the solid material is **characterized by**:

        a beta-sheet content of at least about 55%; and
        a bound water content of less than about 50 wt%; and combinations thereof; and the presence of a plurality of the globular structures, each having at least one dimension between about 5 nm and about 250 nm, wherein individual globular structures are aggregated with one another, and
        wherein the solid material is arranged and constructed so that when a biologically active molecule or macromolecule is encapsulated within the solid material, one or both of its structure and its biological activity is not materially degraded, reduced, and/or inhibited by its encapsulation,
        or (b) the steps of:

        providing an aqueous solution comprising an amphiphilic polypeptide, wherein the at least one amphiphilic polypeptide comprises silk fibroin, wherein the solution is characterized as having an about neutral pH;
        inducing a transition in the aqueous solution to form a solid material, wherein the step of inducing comprises removing water from the solution at a temperature of at least 0 °C, wherein the amphiphilic polypeptide is **characterized by** an ability to adopt a globular structure form,
        wherein the solid material exhibits thermoplasticity,

wherein the solid material is free of sericin,
wherein the solid material is **characterized by**:

a bound water content of less than about 50 wt%; and
the presence of a plurality of the globular structures, each having at least one dimension between about 5 nm and about 30 nm, wherein individual globule structures are aggregated with one another; and combinations thereof, and
wherein the solid material is arranged and constructed so that when a biologically active molecule or macromolecule is encapsulated within the solid material, one or both of its structure and its biological activity is not materially degraded, reduced, and/or inhibited by its encapsulation.

15. The method of claim 14, wherein the amphiphilic polypeptide is silk fibroin and the step of providing the aqueous solution comprises generating a silk fibroin in water solution.


**Patentansprüche**

1.  Festes Material, umfassend:

zumindest ein amphiphiles Polypeptid, welches durch eine Fähigkeit gekennzeichnet ist, zumindest eine Beta-Faltblatt-Sekundärstrukturform und eine globuläre Strukturform anzunehmen, wobei das zumindest eine amphiphile Polypeptid Seidenfibroin umfasst,
wobei das feste Material durch einen Gehalt an gebundenem Wasser von weniger als etwa 50 Gew.-% gekennzeichnet ist; das Vorhandensein einer Vielzahl von globulären Strukturen, von welchen jede zumindest eine Abmessung zwischen etwa 5 nm und etwa 250 nm aufweist, wobei einzelne globuläre Strukturen miteinander aggregiert sind; und optional einen Gehalt an Beta-Faltblatt von zumindest etwa 40 %;
wobei das feste Material so angeordnet und konstruiert ist, dass beim Einkapseln eines biologisch aktiven Moleküls oder Makromoleküls innerhalb des festen Materials eine oder beide von seiner Struktur und seiner biologischen Aktivität durch seine Verkapselung nicht materiell beeinträchtigt, reduziert und/oder gehemmt werden,
wobei das feste Material frei von Sericin ist, und
wobei das feste Material Thermoplastizität zeigt.

2.  Festes Material nach Anspruch 1, wobei die biologisch aktiven Moleküle oder Makromoleküle Elemente umfassen, ausgewählt aus der Gruppe bestehend aus: Antikörpern, Wachstumsfaktoren, Hormonen, Nukleinsäuren, Peptiden, Proteinen oder Impfstoffen.

3.  Festes Material nach Ansprüchen 1 oder 2, wobei das feste Material im Wesentlichen frei von Hexafluorisopropanol ist.

4.  Festes Material nach einem vorstehenden Anspruch, wobei es sich bei dem zumindest einen amphiphilen Polypeptid um eine Mischung aus zumindest zwei amphiphilen Polypeptiden handelt.

5.  Festes Material nach einem vorstehenden Anspruch, wobei das feste Material als ausreichend physikalisch robust gekennzeichnet ist, um einer mechanischen Bearbeitung standzuhalten, sodass das feste Material beim Bearbeiten eine gewünschte Form annimmt und beibehält.

6.  Festes Material nach Anspruch 5, wobei die mechanische Bearbeitung Verwendung oder Anwendung von Hand- und/oder Maschinenwerkzeugen beinhaltet.

7.  Festes Material nach einem der Ansprüche 1 bis 3 und 5 und 6, sofern nicht von Anspruch 4 abhängig, wobei es sich bei dem zumindest einen amphiphilen Polypeptid um Seidenfibroin handelt.

8.  Festes Material nach Anspruch 7, **gekennzeichnet durch** eine Schüttdichte zwischen etwa 1,1 kg/dm$^3$ und etwa 1,4 kg/dm$^3$.

9.  Festes Material nach einem vorstehenden Anspruch, wobei das feste Material durch eine Schüttdichte gekennzeichnet ist, welche im Wesentlichen der Dichte des amphiphilen Polypeptids entspricht.

10. Festes Material nach einem vorstehenden Anspruch, wobei das feste Material durchscheinend oder bernsteinfarben ist.

11. Festes Material nach einem vorstehenden Anspruch, weiter umfassend einen Wirkstoff, ein Additiv und/oder eine funktionelle Einheit.

12. Festes Material nach Anspruch 11, wobei das feste Material durch einen Abbau wie beispielsweise die Freisetzung des Wirkstoffs, des Additivs und/oder der funktionellen Einheit im Laufe der Zeit gekennzeichnet ist; und/oder wobei der Wirkstoff, das Additiv und/oder die funktionelle Einheit in dem festen Material vorhanden ist, sodass der Wirkstoff, das Additiv und/oder die funktionelle Einheit aus dem festen Material freigesetzt wird, wenn sich das feste Material zersetzt, abbaut, denaturiert und/oder delaminiert.

13. Festes Material nach einem vorstehenden Anspruch, wobei das feste Material durch einen Kompressionsmodul von mehr als etwa 60 MPa gekennzeichnet ist; und/oder

wobei das feste Material durch eine Fließfestigkeit von mehr als etwa 10 MPa gekennzeichnet ist; und/oder
wobei das feste Material durch einen Elastizitätsmodul in einem Bereich zwischen etwa 100 Pa und etwa 5000 kPa gekennzeichnet ist; und/oder
wobei das feste Material durch einen Kompressionsmodul in einem Bereich zwischen etwa 500 Pa und etwa 1 GPa gekennzeichnet ist; und/oder
wobei das feste Material durch einen Widerstand gegen Beschädigung durch eine aufgebrachte Ausziehkraft von etwa 500 N gekennzeichnet ist; und/oder
wobei das feste Material durch eine Scherspannung von bis zu etwa 100 MPa gekennzeichnet ist.

14. Verfahren zum Herstellen eines festen Materials, wobei das Verfahren (a) die folgenden Schritte umfasst:

Bereitstellen einer wässrigen Lösung, welche ein amphiphiles Polypeptid umfasst, wobei das zumindest eine amphiphile Polypeptid Seidenfibroin umfasst;
konformes Umwandeln der wässrigen Lösung in ein Gel; und
Herbeiführen eines Phasenübergangs in dem Gel zur Bildung des festen Materials,
wobei der Schritt des Herbeiführens Entfernen von Wasser aus dem Gel, Einfrieren von gebundenem Wasser in dem Gel bei einer Temperatur von zumindest 0 °C oder Kombinationen davon umfasst,
wobei das amphiphile Polypeptid durch die Fähigkeit gekennzeichnet ist, eine Beta-Faltblatt-Sekundärstrukturform anzunehmen,
wobei das feste Material Thermoplastizität zeigt,
wobei das feste Material frei von Sericin ist,
wobei das feste Material **gekennzeichnet ist durch**:

einen Gehalt an Beta-Faltblatt von zumindest etwa 55 %; und
einen Gehalt an gebundenem Wasser von weniger als etwa 50 Gew.-%; und Kombinationen davon; und das Vorhandensein einer Vielzahl von globulären Strukturen, von welchen jede zumindest eine Abmessung zwischen etwa 5 nm und etwa 250 nm aufweist, wobei einzelne globuläre Strukturen miteinander aggregiert sind; und

wobei das feste Material so angeordnet und konstruiert ist, dass beim Einkapseln eines biologisch aktiven Moleküls oder Makromoleküls innerhalb des festen Materials eine oder beide von seiner Struktur und seiner biologischen Aktivität **durch** seine Verkapselung nicht materiell beeinträchtigt, reduziert und/oder gehemmt werden,
oder (b) die folgenden Schritte umfasst:

Bereitstellen einer wässrigen Lösung, welche ein amphiphiles Polypeptid umfasst, wobei das zumindest eine amphiphile Polypeptid Seidenfibroin umfasst, wobei die Lösung **dadurch** gekennzeichnet ist, dass sie einen etwa neutralen pH-Wert aufweist;
Herbeiführen eines Übergangs in der wässrigen Lösung zur Bildung eines festen Materials, wobei der Schritt des Herbeiführens Entfernen von Wasser aus der Lösung bei einer Temperatur von zumindest 0 °C umfasst, wobei das amphiphile Polypeptid **durch** die Fähigkeit gekennzeichnet ist, eine globuläre Strukturform anzunehmen,

wobei das feste Material Thermoplastizität zeigt,
wobei das feste Material frei von Sericin ist,
wobei das feste Material **gekennzeichnet ist durch**:

einen Gehalt an gebundenem Wasser von weniger als etwa 50 Gew.-%; und
das Vorhandensein einer Vielzahl von globulären Strukturen, von welchen jede zumindest eine Abmessung zwischen etwa 5 nm und etwa 30 nm aufweist, wobei einzelne globule Strukturen miteinander aggregiert sind; und Kombinationen davon, und

wobei das feste Material so angeordnet und konstruiert ist, dass beim Einkapseln eines biologisch aktiven Moleküls oder Makromoleküls innerhalb des festen Materials eine oder beide von seiner Struktur und seiner biologischen Aktivität **durch** seine Verkapselung nicht materiell beeinträchtigt, reduziert und/oder gehemmt werden.

**15.** Verfahren nach Anspruch 14, wobei es sich bei dem amphiphilen Polypeptid um Seidenfibroin handelt und der Schritt des Bereitstellens der wässrigen Lösung Erzeugen einer Seidenfibroin-in-Wasser-Lösung umfasst.


**Revendications**

**1.** Matériau solide comprenant :

au moins un polypeptide amphiphile **caractérisé par** une capacité d'adopter au moins une parmi une forme de structure secondaire en feuillet bêta et une forme de structure globulaire, dans lequel le au moins un polypeptide amphiphile comprend de la fibroïne de soie,
dans lequel le matériau solide est **caractérisé par** une teneur en eau liée inférieure à environ 50 % en poids ; la présence d'une pluralité des structures globulaires, présentant chacune au moins une dimension comprise entre environ 5 nm et environ 250 nm, dans lequel les structures globulaires individuelles sont agrégées entre elles ; et éventuellement une teneur en feuillets bêta d'au moins 40 % environ ;
dans lequel le matériau solide est agencé et construit de sorte que lorsqu'une molécule ou une macromolécule biologiquement active est encapsulée dans le matériau solide, sa structure et/ou son activité biologique ne soient pas matériellement dégradées, réduites et/ou inhibées par son encapsulation,
dans lequel le matériau solide est exempt de séricine, et
dans lequel le matériau solide présente une thermoplasticité.

**2.** Matériau solide selon la revendication 1, dans lequel les molécules ou macromolécules biologiquement actives comprennent des éléments sélectionnés dans le groupe consistant en : anticorps, facteurs de croissance, hormones, acides nucléiques, peptides, protéines ou vaccins.

**3.** Matériau solide selon les revendications 1 ou 2, dans lequel le matériau solide est sensiblement exempt d'hexafluoroisopropanol.

**4.** Matériau solide selon une quelconque revendication précédente, dans lequel le au moins un polypeptide amphiphile est un mélange d'au moins deux polypeptides amphiphiles.

**5.** Matériau solide selon une quelconque revendication précédente, dans lequel le matériau solide est **caractérisé en ce qu'**il est suffisamment robuste physiquement pour résister à la manipulation mécanique de sorte que, lorsqu'il est usiné, le matériau solide adopte et conserve une forme souhaitée.

**6.** Matériau solide selon la revendication 5, dans lequel la manipulation mécanique inclut une utilisation ou une application à la main et/ou par des machines-outils.

**7.** Matériau solide selon l'une quelconque des revendications 1 à 3 et 5 et 6 lorsqu'elles ne dépendent pas de la revendication 4, dans lequel le au moins un polypeptide amphiphile est de la fibroïne de soie.

**8.** Matériau solide selon la revendication 7, **caractérisé par** une masse volumique apparente comprise entre environ 1,1 kg/dm$^3$ et environ 1,4 kg/dm$^3$.

9. Matériau solide selon une quelconque revendication précédente, dans lequel le matériau solide est **caractérisé par** une masse volumique apparente sensiblement équivalente à une densité du polypeptide amphiphile.

10. Matériau solide selon une quelconque revendication précédente, dans lequel le matériau solide est translucide ou de couleur ambrée.

11. Matériau solide selon une quelconque revendication précédente, comprenant en outre un agent, un additif et/ou un fragment fonctionnel.

12. Matériau solide selon la revendication 11, dans lequel le matériau solide est **caractérisé par** une dégradation de telle façon que l'agent, l'additif et/ou le fragment fonctionnel soient libérés au fil du temps ; et/ou dans lequel l'agent, l'additif et/ou le fragment fonctionnel sont présents dans le matériau solide de sorte que l'agent, l'additif et/ou le fragment fonctionnel soient libérés par le matériau solide lorsque le matériau solide se décompose, se dégrade, se dénature et/ou est délaminé.

13. Matériau solide selon une quelconque revendication précédente, dans lequel le matériau solide est **caractérisé par** un module de compression supérieur à environ 60 MPa ; et/ou

dans lequel le matériau solide est **caractérisé par** une limite d'élasticité supérieure à environ 10 MPa ; et/ou
dans lequel le matériau solide est **caractérisé par** un module d'élasticité dans une plage entre environ 100 Pa et environ 5 000 kPa ; et/ou
dans lequel le matériau solide est **caractérisé par** un module de compression dans une plage entre environ 500 Pa et environ 1 GPa ; et/ou
dans lequel le matériau solide est **caractérisé par** une résistance aux dommages causés par une force d'arrachement appliquée d'environ 500 N ; et/ou
dans lequel le matériau solide est **caractérisé par** une contrainte de cisaillement pouvant atteindre environ 100 MPa.

14. Procédé de fabrication d'un matériau solide, le procédé comprenant (a) les étapes de :

fourniture d'une solution aqueuse comprenant un polypeptide amphiphile, dans lequel le au moins un polypeptide amphiphile comprend de la fibroïne de soie ;
changement de manière conforme de la solution aqueuse en gel ; et
induction d'une transition dans le gel pour former le matériau solide,
dans lequel l'étape d'induction comprend l'élimination de l'eau du gel, la congélation de l'eau liée dans le gel à une température d'au moins 0 °C, ou des combinaisons de ces étapes,
dans lequel le polypeptide amphiphile est **caractérisé par** sa capacité d'adopter une forme de structure secondaire en feuillet bêta,
dans lequel le matériau solide présente une thermoplasticité,
dans lequel le matériau solide est exempt de séricine,
dans lequel le matériau solide est **caractérisé par** :

une teneur en feuillet bêta d'au moins 55 % environ ; et
une teneur en eau liée inférieure à environ 50 % en poids ; et des combinaisons de celles-ci ; et la présence d'une pluralité des structures globulaires, présentant chacune au moins une dimension comprise entre environ 5 nm et environ 250 nm, dans lequel les structures globulaires individuelles sont agrégées entre elles, et

dans lequel le matériau solide est agencé et construit de sorte que lorsqu'une molécule ou une macromolécule biologiquement active est encapsulée dans le matériau solide, sa structure et/ou son activité biologique ne soient pas matériellement dégradées, réduites et/ou inhibées par son encapsulation,
ou (b) les étapes de :

fourniture d'une solution aqueuse comprenant un polypeptide amphiphile, dans lequel le au moins un polypeptide amphiphile comprend de la fibroïne de soie, dans lequel la solution est **caractérisée en ce qu'**elle présente un pH à peu près neutre ;
induction d'une transition dans la solution aqueuse pour former un matériau solide, dans lequel l'étape d'induction comprend l'élimination de l'eau de la solution à une température d'au moins 0 °C, dans lequel le polypeptide amphiphile est **caractérisé par** une capacité d'adopter une forme de structure globulaire,

dans lequel le matériau solide présente une thermoplasticité,
dans lequel le matériau solide est exempt de séricine,
dans lequel le matériau solide est **caractérisé par** :

une teneur en eau liée inférieure à environ 50 % en poids ; et
la présence d'une pluralité des structures globulaires, présentant chacune au moins une dimension comprise entre environ 5 nm et environ 30 nm, dans lequel les structures globulaires individuelles sont agrégées les unes aux autres ; et des combinaisons de ceux-ci, et

dans lequel le matériau solide est agencé et construit de sorte que lorsqu'une molécule ou une macromolécule biologiquement active est encapsulée dans le matériau solide, sa structure et/ou son activité biologique ne soient pas matériellement dégradées, réduites et/ou inhibées par son encapsulation.

15. Procédé selon la revendication 14, dans lequel le polypeptide amphiphile est de la fibroïne de soie et l'étape de fourniture de la solution aqueuse comprend la génération de fibroïne de soie dans la solution aqueuse.

(a)

*FIG. 1*

Legend:
- Water
- Amorphous
- Helix
- Beta-sheets

Particle/Micelle

- Hydrophobic domain
- Hydrophilic spacer

(b)

*FIG. 1*

- Water
- Amorphous
- Helix
- Beta-sheets

Intermicellar interactions

- Hydrophobic domain
- Hydrophilic spacer

(c)

*FIG. 1*

EP 3 328 438 B1

Fusion of micelles to form crystalline silk fibroin constructs

● Water

〜 Amorphous

▨ Helix

▨ Beta-sheets

〜 Hydrophobic domain

● Hydrophilic spacer

(d)

*FIG. 1*

EP 3 328 438 B1

*FIG. 2*

*FIG. 3*

**FIG. 3**

EP 3 328 438 B1

**FIG. 4**

*FIG. 5*

a

b

*FIG. 6*

**FIG. 7**

*FIG. 8*

**FIG. 9**

**FIG. 10**

**FIG. 11**

**FIG. 12**

EP 3 328 438 B1

FIG. 12

**j**

Hydrophobic domain
Hydrophilic domain

β-sheet

*FIG. 12*

*FIG. 13*

FIG. 13

*FIG. 14*

*FIG. 14*

**FIG. 14**

**FIG. 14**

FIG. 14

*FIG. 15*

**FIG. 16**

(a)

(b)

*FIG. 17*

*FIG. 18*

*FIG. 19*

EP 3 328 438 B1

*FIG. 20*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62197693
- WO 2011005381 A
- US 2011008437 A
- WO 2009100280 A2, Kaplan
- WO 2008127404 A
- WO 2008118211 A
- WO 2008127402 A
- WO 2008127403 A
- WO 2008127401 A
- WO 2008140562 A
- WO 2009061823 A
- WO 2009155397 A
- WO 2010126640 A
- WO 2011046652 A
- WO 2011026101 A
- WO 2012054121 A
- WO 2011130335 A
- WO 2011112931 A
- WO 2012047682 A
- WO 2012031282 A
- WO 2010088585 A
- WO 2013130156 A

- WO 9708315 A
- US 5245012 A
- WO 2004080346 A
- WO 2005012606 A
- WO 2005123114 A
- WO 2007016524 A
- WO 2008150861 A
- WO 2008118133 A
- US 8247384 B
- US 20040067503
- US 020650
- US 10541182 B
- US 11407373 B
- US 11664234 B
- US 07020789 W
- US 0855072 W
- WO 2010036992 A2, Bernhard
- WO 2011005381 A2, Kaplan
- WO 2008150861 A1, Wang
- WO 2012087823 A2, Kaplan
- WO 2010042798 A2, Kaplan

**Non-patent literature cited in the description**

- **U. G. K. WEGST**. *14 Nature Mater.*, 2015, vol. 23
- **MEYERS ; MILLER**. *CABIOS*, 1989, vol. 4, 11-17
- **CATERSON et al.** *J. Biomed. Mater. Res.*, 2001, vol. 57, 394-403
- **KARP et al.** *J. Craniofacial Surgery 3*, 2003, vol. 14, 317-323
- **HARRIS et al.** *J. Biomed. Mater. Res.*, 1998, vol. 42, 396-402
- **HOLY et al.** *J. Biomed. Mater. Res*, 2003, vol. 65A, 447-453
- **HUTMACHER D**. *Biomaterials*, 2000, vol. 21, 2529-2543
- **MARTIN et al.** *J. Biomed. Mater. Res*, 2001, vol. 55, 229-235
- **PEREZ-RIGUEIRO et al.** *Science*, 1998, vol. 70, 2439-2447
- **PETITE et al.** *Nat. Biotechnol.*, 2000, vol. 18, 959-96
- **SOFIA et al.** *J. of Biomedical Materials Research*, 2001, vol. 54, 139-148

- **ELISSEEFF**. The Silk Roads: Highways of Culture and Commerce. Berghahn Books/UNESCO, 2000
- **VAINKER**. Chinese Silk: A Cultural History. Rutgers University Press, 2004
- **ALTMAN et al.** *Biomaterials*, 2003, vol. 24, 401
- **SASHINA et al.** *Russ. J. Appl. Chem.*, 2006, vol. 79, 869
- **OMENETTO ; KAPLAN**. *Science*, 2010, vol. 329, 528-531
- **TAKEI, F ; KIKUCHI, Y ; KIKUCHI, A ; MIZUNO, S ; SHIMURA, K**. *J. Cell Biol.*, 1987, vol. 105, 175-180
- **TANAKA, K ; MORI, K. ; MIZUNO, S.** *J. Biochem. (Tokyo)*, 1993, vol. 114, 1-4
- **TANAKA, K. ; KAJIYAMA, N ; ISHIKURA, K. ; WAGA, S ; KIKUCHI, A ; OHTOMO, K ; TAKAGI, T ; MIZUNO, S.** *Biochim. Biophys. Acta*, 1999, vol. 1432, 92-103

- **Y KIKUCHI**; **K MORI**; **S SUZUKI**; **K YAMAGUCHI**; **S MIZUNO**. Structure of the Bombyx mori fibroin light-chain-encoding gene: upstream sequence elements common to the light and heavy chain. *Gene*, 1992, vol. 110, 151-158
- **LUCAS et al.** *Adv. Protein Chem*, 1958, vol. 13, 107-242
- **VALEUR, B.** Molecular Fluorescence: Principles and Applications. John Wiley and Sons, 2002
- Handbook of Fluorescent Probes and Research Products, Molecular Probes. 2002
- The Handbook - A Guide to Fluorescent Probes and Labeling Technologies. Invitrogen
- **ALTMAN et al.** *Biomats*, 2003, vol. 24, 401-16
- **JIN**; **KAPLAN**. *Nature*, 2003, vol. 424, 1057-61
- **HORAN et al.** *Biomats*, 2005, vol. 26, 3385-93
- **KIM et al.** *Biomats*, 2005, vol. 26, 2775-85
- **ISHIDA et al.** *Macromolecules*, 1990, vol. 23, 88-94
- **NAZAROV et al.** *Biomacromolecules*, 2004, vol. 5, 718-26
- **JIN et al.** *Biomacromolecules*, 2004, vol. 5, 711-7
- **JIN et al.** *Biomacromolecules*, 2002, vol. 3, 1233-39
- **HINO et al.** *J. Colloid Interface Sci.*, 2003, vol. 266, 68-73
- **WANG et al.** *J. Control Release*, 2007, vol. 117, 360-70
- **LEE et al.** *Int'l J. Pharma*, 2001, vol. 221, 1-22
- **SMIDSRØD et al.** *Trends Biotech.*, 1990, vol. 8, 71-78
- **WANG et al.** *Int'l J. Biol. Macromol.*, 2005, vol. 36, 66-70
- **KIM et al.** *Biomacromolecules*, 2004, vol. 5, 786-92
- **MATSUMOTO et al.** *J. Phys. Chem. B*, 2006, vol. 110, 21630-38
- **PAULUSSE**; **SIJBESMA**. *J. Polym. Sci.-Polym. Chem.*, 2006, vol. 44, 5445-53
- **KEMMERE et al.** *Macromol. Mater. Eng.*, 2005, vol. 290, 302-10
- **MOTTA et al.** *J. Biomater. Sci. Polymer. Edu.*, 2004, vol. 15, 851-64
- **NUTTELMAN et al.** *Matrix Biol.*, 2005, vol. 24, 208-18
- **NUTTELMAN et al.** *Biomats*, 2006, vol. 27, 1377-86
- **MAUCK et al.** *Osteoarthr. Cartilage*, 2006, vol. 14, 179-89
- **LEWUS**; **NAUMAN**. *Tissue Eng*, 2005, vol. 11, 1015-22
- **MAJUMDAR et al.** *J. Cell Physiol.*, 2000, vol. 185, 98-106
- **BOISON**. *Trends Pharmacol. Sci.*, 2006, vol. 27, 652-58
- **VOLLRATH**; **KNIGHT**. *Nature*, 2001, vol. 410, 541-48
- **CHEN et al.** *Biomacromolecules*, 2002, vol. 3, 644-8
- **ZHOU et al.** *J. Phys. Chem. B*, 2005, vol. 109, 16937-45
- **DICKO et al.** *Biomacromolecules*, 2004, vol. 5, 704-10
- **TERRY et al.** *Biomacromolecules*, 2004, vol. 5, 768-72
- **D. N. ROCKWOOD**. *Nat. Protoc.*, 2011, vol. 6, 1612
- **J. LUO**. *Int. J. Biol. Macromol*, 2014, vol. 66, 319
- **M. SUN**. *J. Mater. Chem.*, 2012, vol. 22, 18372
- **G. ZHOU**. *Adv. Mater.*, 2009, vol. 21, 366
- **D. S. GOLDBERG**. *J. Craniofac. Surg.*, 1995, vol. 6, 301
- **J. ZHANG**. *J. Trauma Acute Care Surg.*, 2012, vol. 72, E106
- **D. EGLIN**. *European cells & materials*, 2008, vol. 16, 80
- **G. S. PERRONE**. *Nat. Commun*, 2014, vol. 5
- **C. VEPARI**. *Prog. Polym. Sci.*, 2007, vol. 32, 991
- **J. PÉREZ-RIGUEIRO**. *Macromolecules*, 2007, vol. 40, 5360
- **C. P. BROWN**. *ACS Nano*, 2012, vol. 6, 1961
- **D. REINSTÄDLER**. *Proteins: Struct. Funct. Bioinform*, 1999, vol. 34, 303
- **A. BARTH**. *Progress in Biophysics and Molecular Biology*, 2000, vol. 74, 141
- **X. HU**. *Macromolecules*, 2006, vol. 39, 6161
- **U. G. K. WEGST**. *Nature Mater*, 2015, vol. 14, 23
- **A. SALUJA**. *Int. J. Pharm.*, 2008, vol. 358, 1
- **A. STRADNER**. *Nature*, 2004, vol. 432, 492
- **E.J. YEARLEY**. *Biophys. J.,*, 2014, vol. 106, 1763
- **K. J. BOZIC**. *J. Hand Surg. Am.*, 2001, vol. 26, 755
- **F. MUJIKA**. *Polym. Test.*, 2006, vol. 25, 214
- **M. M. SHALABI**. *J. Dent. Res.*, 2006, vol. 85, 496
- **M. JAGER, J.** *Biomed. Biotechnol*, 2007
- **N. ASHAMMAKHI**. *J. Mater. Sci. Mater. Med.*, 2006, vol. 17, 1275
- **S. LEINONEN**. *J. Craniofac. Surg.*, 2002, vol. 13, 212
- **A. B. LI**. *J. Controlled Release*, 2015, vol. 416, 219
- **T. J. MÄKINEN**. *Bone*, 2005, vol. 36, 292
- **E. M. PRITCHARD**. *Adv. Funct. Mater*, 2013, vol. 23, 854
- **J. ZHANG**. *Proc. Natl. Acad. Sci. USA*, 2012, vol. 109, 11981

- **M. JOHNSTON**. *Arthroscopy*, 2011, vol. 27, 1671
- Arthrex Research and Development. *Arthrex Bio-Composite Interference Screws for ACL and PCL Reconstruction*, 2010
- **L. MACARINI**. *La radiologia medica*, 2008, vol. 113, 1185